(19) Europäisches Patentamt
European Patent Office
Office européen des brevets

(11) EP 3 617 201 B1

(12) EUROPEAN PATENT SPECIFICATION

(45) Date of publication and mention of the grant of the patent:
**04.08.2021 Bulletin 2021/31**

(51) Int Cl.:
***C07D 401/14*** *(2006.01)* ***C09K 11/06*** *(2006.01)*
***H01L 51/50*** *(2006.01)*

(21) Application number: **19192990.0**

(22) Date of filing: **22.08.2019**

(54) **ORGANIC MOLECULES FOR OPTOELECTRONIC DEVICES**

ORGANISCHE MOLEKÜLE FÜR OPTOELEKTRONISCHE VORRICHTUNGEN

MOLÉCULES ORGANIQUES POUR DISPOSITIFS OPTOÉLECTRONIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.08.2018 DE 102018120889**

(43) Date of publication of application:
**04.03.2020 Bulletin 2020/10**

(73) Proprietor: **CYNORA GMBH**
**76646 Bruchsal (DE)**

(72) Inventors:
- **DIGENNARO, Dr. Angela**
**69120 Heidelberg (DE)**
- **VELLAIYAPPAN, Arun**
**76131 Karlsruhe (DE)**

(74) Representative: **Hoppe, Georg Johannes**
**Darani Anwaltskanzlei**
**Beuckestrasse 20**
**14163 Berlin (DE)**

(56) References cited:
**EP-A1- 3 421 461**
**WO-A1-2019/002355**
**US-A1- 2017 186 962**
**US-A1- 2018 215 711**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 617 201 B1

## Description

The invention relates to organic molecules and their use in organic light-emitting diodes (OLEDs) and in other optoelectronic devices.

## Description

The object of the present invention is to provide molecules which are suitable for use in organic optoelectronic devices.

This object is achieved by the invention which provides a new class of organic molecules.

According to the invention, the organic molecules are purely organic molecules, i.e. they do not contain any metal ions in contrast to metal complexes known for use in organic optoelectronic devices. Therefore, according to the present invention, it is preferred that the organic molecules are free of metal atoms or metal ions. The pure organic molecules may, however, include metalloids, in particular, B and Si or Sn, Se, and Ge.

According to the present invention, the organic molecules exhibit emission maxima in the blue, sky-blue or green spectral range. The organic molecules exhibit in particular emission maxima between 420 nm and 520 nm, preferably between 440 nm and 495 nm, more preferably between 450 nm and 470 nm. The photoluminescence quantum yields of the organic molecules according to the invention are, in particular, 50 % or more. The molecules according to the invention exhibit in particular thermally activated delayed fluorescence (TADF). The use of the molecules according to the invention in an optoelectronic device, for example an organic light-emitting diode (OLED), leads to higher efficiencies of the device. Corresponding OLEDs have a higher stability than OLEDs with known emitter materials and comparable color. Other OLED compounds incorporating a phenyl-pyridyl fragment are known from EP 3 421 461 and WO2019/002355. Further biphenyl type compounds are known from US2018/0215711 and US2017/0186962.

The organic light-emitting molecules of the invention consist of one first chemical moiety comprising or consisting of a structure of Formula I,

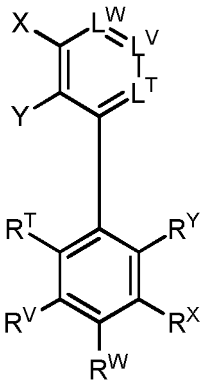

Formula I

and

- two second chemical moieties, each independently from another comprising or consisting of a structure of Formula II,

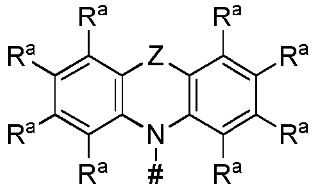

Formula II

wherein the first chemical moiety is linked to each of the two second chemical moieties via a single bond.
$L^T$ is N or C-$R^1$.
$L^V$ is N or C-$R^1$.
$L^W$ is N or C-W.

X is the binding site of a single bond linking the first chemical moiety to one of the two second chemical moieties or is $R^2$.
Y is the binding site of a single bond linking the first chemical moiety to one of the two second chemical moieties or is $R^2$.
W is the binding site of a single bond linking the first chemical moiety to one of the two second chemical moieties or is $R^2$.

$R^T$ is selected from the group consisting of $R^I$, CN and 1,3,5-triazinyl, which is substituted with two substituents $R^{Tz}$.
$R^V$ is selected from the group consisting of $R^I$, CN and 1,3,5-triazinyl, which is substituted with two substituents $R^{Tz}$.
$R^W$ is selected from the group consisting of $R^I$, CN and 1,3,5-triazinyl, which is substituted with two substituents $R^{Tz}$.
$R^X$ is selected from the group consisting of $R^I$, CN and 1,3,5-triazinyl, which is substituted with two substituents $R^{Tz}$.
$R^Y$ is the binding site of a single bond linking the first chemical moiety to one of the two second chemical moieties.
# represents the binding site of a single bond linking the second chemical moieties to the first chemical moiety;
Z is at each occurrence independently from another selected from the group consisting of a direct bond, $CR^3R^4$, $C=CR^3R^4$, C=O, $C=NR^3$, $NR^3$, O, $SiR^3R^4$, S, S(O) and $S(O)_2$;
$R^1$ is at each occurrence independently from another selected from the group consisting of hydrogen,
deuterium,
$C_1$-$C_5$-alkyl,

wherein one or more hydrogen atoms are optionally substituted by deuterium; C2-C8-alkenyl,
wherein one or more hydrogen atoms are optionally substituted by deuterium; C2-C8-alkynyl,
wherein one or more hydrogen atoms are optionally substituted by deuterium; and $C_6$-$C_{18}$-aryl,
which is optionally substituted with one or more substituents $R^6$.

$R^2$ is at each occurrence independently from another selected from the group consisting of hydrogen,
deuterium,
$C_1$-$C_5$-alkyl,
wherein one or more hydrogen atoms are optionally substituted by deuterium;
C2-C8-alkenyl,
wherein one or more hydrogen atoms are optionally substituted by deuterium;
C2-C8-alkynyl,
wherein one or more hydrogen atoms are optionally substituted by deuterium; and
$C_6$-$C_{18}$-aryl,
which is optionally substituted with one or more substituents $R^6$.
$R^I$ is at each occurrence independently from another selected from the group consisting of hydrogen,
deuterium,
$C_1$-$C_5$-alkyl,
wherein one or more hydrogen atoms are optionally substituted by deuterium;
C2-C8-alkenyl,
wherein one or more hydrogen atoms are optionally substituted by deuterium;
C2-C8-alkynyl,
wherein one or more hydrogen atoms are optionally substituted by deuterium; and
$C_6$-$C_{18}$-aryl,
which is optionally substituted with one or more substituents $R^6$.
$R^{Tz}$ is at each occurrence independently from another selected from the group consisting of: hydrogen,
deuterium,
$C_1$-$C_5$-alkyl,
wherein one or more hydrogen atoms are optionally substituted by deuterium;
$C_6$-$C_{18}$-aryl,
which is optionally substituted with one or more substituents $R^6$, and
$C_3$-$C_{17}$-heteroaryl,
which is optionally substituted with one or more substituents $R^6$.
$R^a$, $R^3$ and $R^4$ is at each occurrence independently from another selected from the group consisting of hydrogen, deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-alkyl,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$,

$Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$.

$R^5$ is at each occurrence independently from another selected from the group consisting of hydrogen, deuterium, $N(R^6)_2$, $OR^6$, $Si(R^6)_3$, $B(OR^6)_2$, $OSO_2R^6$, $CF_3$, CN, F, Br, I,
$C_1$-$C_{40}$-alkyl,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-alkoxy,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-thioalkoxy,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkenyl,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkynyl,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_6$-$C_{60}$-aryl,
which is optionally substituted with one or more substituents $R^6$; and
$C_3$-$C_{57}$-heteroaryl,
which is optionally substituted with one or more substituents $R^6$.

$R^6$ is at each occurrence independently from another selected from the group consisting of hydrogen, deuterium, OPh, $CF_3$, CN, F,
$C_1$-$C_5$-alkyl,
wherein optionally one or more hydrogen atoms are independently from each other substituted by deuterium, CN, $CF_3$, or F;
$C_1$-$C_5$-alkoxy,
wherein optionally one or more hydrogen atoms are independently from each other substituted by deuterium, CN, $CF_3$, or F;
$C_1$-$C_5$-thioalkoxy,
wherein optionally one or more hydrogen atoms are independently from each other substituted by deuterium, CN, $CF_3$, or F;
C2-C5-alkenyl,
wherein optionally one or more hydrogen atoms are independently from each other substituted by deuterium, CN, $CF_3$, or F;
C2-C5-alkynyl,
wherein optionally one or more hydrogen atoms are independently from each other substituted by deuterium, CN, $CF_3$, or F;
$C_6$-$C_{18}$-aryl,
which is optionally substituted with one or more $C_1$-$C_5$-alkyl substituents;
$C_3$-$C_{17}$-heteroaryl,
which is optionally substituted with one or more $C_1$-$C_5$-alkyl substituents;
$N(C_6\text{-}C_{18}\text{-aryl})_2$,
$N(C_3\text{-}C_{17}\text{-heteroaryl})_2$; and
$N(C_3\text{-}C_{17}\text{-heteroaryl})(C_6\text{-}C_{18}\text{-aryl})$.

The substituents $R^a$, $R^3$, $R^4$ or $R^5$ independently from each other can optionally form a mono- or polycyclic, aliphatic, aromatic and/or benzo-fused ring system with one or more substituents $R^a$, $R^3$, $R^4$ or $R^5$.

According to the invention, exactly one ring member (atom or group) selected from the group consisting of $L^T$, $L^V$ and $L^W$ is N;
exactly one substituent selected from the group consisting of $R^T$, $R^V$, $R^W$ and $R^X$ is 1,3,5-triazinyl, which is substituted with two substituents $R^{Tz}$, exactly one substituent selected from the group consisting of $R^T$, $R^V$, $R^W$ and $R^X$ is CN; and exactly one substituent selected from the group consisting of W, Y and X represents the binding site of a single bond linking the first chemical moiety and one of the two second chemical moieties.

In one embodiment of the invention, the first chemical moiety comprises or consists of a structure of Formula Ia:

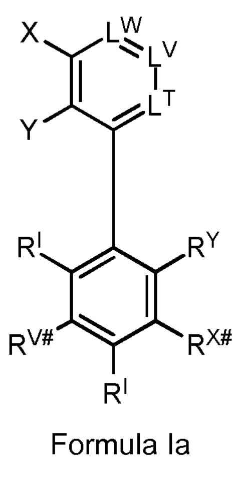

Formula Ia

wherein X, Y, $L^W$, $L^V$, $L^T$, $R^I$ and $R^Y$ are defined as above;
exactly one substituent selected from the group consisting of $R^{V\#}$ and $R^{X\#}$ is 1,3,5-triazinyl, which is substituted with two substituents $R^{Tz}$,
exactly one substituent selected from the group consisting of $R^{V\#}$ and $R^{X\#}$ is CN.

In one embodiment, $R^1$, $R^2$ and $R^1$ are at each occurrence independently from another selected from the group consisting of hydrogen (H), methyl, mesityl, tolyl and phenyl (Ph). The term tolyl comprises 2-tolyl, 3-tolyl and 4-tolyl.

In one embodiment, $R^1$, $R^2$ and $R^1$ are at each occurrence independently from another selected from the group consisting of hydrogen (H), methyl, and phenyl.

In one embodiment, $R^1$, $R^2$ and $R^1$ are at each occurrence hydrogen (H).

In one embodiment, $R^X$ is 1,3,5-triazinyl, which is substituted with two substituents $R^{Tz}$.

In one embodiment, $R^V$ is 1,3,5-triazinyl, which is substituted with two substituents $R^{Tz}$.

In one embodiment, $R^W$ is 1,3,5-triazinyl, which is substituted with two substituents $R^{Tz}$.

In one embodiment, $R^T$ is 1,3,5-triazinyl, which is substituted with two substituents $R^{Tz}$.

In one embodiment, $R^X$ is CN.

In one embodiment, $R^V$ is CN.

In one embodiment, $R^W$ is CN.

In one embodiment, $R^T$ is CN.

In one embodiment, $R^X$ is 1,3,5-triazinyl, which is substituted with two substituents $R^{Tz}$ and $R^V$ is CN.

In one embodiment, $R^V$ is 1,3,5-triazinyl, which is substituted with two substituents $R^{Tz}$ and $R^X$ is CN.

In one embodiment, $L^V$ is N.

In one embodiment, $L^T$ is N and Y is the binding site of a single bond linking the first chemical moiety to one of the two second chemical moieties.

In one embodiment, $L^W$ is N.

In one embodiment, $L^T$ is N.

In one embodiment, $L^T$ is N and X is the binding site of a single bond linking the first chemical moiety to one of the two second chemical moieties.

In a further embodiment of the invention $R^{Tz}$ is independently from each other selected from the group consisting of H, methyl,
phenyl, which is optionally substituted with one or more substituents $R^6$, 1,3,5-triazinyl, which is optionally substituted with one or more substituents $R^6$, and pyrimidinyl, which is optionally substituted with one or more substituents $R^6$.

In a further embodiment of the invention $R^{Tz}$ is independently from each other selected from the group consisting of H, methyl and phenyl.

In a further embodiment of the invention $R^{Tz}$ is phenyl at each occurrence, which is optionally substituted with one or more substituents $R^5$.

In a further embodiment of the invention $R^{Tz}$ is phenyl at each occurrence, which is optionally substituted with one or more substituents $R^6$.

In a further embodiment of the invention $R^{Tz}$ is phenyl at each occurrence.

In one embodiment, at least one $R^a$ is not H.

In one embodiment, the two second chemical moieties are different from each other. Exactly one $R^8$ at each second chemical moiety is not H in some embodiments; in other embodiments, exactly one $R^a$ at one second chemical moiety is not H.

In one embodiment, the two second chemical moieties are different from each other.

In one embodiment, the two second chemical moieties are identical to each other.

In a further embodiment of the invention, the two second chemical moieties independently from another comprise or consist of a structure of Formula IIa:

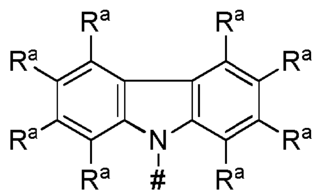

Formula IIa

wherein # and $R^a$ are defined as above.

In a further embodiment of the invention, $R^a$ is at each occurrence independently from another selected from the group consisting of H,

Me,
$^{i}Pr$,
$^{t}Bu$,
CN,
$CF_3$,
Ph, which is optionally substituted with one or more substituents independently from each other selected from the group consisting of Me, $^{i}Pr$, $^{t}Bu$, CN, $CF_3$, and Ph,
pyridinyl, which is optionally substituted with one or more substituents independently from each other selected from the group consisting of Me, $^{i}Pr$, $^{t}Bu$, CN, $CF_3$, and Ph,
pyrimidinyl, which is optionally substituted with one or more substituents independently from each other selected from the group consisting of Me, $^{i}Pr$, $^{t}Bu$, CN, $CF_3$, and Ph,
carbazolyl, which is optionally substituted with one or more substituents independently from each other selected from the group consisting of Me, $^{i}Pr$, $^{t}Bu$, CN, $CF_3$, and Ph,
triazinyl, which is optionally substituted with one or more substituents independently from each other selected from the group consisting of Me, $^{i}Pr$, $^{t}Bu$, CN, $CF_3$, and Ph,
and $N(Ph)_2$.

In a further embodiment of the invention, $R^a$ is at each occurrence independently from another selected from the group consisting of H,
Me,
$^{i}Pr$,
$^{t}Bu$,
CN,
$CF_3$,
Ph, which is optionally substituted with one or more substituents independently from each other selected from the group consisting of Me, $^{i}Pr$, $^{t}Bu$, CN, $CF_3$, and Ph,
pyridinyl, which is optionally substituted with one or more substituents independently from each other selected from the group consisting of Me, $^{i}Pr$, $^{t}Bu$, CN, $CF_3$, and Ph,
pyrimidinyl, which is optionally substituted with one or more substituents independently from each other selected from the group consisting of Me, $^{i}Pr$, $^{t}Bu$, CN, $CF_3$, and Ph, and
triazinyl, which is optionally substituted with one or more substituents independently from each other selected from the group consisting of Me, $^{i}Pr$, $^{t}Bu$, CN, $CF_3$, and Ph.

In a further embodiment of the invention, $R^a$ is at each occurrence independently from another selected from the group consisting of H,
Me,
$^{t}Bu$,
Ph, which is optionally substituted with one or more substituents independently from each other selected from the group consisting of Me, $^{i}Pr$, $^{t}Bu$, CN, $CF_3$, and Ph,
triazinyl, which is optionally substituted with one or more substituents independently from each other selected from the group consisting of Me, $^{i}Pr$, $^{t}Bu$, CN, $CF_3$, and Ph.

In a further embodiment of the invention, $R^a$ is H at each occurrence

In a further embodiment of the invention, one or both of the two second chemical moieties independently from another comprise or consist of a structure of Formula IIb, a structure of Formula IIb-2, a structure of Formula IIb-3 or a structure of Formula IIb-4:

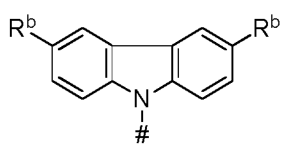
Formula IIb

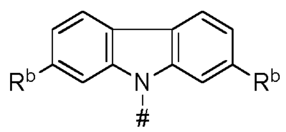
Formula IIb-2

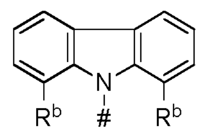
Formula IIb-3

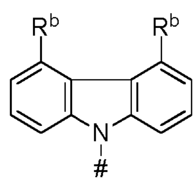
Formula IIb-4

wherein
$R^b$ is at each occurrence independently from another selected from the group consisting of deuterium,
$N(R^5)_2$,
$OR^5$,
$Si(R^5)_3$,

$B(OR^5)_2$,
$OSO_2R^5$,
$CF_3$,
CN,
F,
Br,
I,
$C_1$-$C_{40}$-alkyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$.
Apart from that the aforementioned definitions apply.

In a further embodiment of the invention, the two second chemical moieties each at each occurrence independently from another comprise or consist of a structure of Formula IIb, a structure of Formula IIb-2, a structure of Formula IIb-3 or a structure of Formula IIb-4.

In one additional embodiment of the invention, one or both of the two second chemical moieties each at each occurrence independently from another comprise or consist of a structure of Formula IIc, a structure of Formula IIc-2, a structure of Formula IIc-3 or a structure of Formula IIc-4:

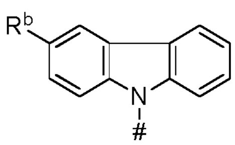
Formula IIc

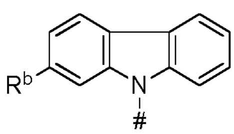
Formula IIc-2

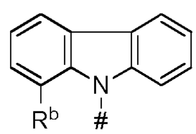
Formula IIc-3

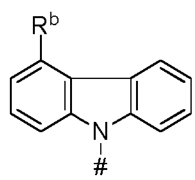
Formula IIc-4

wherein the aforementioned definitions apply.

In one additional embodiment of the invention, the two second chemical moieties each at each occurrence independently from another comprise or consist of a structure of Formula IIc, a structure of Formula IIc-2, a structure of Formula IIc-3 or a structure of Formula IIc-4.

In a further embodiment of the invention, $R^b$ is at each occurrence independently from another selected from the group consisting of
Me,
$^i$Pr,
$^t$Bu,
CN,
$CF_3$,
Ph, which is optionally substituted with one or more substituents independently from each other selected from the group consisting of Me, $^i$Pr, $^t$Bu, CN, $CF_3$, and Ph,
pyridinyl, which is optionally substituted with one or more substituents independently from each other selected from the group consisting of Me, $^i$Pr, $^t$Bu, CN, $CF_3$, and Ph,
pyrimidinyl, which is optionally substituted with one or more substituents independently from each other selected from the group consisting of Me, $^i$Pr, $^t$Bu, CN, $CF_3$, and Ph, and
carbazolyl, which is optionally substituted with one or more substituents independently from each other selected from the group consisting of Me, $^i$Pr, $^t$Bu, CN, $CF_3$, and Ph,
triazinyl, which is optionally substituted with one or more substituents independently from each other selected from the group consisting of Me, $^i$Pr, $^t$Bu, CN, $CF_3$, and Ph,
and $N(Ph)_2$.

In a further embodiment of the invention, $R^b$ is at each occurrence independently from another selected from the group consisting of
Me,
$^i$Pr,
$^t$Bu,
CN,
$CF_3$,
Ph, which is optionally substituted with one or more substituents independently from each other selected from the group consisting of Me, $^i$Pr, $^t$Bu, CN, $CF_3$, and Ph,
pyridinyl, which is optionally substituted with one or more substituents independently from each other selected from the group consisting of Me, $^i$Pr, $^t$Bu, CN, $CF_3$, and Ph,
pyrimidinyl, which is optionally substituted with one or more substituents independently from each other selected from the group consisting of Me, $^i$Pr, $^t$Bu, CN, $CF_3$, and Ph, and
triazinyl, which is optionally substituted with one or more substituents independently from each other selected from the group consisting of Me, $^i$Pr, $^t$Bu, CN, $CF_3$, and Ph.

In a further embodiment of the invention, $R^b$ is at each occurrence independently from another selected from the group consisting of
Me,
$^t$Bu,
Ph, which is optionally substituted with one or more substituents independently from each other selected from the group consisting of Me, $^i$Pr, $^t$Bu, CN, $CF_3$, and Ph,
triazinyl, which is optionally substituted with one or more substituents independently from each other selected from the group consisting of Me, $^i$Pr, $^t$Bu, CN, $CF_3$, and Ph.

In the following, exemplary embodiments of the second chemical moiety are shown:

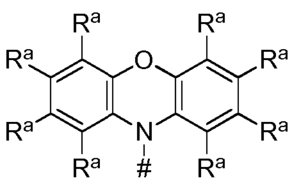
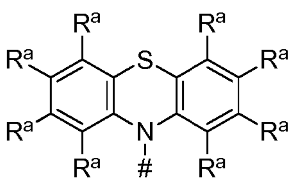
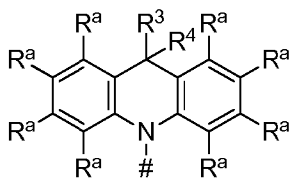
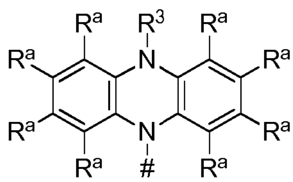

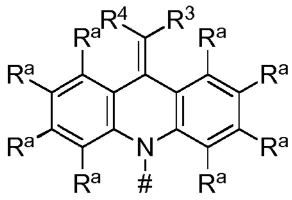
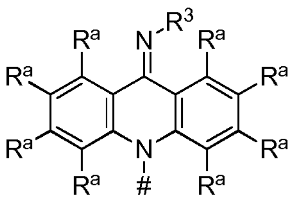
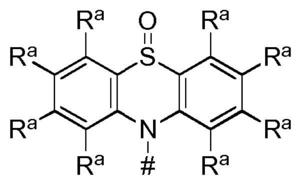

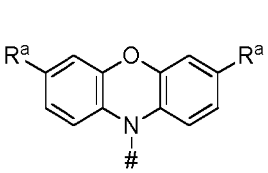
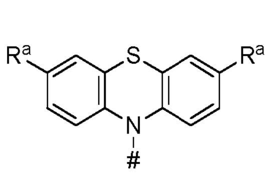
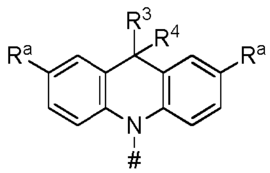
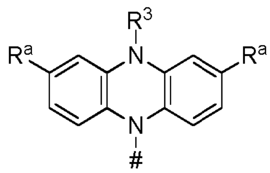

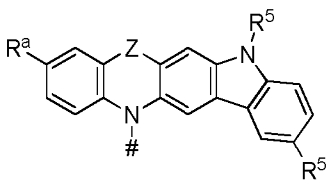
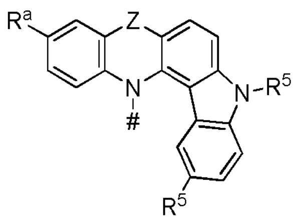
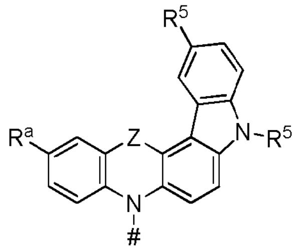

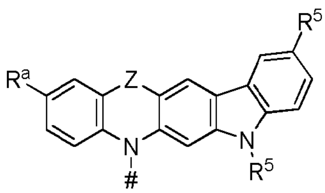
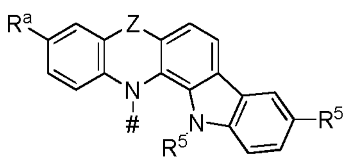
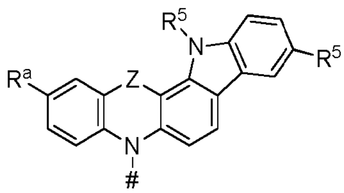

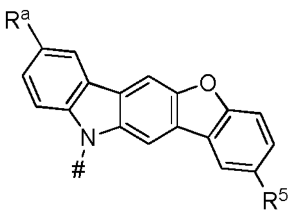
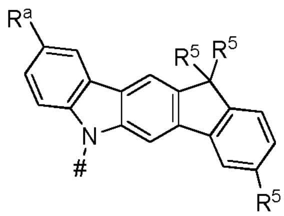
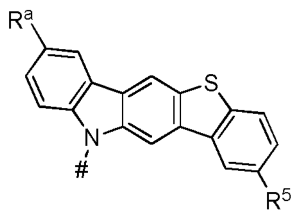

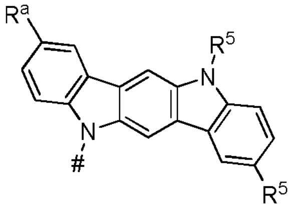

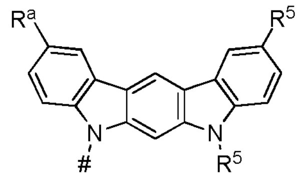
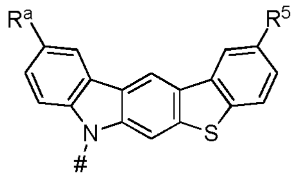
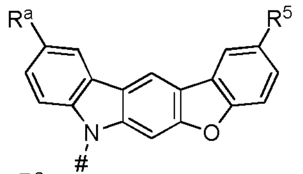
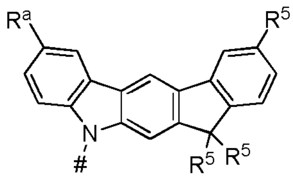
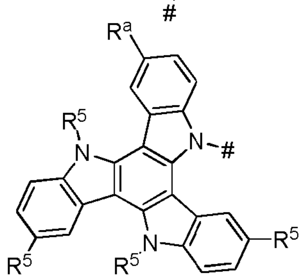
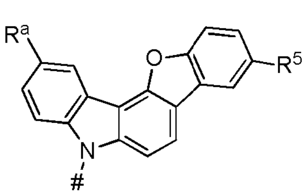
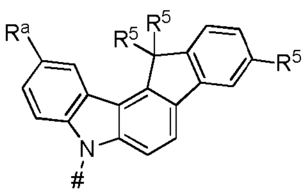
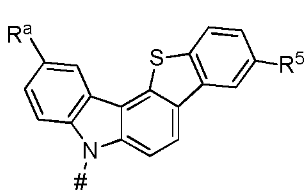
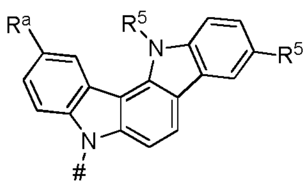
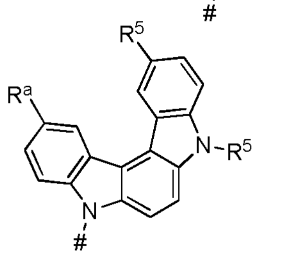
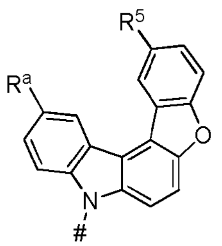
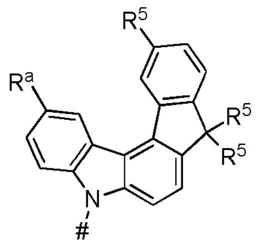
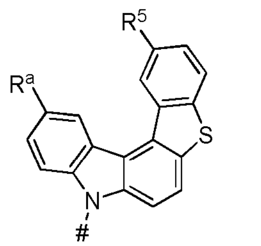
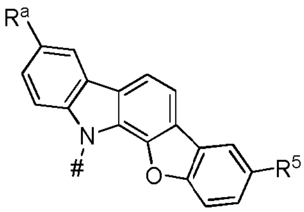
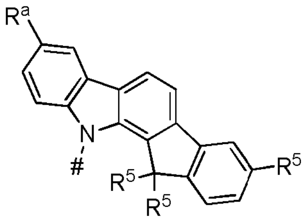
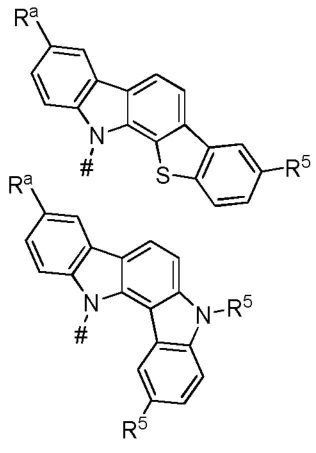
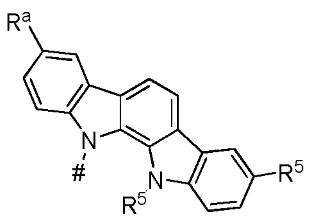

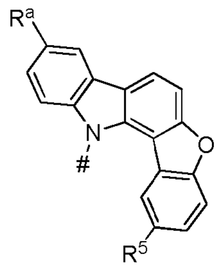

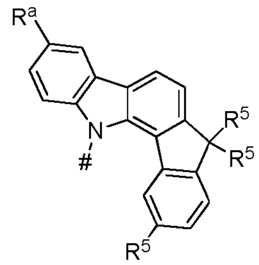

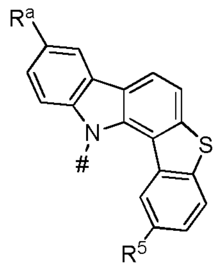

wherein for #, Z, $R^a$, $R^3$, $R^4$ and $R^5$ the aforementioned definitions apply.

In one embodiment, $R^a$ and $R^5$ is at each occurrence independently from another selected from the group consisting of hydrogen (H), methyl (Me), i-propyl ($CH(CH_3)_2$) ($^iPr$), t-butyl ($^tBu$), phenyl (Ph), CN, $CF_3$, and diphenylamine ($NPh_2$).

In one embodiment of the invention, the organic molecules comprise or consist of a structure of Formula III:

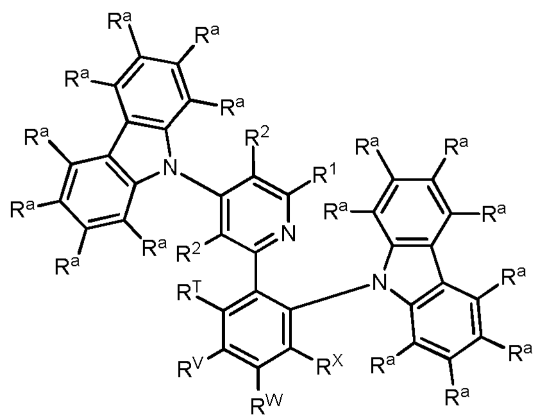

Formula III

wherein any one of the aforementioned definitions apply.

In a further embodiment of the invention, the organic molecules comprise or consist of a structure selected from the group of Formula III-1 to Formula III-12:

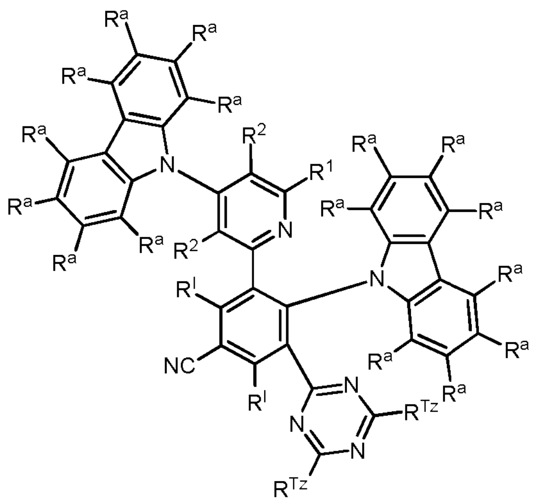

Formula III-1

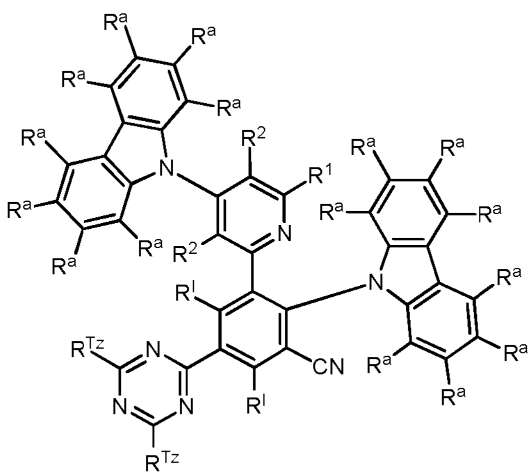

Formula III-2

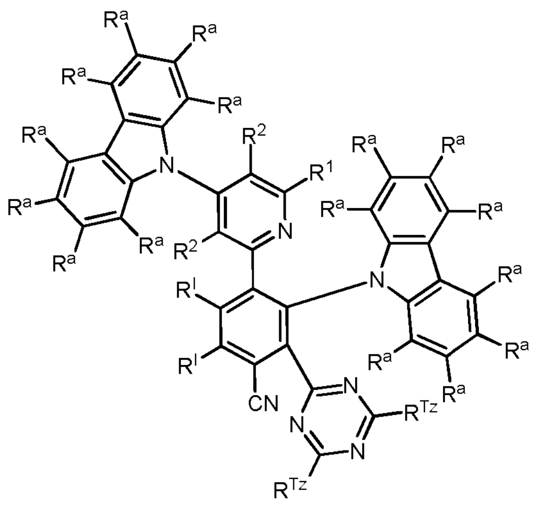

Formula III-3

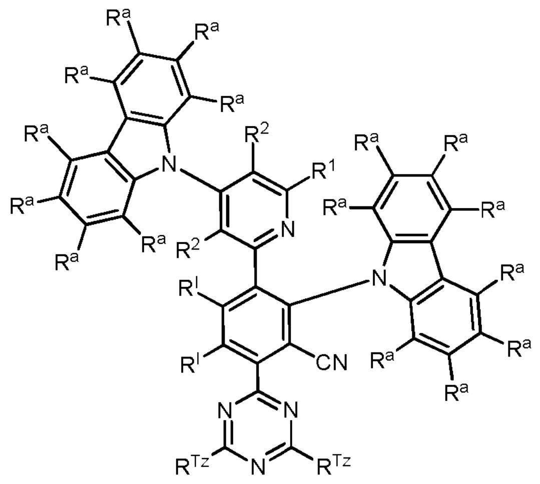

Formula III-4

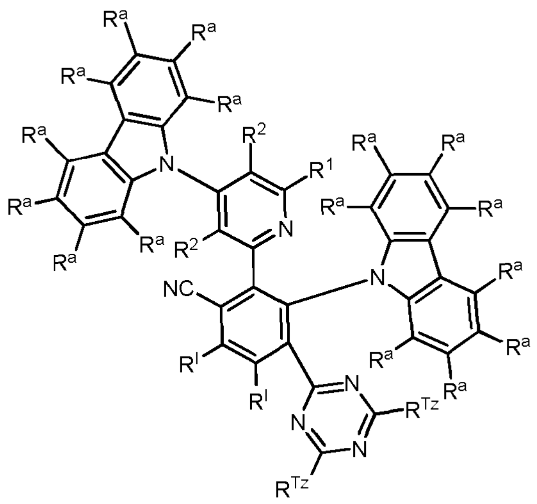

Formula III-5

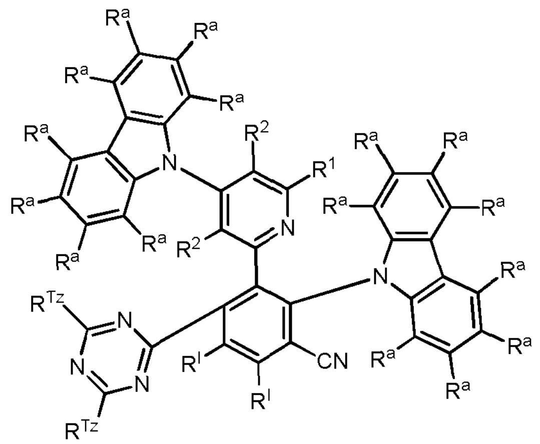

Formula III-6

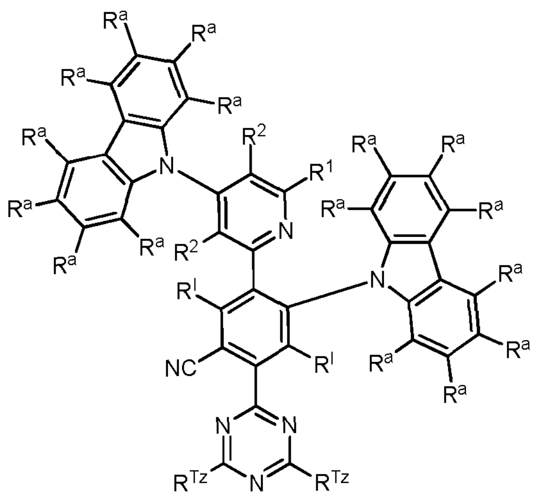

Formula III-7

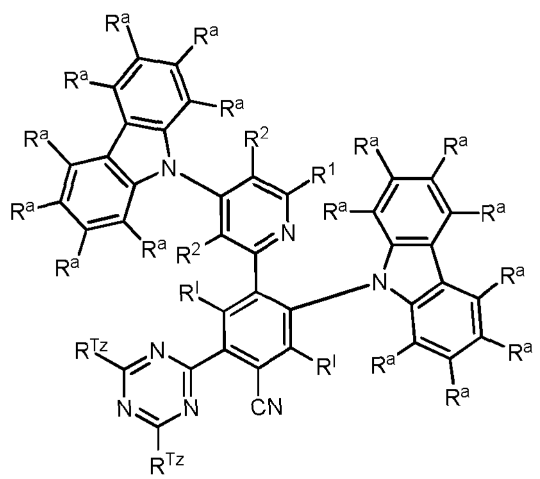

Formula III-8

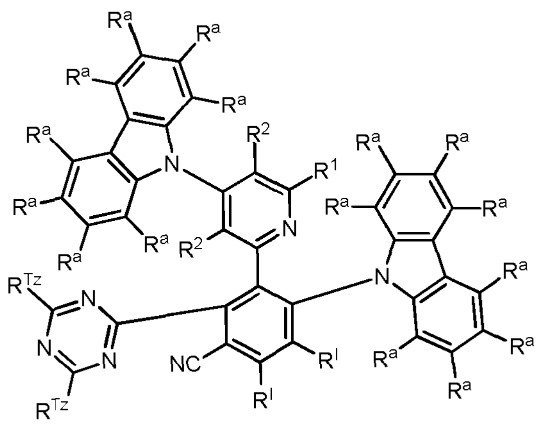

Formula III-9

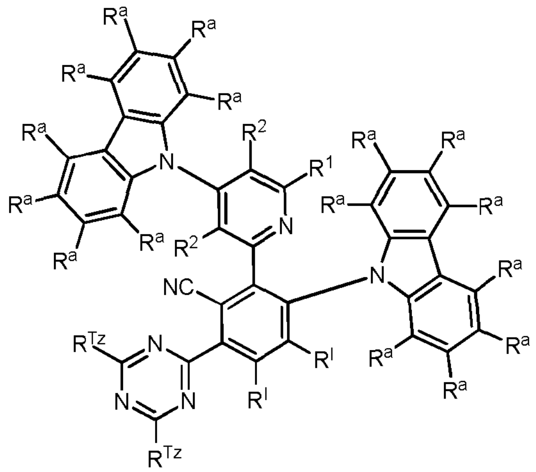

Formula III-10

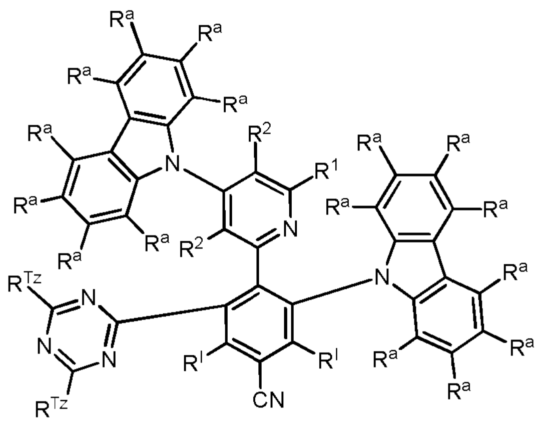

Formula III-11

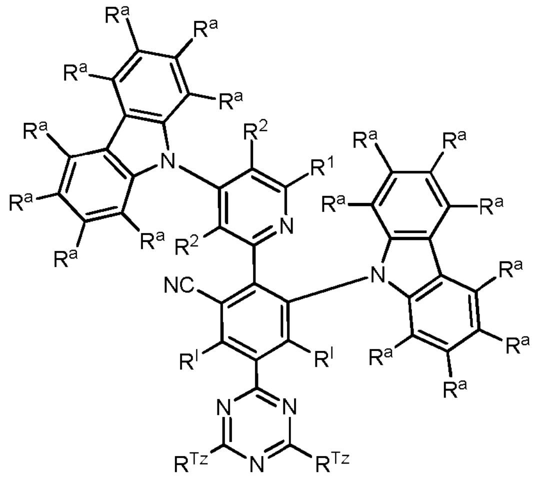

Formula III-12

wherein any one of the aforementioned definitions apply.

In a further embodiment of the invention, the organic molecules comprise or consist of a structure selected from the group of Formula III-1 and Formula III-2.

In a further embodiment of the invention, the organic molecules comprise or consist of a structure selected from the group of Formula IIIa-1 to Formula IIIa-12:

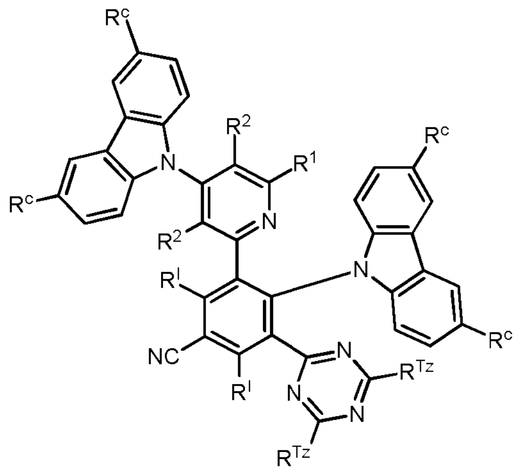

Formula IIIa-1

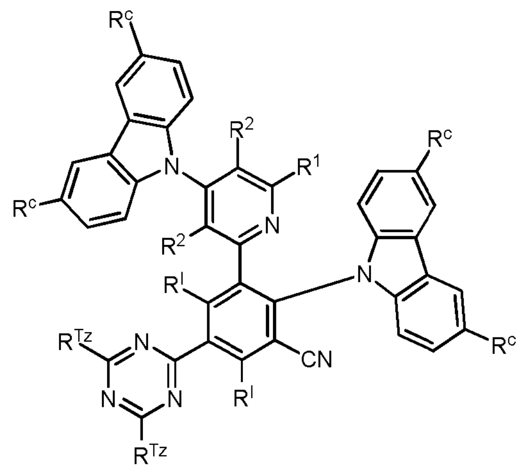

Formula IIIa-2

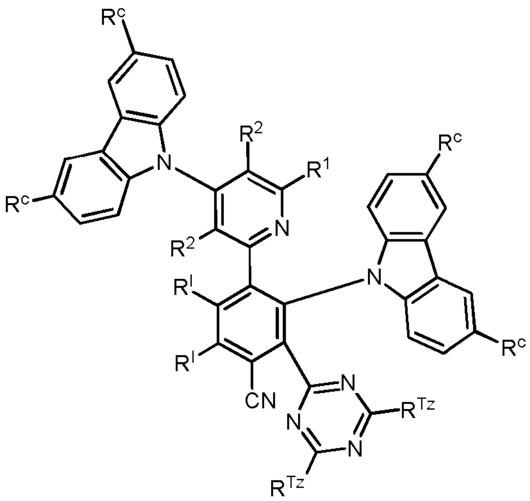

Formula IIIa-3

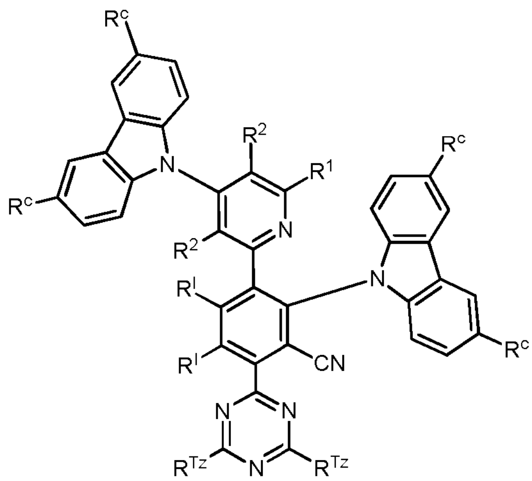

Formula IIIa-4

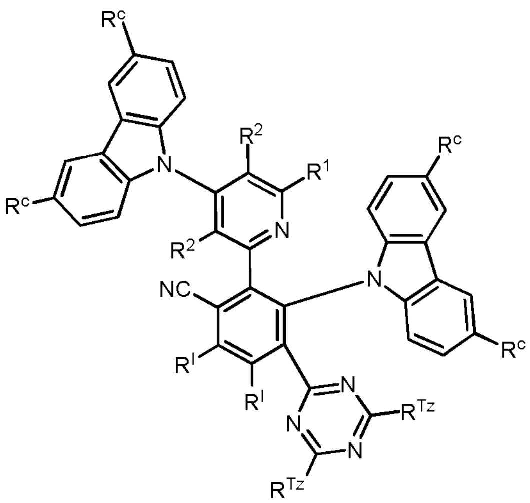

Formula IIIa-5

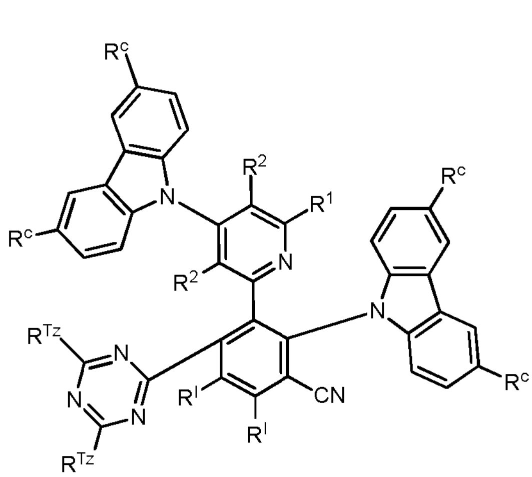

Formula IIIa-6

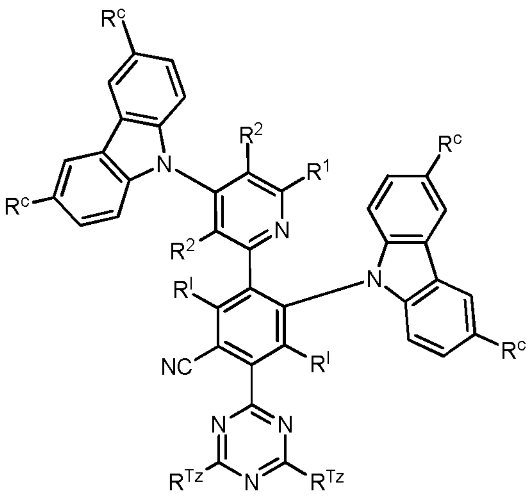

Formula IIIa-7

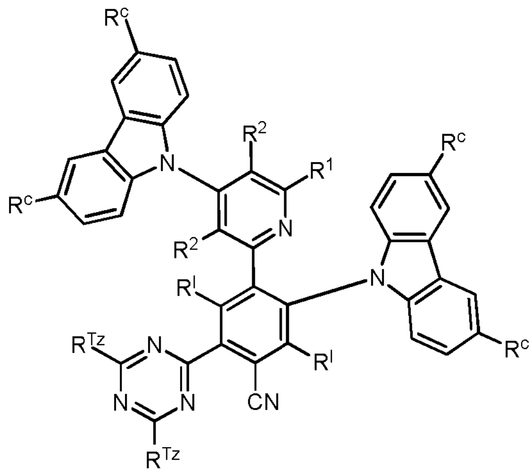

Formula IIIa-8

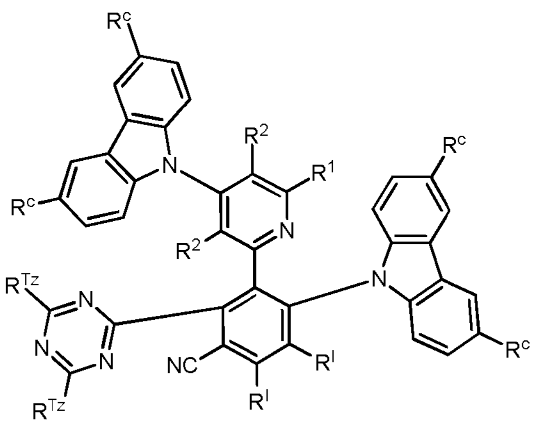

Formula IIIa-9

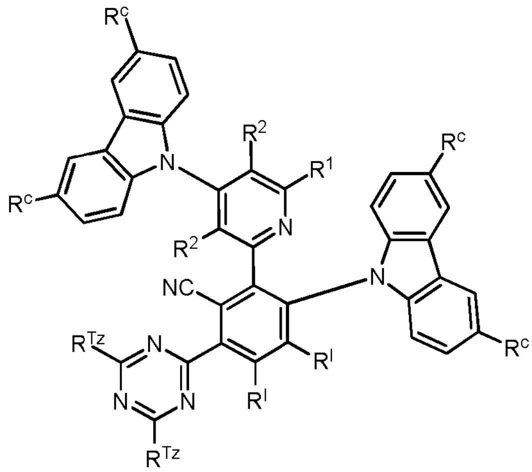

Formula IIIa-10

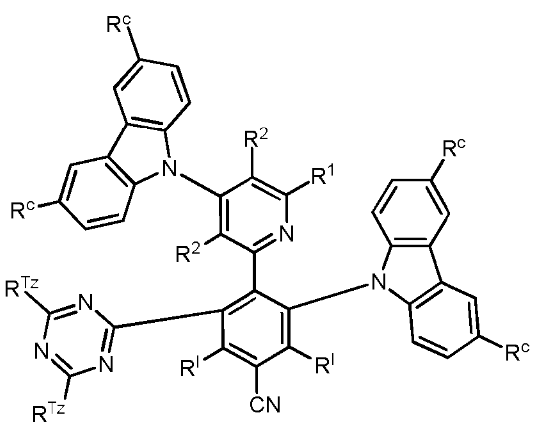

Formula IIIa-11

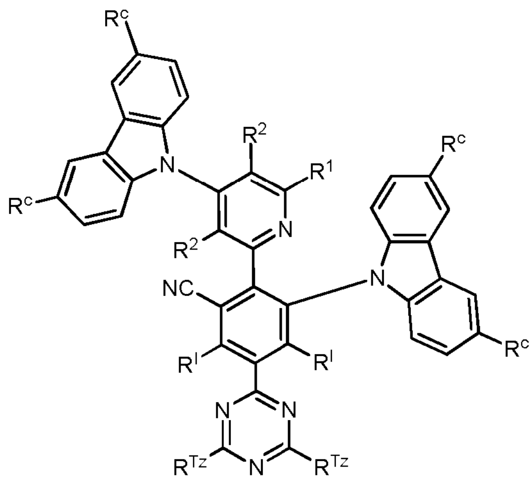

Formula IIIa-12

wherein

$R^c$ is at each occurrence independently from another selected from the group consisting of Me, $^iPr$,

$^t$Bu,
Ph, which is optionally substituted with one or more substituents independently from each other selected from the group consisting of Me, $^i$Pr, $^t$Bu, CN, $CF_3$, and Ph,
pyridinyl, which is optionally substituted with one or more substituents independently from each other selected from the group consisting of Me, $^i$Pr, $^t$Bu, CN, $CF_3$, and Ph,
pyrimidinyl, which is optionally substituted with one or more substituents independently from each other selected from the group consisting of Me, $^i$Pr, $^t$Bu, CN, $CF_3$, and Ph,
carbazolyl, which is optionally substituted with one or more substituents independently from each other selected from the group consisting of Me, $^i$Pr, $^t$Bu, CN, $CF_3$, and Ph,
triazinyl, which is optionally substituted with one or more substituents independently from each other selected from the group consisting of Me, $^i$Pr, $^t$Bu, CN, $CF_3$, and Ph,
and $N(Ph)_2$.

In a preferred embodiment of the invention, the organic molecules comprise or consist of a structure selected from the group of Formula IIIa-1 and Formula IIIa-2.

In a further embodiment of the invention, the organic molecules comprise or consist of a structure selected from the group of Formula IIIb-1 to Formula IIIb-12:

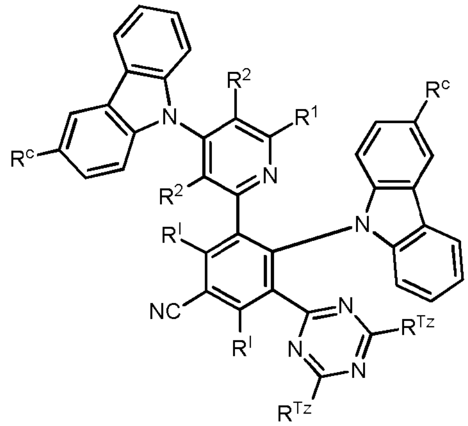

Formula IIIb-1

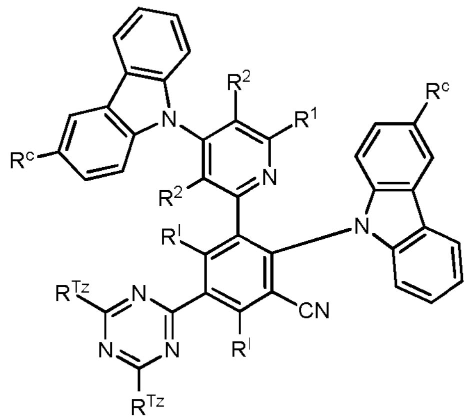

Formula IIIb-2

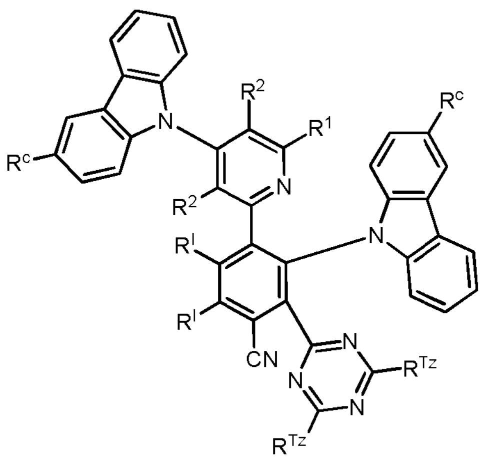

Formula IIIb-3

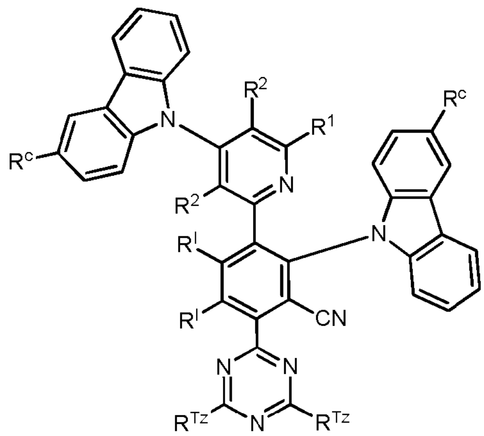

Formula IIIb-4

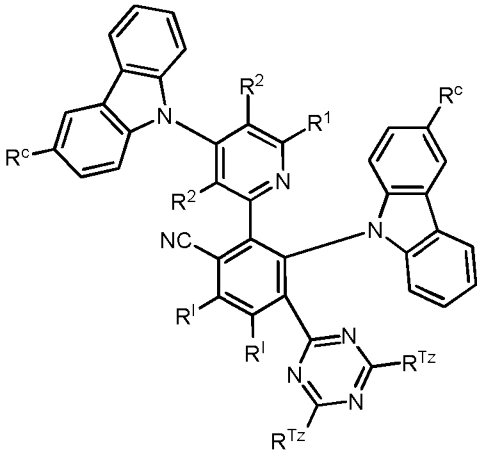

Formula IIIb-5

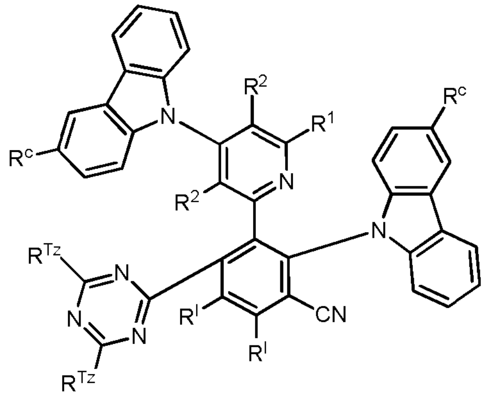

Formula IIIb-6

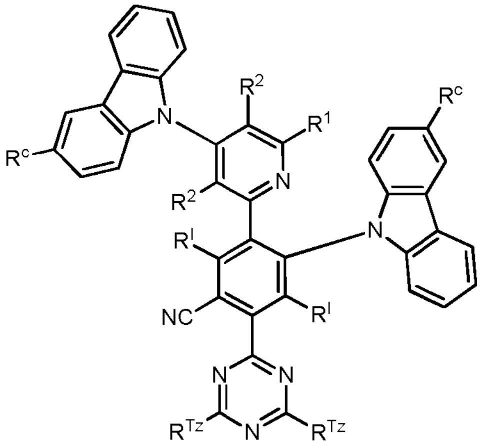

Formula IIIb-7

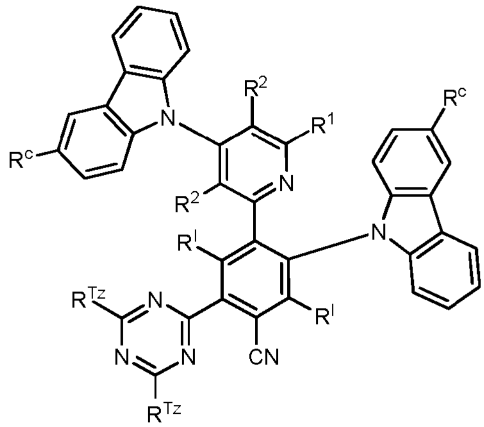

Formula IIIb-8

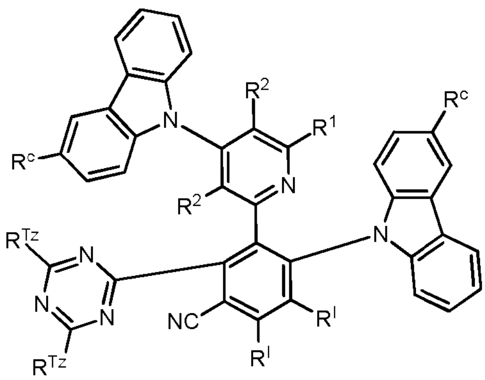

Formula IIIb-9

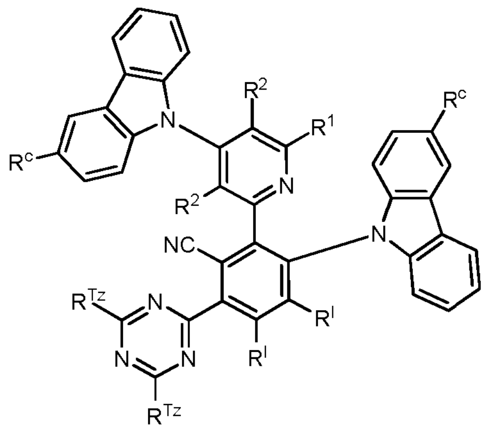

Formula IIIb-10

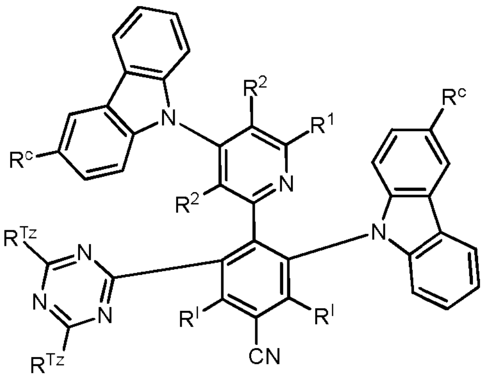

Formula IIIb-11

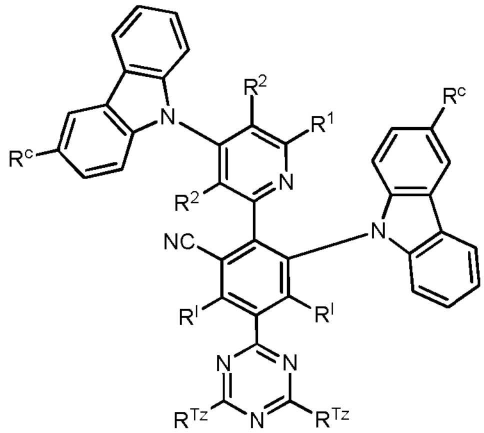

Formula IIIb-12

wherein the aforementioned definitions apply.

In a preferred embodiment of the invention, the organic molecules comprise or consist of a structure selected from the group of Formula IIIb-1 and Formula IIIb-2.

In one embodiment of the invention, the organic molecules comprise or consist of a structure of Formula IV:

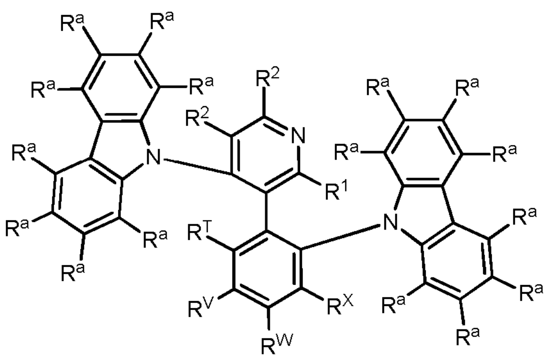

Formula IV

In a further embodiment of the invention, the organic molecules comprise or consist of a structure selected from the group of Formula IV-1 to Formula IV-12:

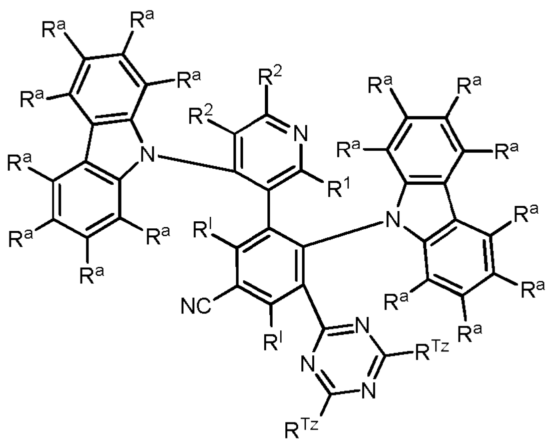

Formula IV-1

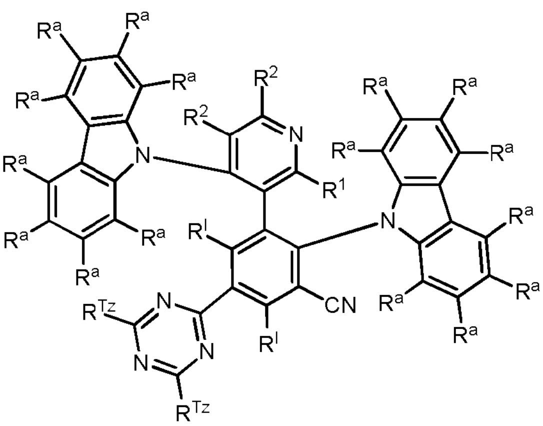

Formula IV-2

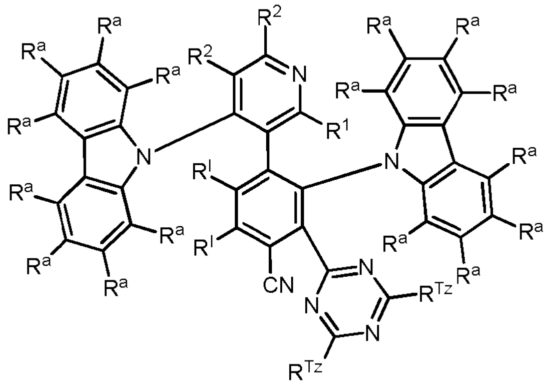

Formula IV-3

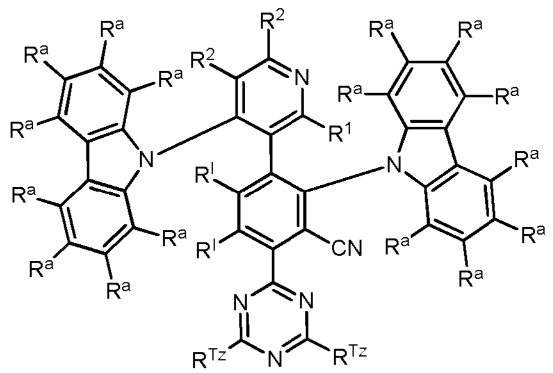

Formula IV-4

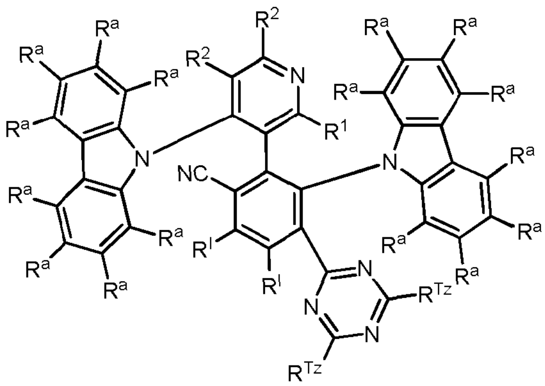

Formula IV-5

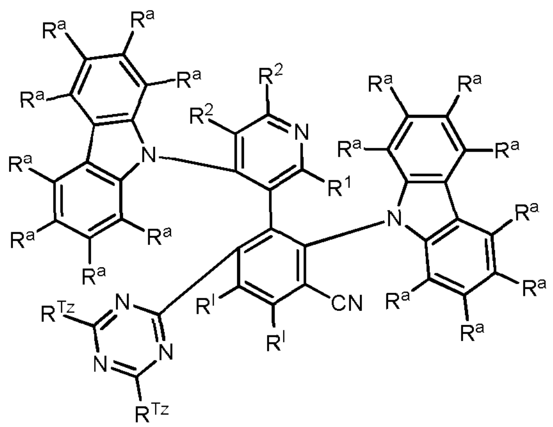

Formula IV-6

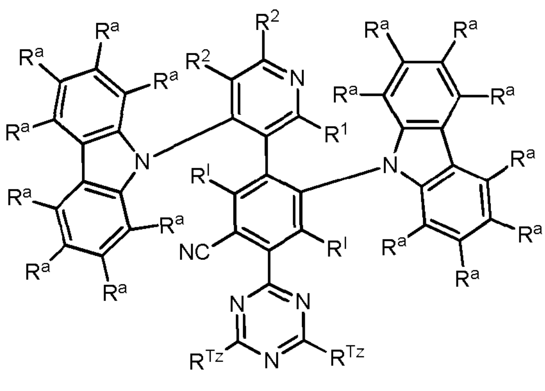

Formula IV-7

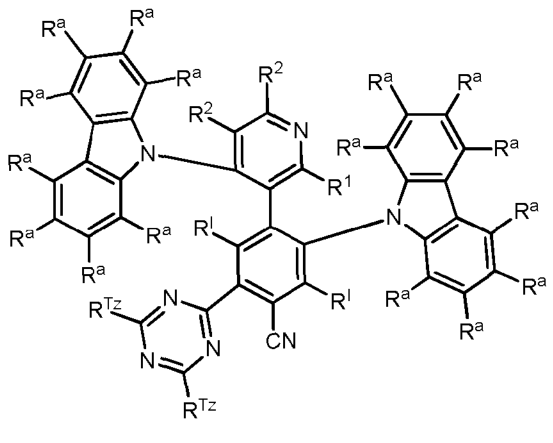

Formula IV-8

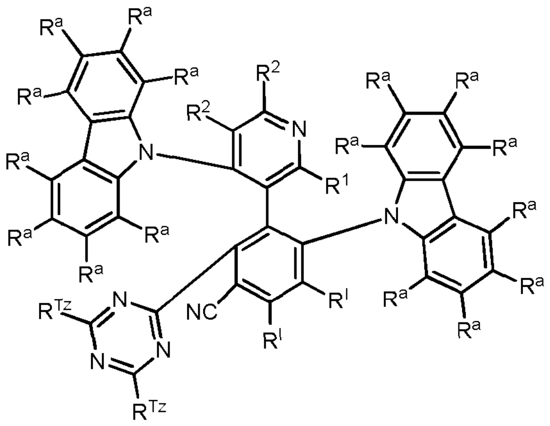

Formula IV-9

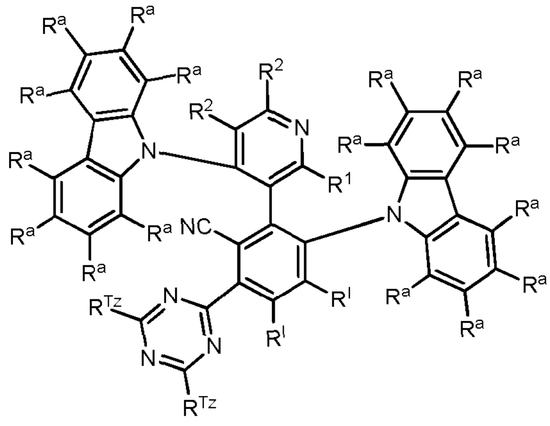

Formula IV-10

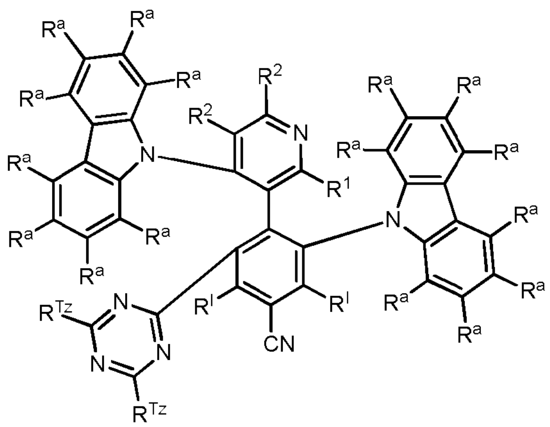

Formula IV-11

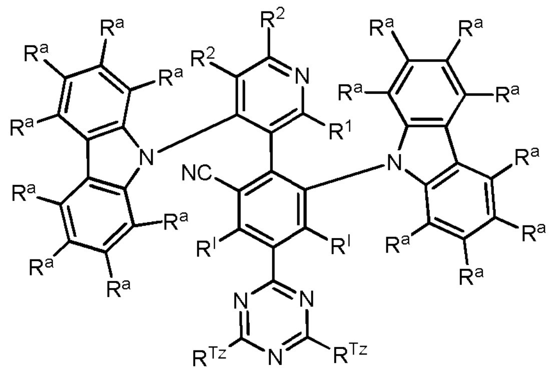

Formula IV-12

wherein any one of the aforementioned definitions apply.

In a preferred embodiment of the invention, the organic molecules comprise or consist of a structure selected from the group of Formula IV-1 and Formula IV-2.

In a further embodiment of the invention, the organic molecules comprise or consist of a structure selected from the group of Formula IVa-1 to Formula IVa-2:

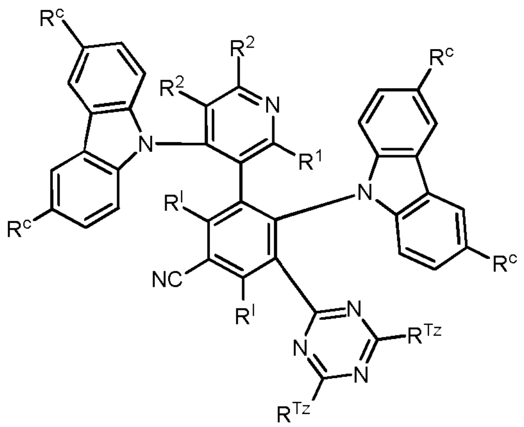

Formula IVa-1

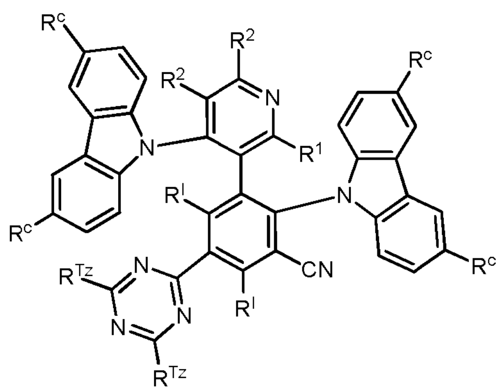

Formula IVa-2

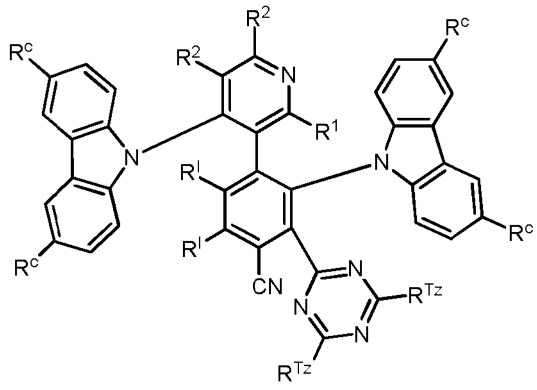

Formula IVa-3

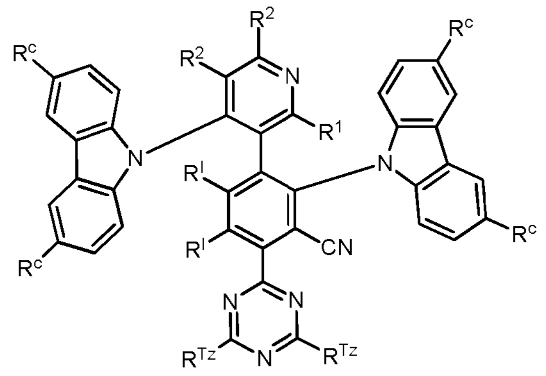

Formula IVa-4

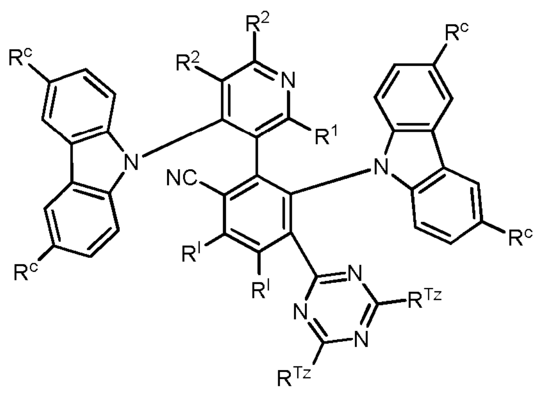

Formula IVa-5

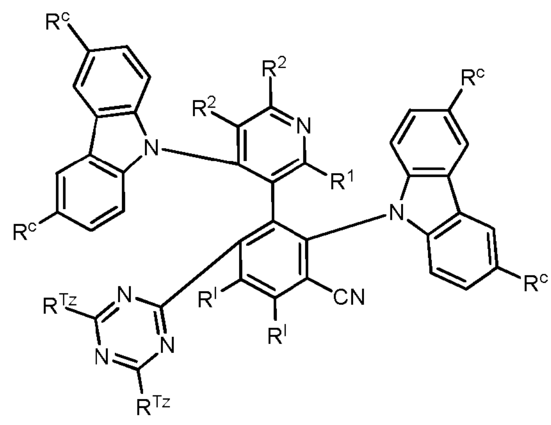

Formula IVa-6

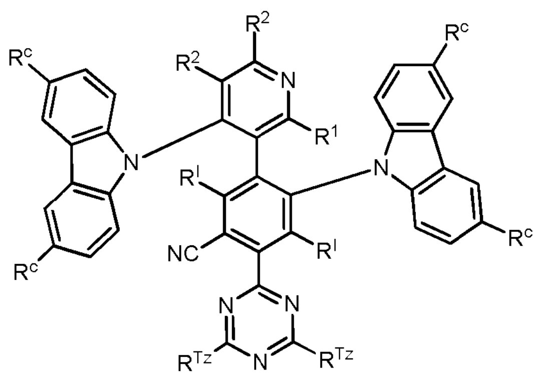

Formula IVa-7

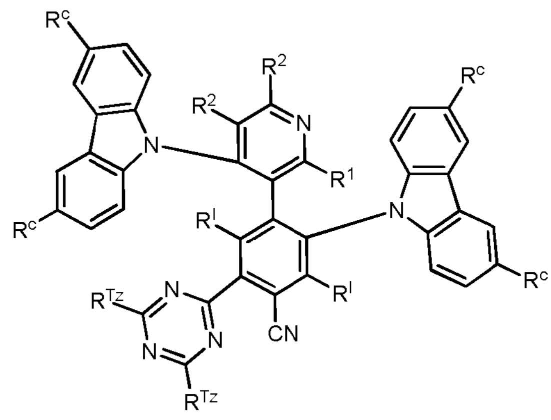

Formula IVa-8

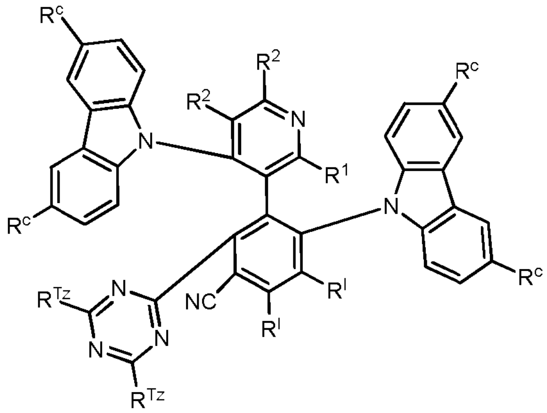

Formula IVa-9

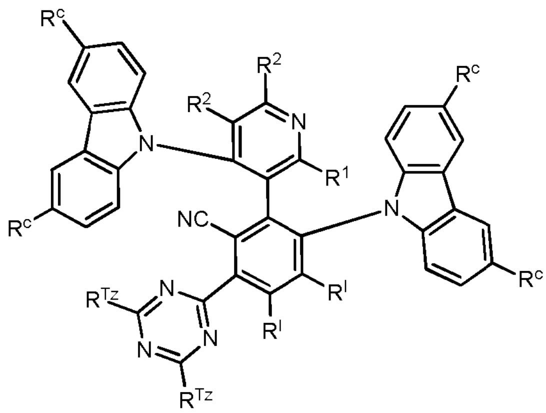

Formula IVa-10

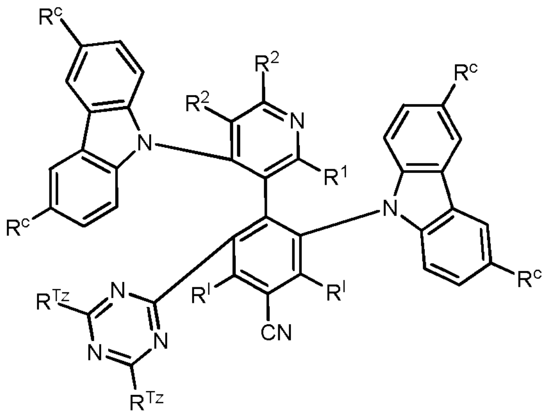

Formula IVa-11

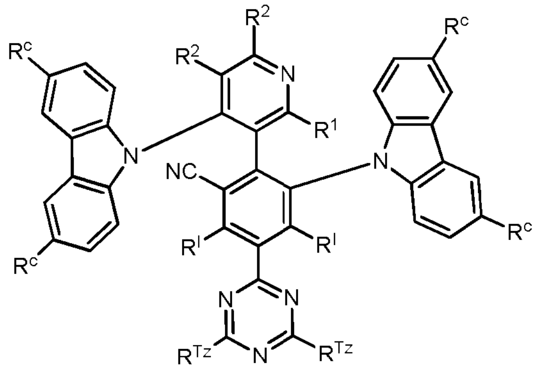

Formula IVa-12

wherein the aforementioned definitions apply.

In a preferred embodiment of the invention, the organic molecules comprise or consist of a structure selected from the group of Formula IVa-1 and Formula IVa-2.

In a further embodiment of the invention, the organic molecules comprise or consist of a structure selected from the group of Formula IVb-1 to Formula IVb-12:

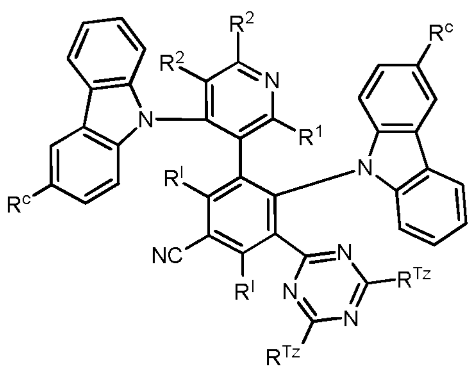

Formula IVb-1

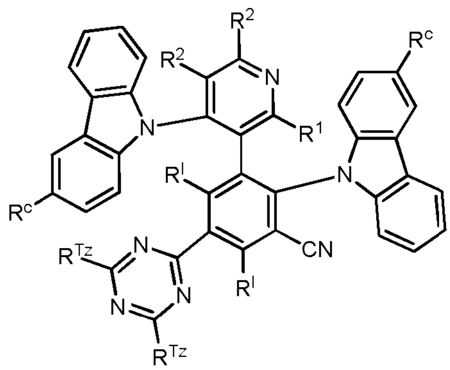

Formula IVb-2

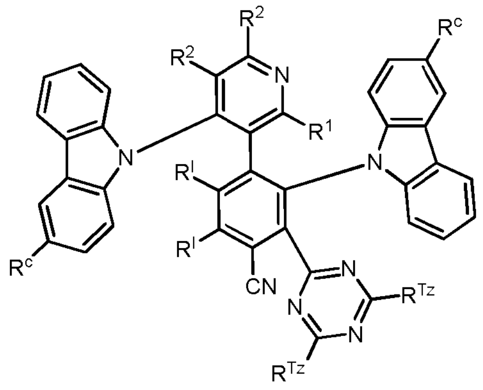

Formula IVb-3

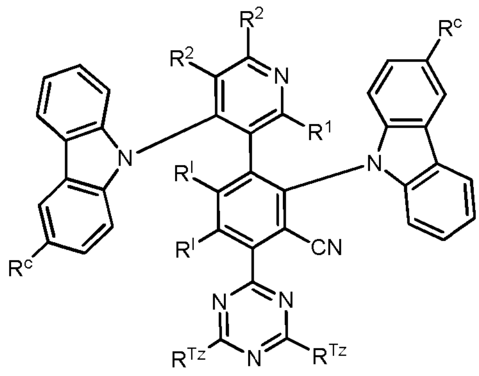

Formula IVb-4

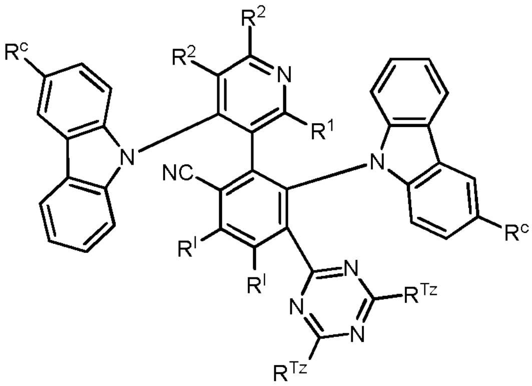

Formula IVb-5

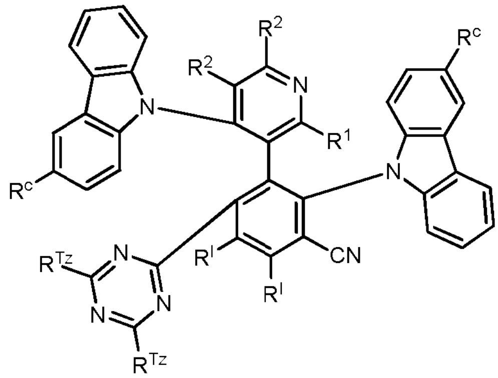

Formula IVb-6

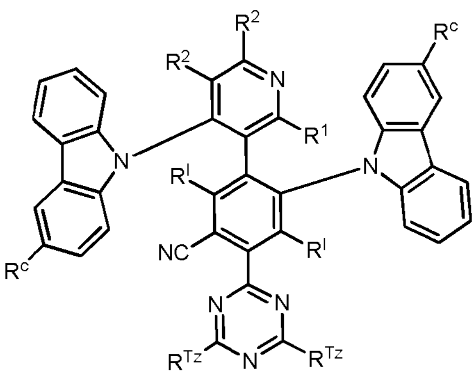

Formula IVb-7

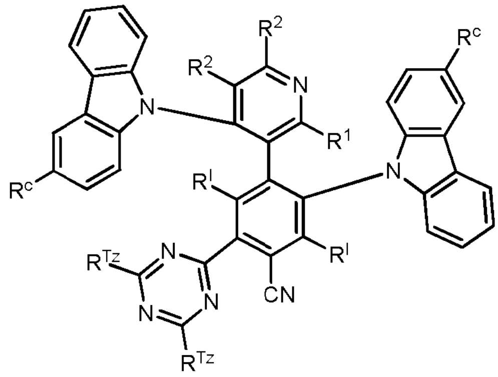

Formula IVb-8

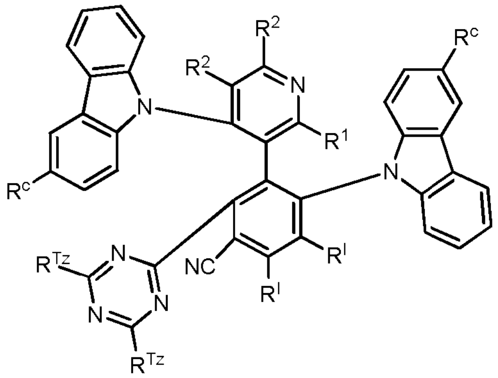

Formula IVb-9

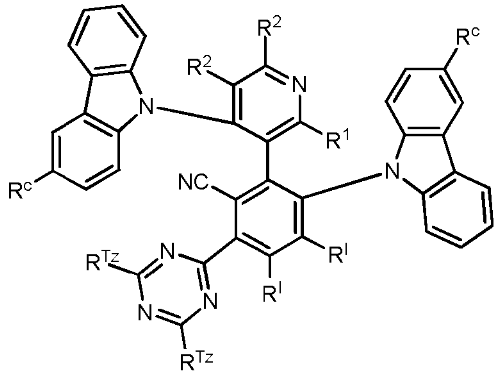

Formula IVb-10

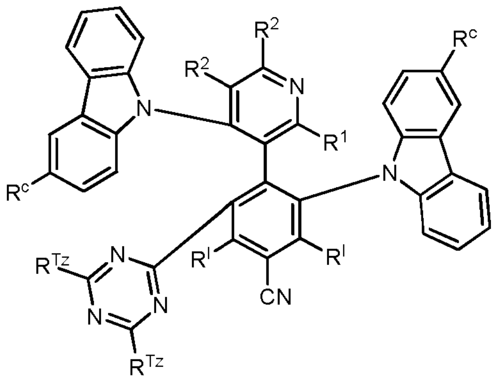

Formula IVb-11

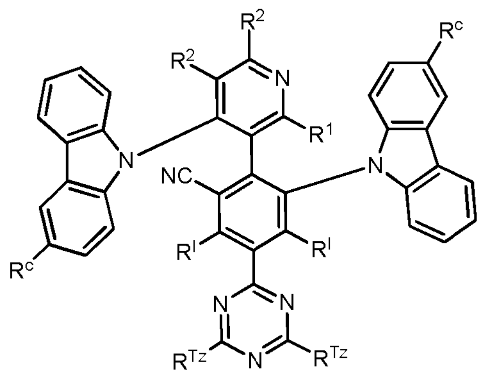

Formula IVb-12

wherein the aforementioned definitions apply.

In a preferred embodiment of the invention, the organic molecules comprise or consist of a structure selected from the group of Formula IVb-1 and Formula IVb-2.

In one embodiment of the invention, the organic molecules comprise or consist of a structure of Formula V:

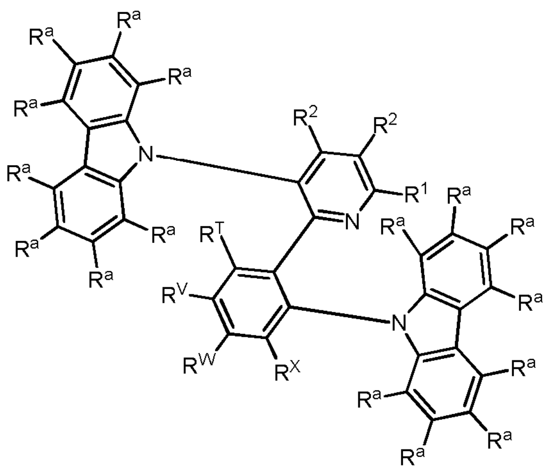

Formula V

wherein any one of the aforementioned definitions apply.

In a further embodiment of the invention, the organic molecules comprise or consist of a structure selected from the group of Formula V-1 and Formula V-2:

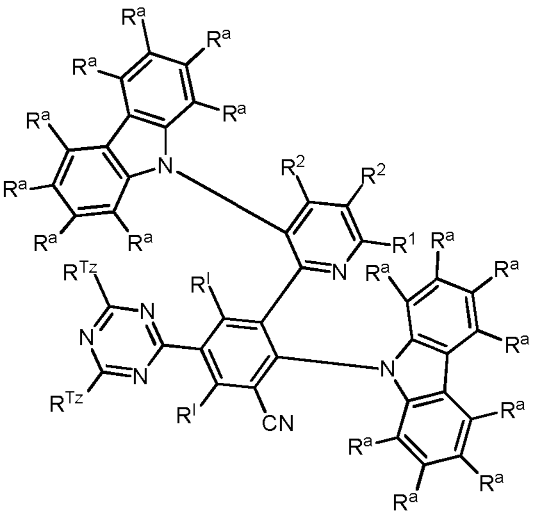

Formula V-1

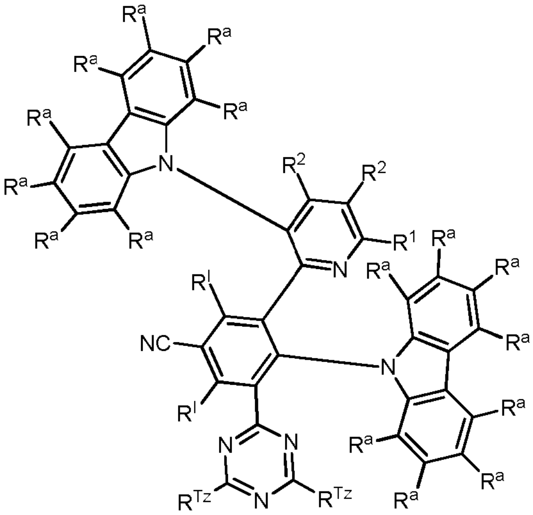

Formula V-2

wherein the aforementioned definitions apply.

In one embodiment of the invention, the organic molecules comprise or consist of a structure of Formula VI:

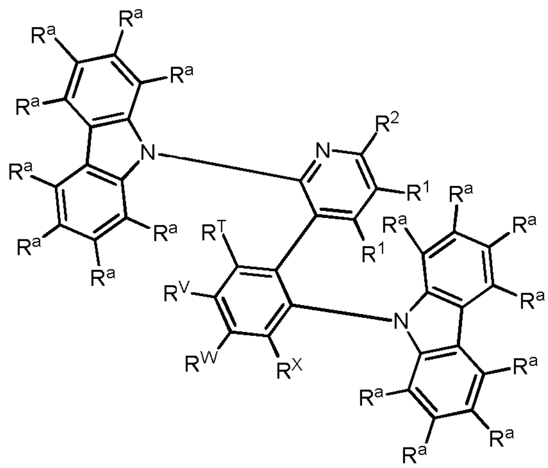

Formula VI

wherein any one of the aforementioned definitions apply.

In a further embodiment of the invention, the organic molecules comprise or consist of a structure selected from the group of Formula VI-1 and Formula VI-2:

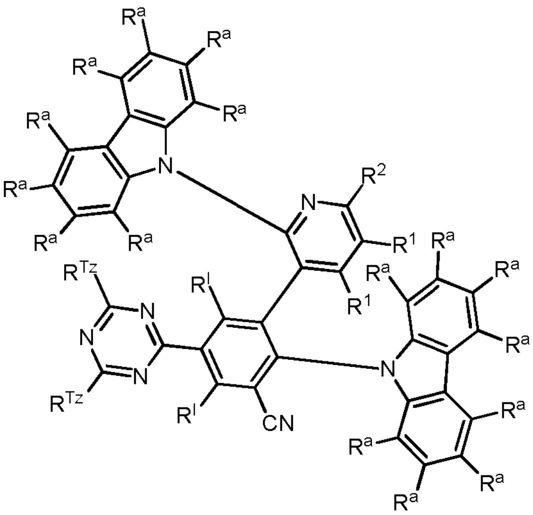

Formula VI-1

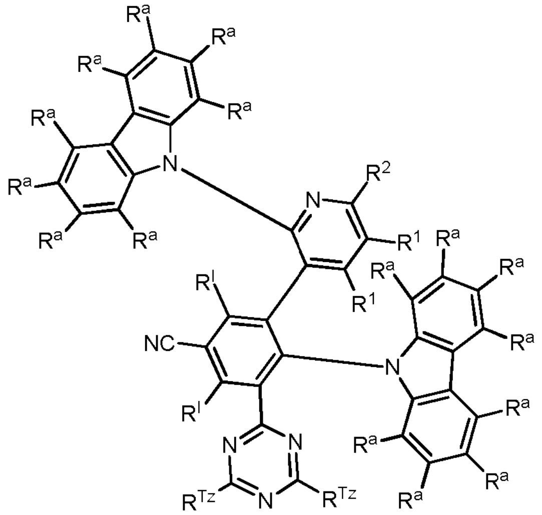

Formula VI-2

wherein the aforementioned definitions apply.

In one embodiment of the invention, the organic molecules comprise or consist of a structure of Formula VII:

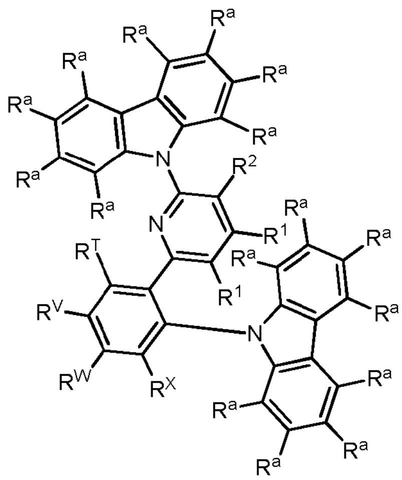

Formula VII

wherein any one of the aforementioned definitions apply.

In one embodiment of the invention, the organic molecules comprise or consist of a structure of Formula VIII:

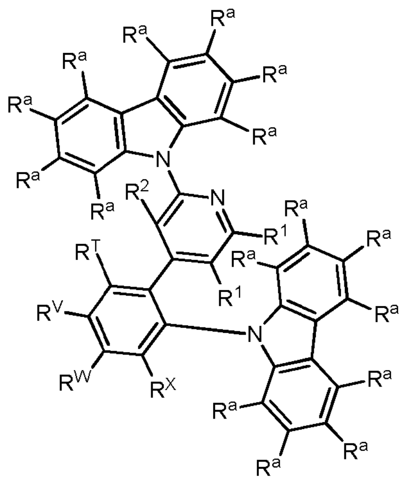

Formula VIII

wherein any one of the aforementioned definitions apply.

In one embodiment of the invention, the organic molecules comprise or consist of a structure of Formula IX:

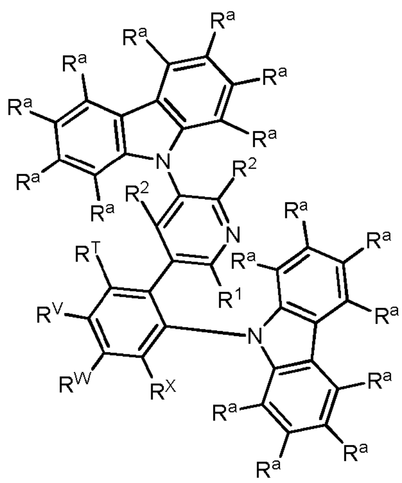

Formula IX

wherein any one of the aforementioned definitions apply.

In one embodiment of the invention, the organic molecules comprise or consist of a structure of Formula X:

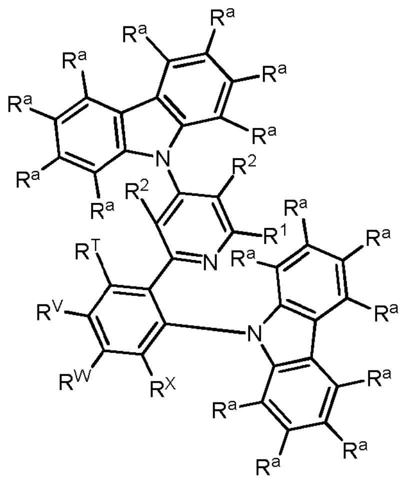

Formula X

wherein any one of the aforementioned definitions apply.

In one embodiment of the invention, the organic molecules comprise or consist of a structure of Formula XI:

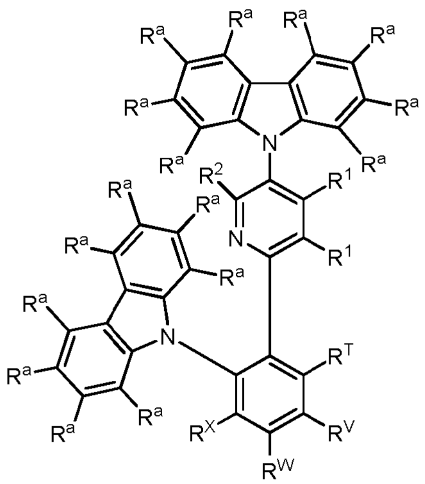

Formula XI

wherein any one of the aforementioned definitions apply.

In one embodiment of the invention, the organic molecules comprise or consist of a structure of Formula XII:

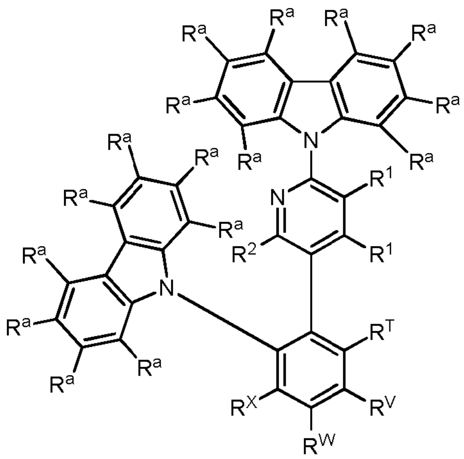

Formula XII

wherein any one of the aforementioned definitions apply.

In one embodiment of the invention, the organic molecules comprise or consist of a structure of Formula XIII:

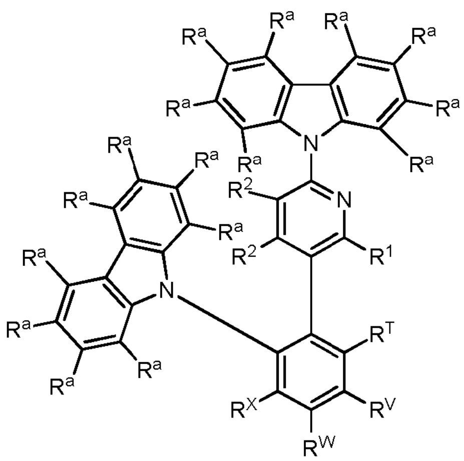

Formula XIII

wherein any one of the aforementioned definitions apply.

In one embodiment of the invention, the organic molecules comprise or consist of a structure of Formula XIV:

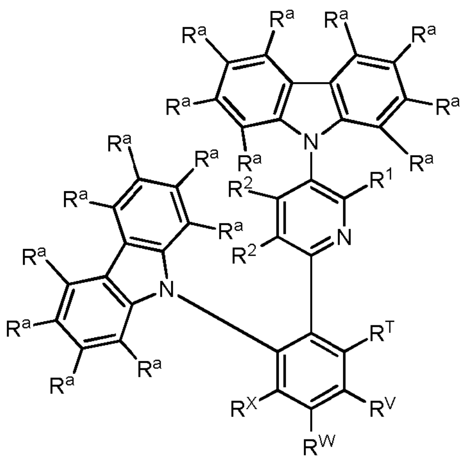

Formula XIV

wherein any one of the aforementioned definitions apply.

In one embodiment of the invention, the organic molecules comprise or consist of a first chemical moiety comprising or consisting of a structure of Formula A1:

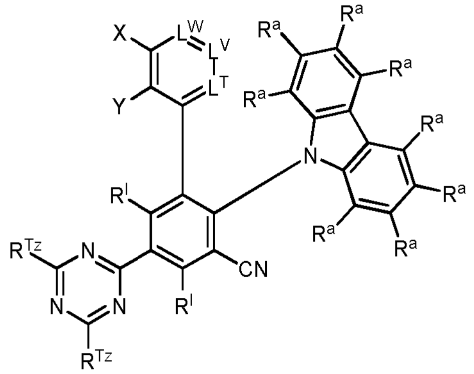

Formula A1

and

- one second chemical moiety, comprising or consisting of a structure of Formula II,

wherein the first chemical moiety is linked to the second chemical moiety via a single bond, and
wherein the aforementioned definitions apply, and
wherein exactly one ring member selected from the group consisting of $L^T$, $L^V$ and $L^W$ is N, and exactly one substituent selected from the group consisting of W, Y and X represents the binding site of a single bond linking the first chemical moiety and the second chemical moiety.

In a further embodiment, the organic molecules comprise or consist of a first chemical moiety comprising or consisting of a structure of Formula A1 and Y represents the binding site of a single bond linking the first chemical moiety and the second chemical moiety.

In a further embodiment, the organic molecules comprise or consist of a first chemical moiety comprising or consisting of a structure of Formula A1 and X represents the binding site of a single bond linking the first chemical moiety and the second chemical moiety.

In one embodiment of the invention, the organic molecules comprise or consist of a first chemical moiety comprising or consisting of a structure of Formula B1:

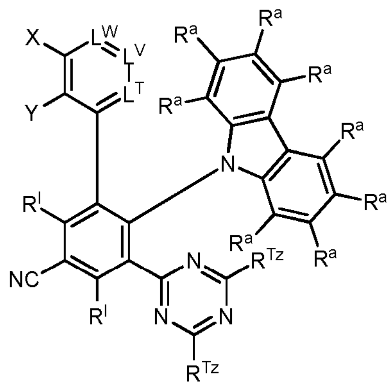

Formula B1

and

- one second chemical moiety, comprising or consisting of a structure of Formula II,

wherein the first chemical moiety is linked to the second chemical moiety via a single bond, and
wherein the aforementioned definitions apply, and
wherein exactly one ring member selected from the group consisting of $L^T$, $L^V$ and $L^W$ is N, and exactly one substituent selected from the group consisting of W, Y and X represents the binding site of a single bond linking the first chemical moiety and the second chemical moiety.

In a further embodiment, the organic molecules comprise or consist of a first chemical moiety comprising or consisting of a structure of Formula B1 and X represents the binding site of a single bond linking the first chemical moiety and the second chemical moiety.

In a further embodiment, the organic molecules comprise or consist of a first chemical moiety comprising or consisting of a structure of Formula B1 and Y represents the binding site of a single bond linking the first chemical moiety and the second chemical moiety.

In one embodiment of the invention $R^c$ is at each occurrence independently from another selected from the group consisting of
H,
Me,
$^i$Pr,
$^t$Bu,
Ph, which is optionally substituted with one or more substituents independently from each other selected from the group consisting of Me, $^i$Pr, $^t$Bu, CN, $CF_3$ and Ph; and
triazinyl, which is optionally substituted with one or more substituents independently from each other selected from the group consisting of Me, $^i$Pr, $^t$Bu, CN, $CF_3$ and Ph.

In one embodiment of the invention $R^c$ is at each occurrence independently from another selected from the group consisting of

Me,
$^i$Pr,
$^t$Bu,
Ph, which is optionally substituted with one or more substituents independently from each other selected from the group consisting of Me, $^i$Pr, $^t$Bu, CN, $CF_3$ and Ph; and
triazinyl, which is optionally substituted with one or more substituents independently from each other selected from the group consisting of Me, $^i$Pr, $^t$Bu, CN, $CF_3$ and Ph.

The following describes preferred subgroups (subgroup α, subgroup β, subgroup γ, subgroup δ) of features. Firstly, two preferred variants for the first chemical moiety are defined (subgroup α, subgroup β) thereafter two preferred variants for the second chemical moiety (subgroup γ, subgroup δ) are described. The further preferred restrictions to individual substituents as described throughout herein are applicable to each of these subgroup variants unless defined specifically otherwise.

In a first preferred feature subgroup α of the present invention, $L^T$ is N or C-$R^1$;
$L^V$ is N or C-$R^1$;
$L^W$ is N or C-W; wherein W is $R^2$;
X is the binding site of a single bond linking the first chemical moiety to one of the two second chemical moieties or is $R^2$;

Y is the binding site of a single bond linking the first chemical moiety to one of the two second chemical moieties or is $R^2$;

Additionally, it is preferred for $R^1$ and $R^2$ in the subgroup $\alpha$ to be selected from the group consisting of H, methyl and phenyl.

Additionally, it is preferred for Y to be the binding site of a single bond linking the first chemical moiety to one of the two second chemical moieties when $L^V$ is N.

Additionally, it is preferred for X to be the binding site of a single bond linking the first chemical moiety to one of the two second chemical moieties when $L^T$ is N.

Furthermore, it is preferred for the organic molecule of subgroup $\alpha$ to include the substituent $R^I$, wherein $R^I$ is selected from the group consisting of H, methyl and phenyl.

Additionally, it is even more preferred for $R^1$, $R^2$, $R^I$ the subgroup $\alpha$ to be H at each occurrence.

Further, in view of subgroup $\alpha$, $R^{Tz}$ is preferably Ph, which is optionally substituted with one or more substituents $R^5$.

Further, in view of subgroup $\alpha$, $R^{Tz}$ is preferably Ph, which is optionally substituted with one or more substituents $R^6$.

Further, in view of subgroup $\alpha$, $R^{Tz}$ is Ph at each occurrence.

Additionally, in view of subgroup $\alpha$, the exactly one of the two second chemical moieties, which is attached to exactly one substituent selected from group consisting of W, Y and X comprises or consists of a structure of Formula IId:

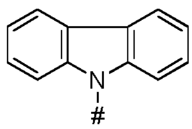

Formula IId

Chemical molecules of subgroup $\alpha$ as described herein provide an emission maximum in the blue/green-range and provide a high photoluminescence quantum yield (PLQY).

In one embodiment, the organic molecules according to the invention have an excited state lifetime of not more than 150 $\mu$s, of not more than 100 $\mu$s, in particular of not more than 50 $\mu$s, more preferably of not more than 10 $\mu$s or not more than 7 $\mu$s in a film of poly(methyl methacrylate) (PMMA) with 10 % by weight of organic molecule at room temperature.

Furthermore, in an even more preferred subgroup $\beta$ of the present invention, $R^T$ and $R^W$ is $R^I$ at each occurrence, wherein $R^I$ is selected from the group consisting of H, methyl and phenyl.

Additionally, in the more preferred subgroup $\beta$, exactly one substituent selected from the group consisting of $R^V$ and $R^X$ is CN and exactly one substituent selected from the group consisting of $R^V$ and $R^X$ is 1,3,5-triazinyl, which is substituted with two substituents $R^{Tz}$, wherein $R^{Tz}$ is preferably Ph, which is optionally substituted with one or more substituents $R^5$.

Additionally, in view of subgroup $\beta$, it is more preferred that the two second chemical moieties each at each occurrence independently from another comprise or consist of a structure of Formula IIa:

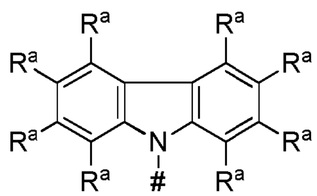

Formula IIa

Additionally, in view of subgroup $\beta$, it is even more preferred that $L^T$ is N or C-$R^1$;
$L^V$ is N or C-$R^1$;
$L^W$ is N or C-W; wherein W is $R^2$;
X is the binding site of a single bond linking the first chemical moiety to one of the two second chemical moieties or is $R^2$;
Y is the binding site of a single bond linking the first chemical moiety to one of the two second chemical moieties or is $R^2$;
Additionally, it is preferred for $R^1$, $R^2$ and $R^I$ in the subgroup $\beta$ to be selected from the group consisting of H, methyl and phenyl.

Further, in view of subgroup $\beta$, $R^{Tz}$ is preferably Ph, which is optionally substituted with one or more substituents $R^6$.

Additionally, in view of subgroup β, it is even more preferred that $R^{Tz}$ is Ph at each occurrence.

Hereby, the organic substances of subgroup β show an even more increased photoluminescence quantum yield compared to comparable organic molecules of the state of the art and provide an emission maximum in the blue/green-range.

In another preferred subgroup γ of the first aspect of the present invention, it is preferred for Z to be a direct bond at each occurrence.

Furthermore, in subgroup γ, it is preferred, that at least one of the two second chemical moieties each at each occurrence independently from another comprise or consist of a structure of Formula IIb:

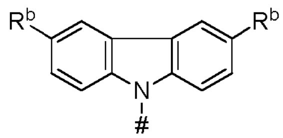

Formula IIb

Furthermore, in subgroup γ, it is preferred, that at least one of the two second chemical moieties each at each occurrence independently from another comprise or consist of a structure of Formula IIc:

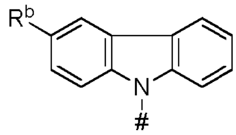

Formula IIc

Furthermore, in subgroup γ, it is preferred for the one second chemical moiety attached to the binding site Y to be different to the two further second chemical moieties, which are identical to each other.

Additionally, in view of subgroup γ, it is preferred that $R^b$ is at each occurrence independently from another selected from the group consisting of

Me,
$^i$Pr,
$^t$Bu,
CN,
$CF_3$,
Ph, which is optionally substituted with one or more substituents independently from each other selected from the group consisting of Me, $^i$Pr, $^t$Bu, CN, $CF_3$, and Ph,
pyridinyl, which is optionally substituted with one or more substituents independently from each other selected from the group consisting of Me, $^i$Pr, $^t$Bu, CN, $CF_3$, and Ph,
pyrimidinyl, which is optionally substituted with one or more substituents independently from each other selected from the group consisting of Me, $^i$Pr, $^t$Bu, CN, $CF_3$, and Ph, and
triazinyl, which is optionally substituted with one or more substituents independently from each other selected from the group consisting of Me, $^i$Pr, $^t$Bu, CN, $CF_3$, and Ph.

In an additional subgroup δ selected according to the present invention, it is especially preferred for Z to be a direct bond in all of the three chemical moieties of the organic molecule of the invention.

Furthermore, in subgroup δ, it is preferred for the two second chemical moieties to be different to each other.

Furthermore, in subgroup δ, it is even more preferred that the exactly one of the two second chemical moieties, which is attached to exactly one substituent selected from group consisting of W, Y and X comprises or consists of a structure of Formula IId:

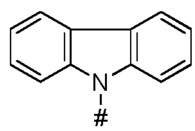

Formula IId

Furthermore, in subgroup δ, it is even more preferred that the exactly one of the two second chemical moieties,

which is attached to exactly one substituent selected from group consisting of W, Y and X comprises or consists of a structure of Formula IId:

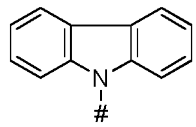

Formula IId

and the second of the two second chemical moieties comprise or consist of a structure of Formula IIc:

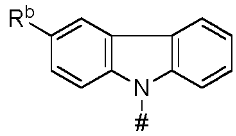

Formula IIc

wherein $R^b$ is at each occurrence independently from another selected from the group consisting of
Me,
$^tBu$,
Ph, which is optionally substituted with one or more substituents independently from each other selected from the group consisting of Me, $^iPr$, $^tBu$, CN, $CF_3$, and Ph,
triazinyl, which is optionally substituted with one or more substituents independently from each other selected from the group consisting of Me, $^iPr$, $^tBu$, CN, $CF_3$, and Ph.

Chemical molecules of the above subgroups $\gamma$ and $\delta$ hereby provide an emission maximum in the blue/green-range and further increase the photoluminescence quantum yield (PLQY).

As used throughout the present application, the terms "aryl" and "aromatic" may be understood in the broadest sense as any mono-, bi- or polycyclic aromatic moieties. Accordingly, an aryl group contains 6 to 60 aromatic ring atoms, and a heteroaryl group contains 5 to 60 aromatic ring atoms, of which at least one is a heteroatom. Notwithstanding, throughout the application the number of aromatic ring atoms may be given as subscripted number in the definition of certain substituents. In particular, the heteroaromatic ring includes one to three heteroatoms. Again, the terms "heteroaryl" and "heteroaromatic" may be understood in the broadest sense as any mono-, bi- or polycyclic hetero-aromatic moieties that include at least one heteroatom. The heteroatoms may at each occurrence be the same or different and be individually selected from the group consisting of N, O and S. Accordingly, the term "arylene" refers to a divalent substituent that bears two binding sites to other molecular structures and thereby serving as a linker structure. In case, a group in the exemplary embodiments is defined differently from the definitions given here, for example, the number of aromatic ring atoms or number of heteroatoms differs from the given definition, the definition in the exemplary embodiments is to be applied. According to the invention, a condensed (annulated) aromatic or heteroaromatic polycycle is built of two or more single aromatic or heteroaromatic cycles, which formed the polycycle via a condensation reaction.

In particular, as used throughout the present application the term aryl group or heteroaryl group comprises groups which can be bound via any position of the aromatic or heteroaromatic group, derived from benzene, naphthaline, anthracene, phenanthrene, pyrene, dihydropyrene, chrysene, perylene, fluoranthene, benzanthracene, benzphenanthrene, tetracene, pentacene, benzpyrene, furan, benzofuran, isobenzofuran, dibenzofuran, thiophene, benzothiophene, isobenzothiophene, dibenzothiophene; pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthoimidazole, phenanthroimidazole, pyridoimidazole, pyrazinoimidazole, quinoxalinoimidazole, oxazole, benzoxazole, napthooxazole, anthroxazol, phenanthroxazol, isoxazole, 1,2-thiazole, 1,3-thiazole, benzothiazole, pyridazine, benzopyridazine, pyrimidine, benzopyrimidine, 1,3,5-triazine, quinoxaline, pyrazine, phenazine, naphthyridine, carboline, benzocarboline, phenanthroline, 1,2,3-triazole, 1,2,4-triazole, benzotriazole, 1,2,3-oxadiazole, 1,2,4-oxadiazole, 1,2,5-oxadiazole, 1,2,3,4-tetrazine, purine, pteridine, indolizine and benzothiadiazole or combinations of the abovementioned groups.

As used throughout the present application the term cyclic group may be understood in the broadest sense as any mono-, bi- or polycyclic moieties.

As used throughout the present application the term biphenyl as a substituent may be understood in the broadest sense as ortho-biphenyl, meta-biphenyl, or para-biphenyl, wherein ortho, meta and para is defined in regard to the binding site to another chemical moiety.

As used throughout the present application the term alkyl group may be understood in the broadest sense as any linear, branched, or cyclic alkyl substituent. In particular, the term alkyl comprises the substituents methyl (Me), ethyl (Et), n-propyl ($^n$Pr), i-propyl ($^i$Pr), cyclopropyl, n-butyl ($^n$Bu), i-butyl ($^t$Bu), s-butyl ($^s$Bu), t-butyl($^t$Bu), cyclobutyl, 2-methylbutyl, n-pentyl, s-pentyl, t-pentyl, 2-pentyl, neo-pentyl, cyclopentyl, n-hexyl, s-hexyl, t-hexyl, 2-hexyl, 3-hexyl, neo-hexyl, cyclohexyl, 1-methylcyclopentyl, 2-methylpentyl, n-heptyl, 2-heptyl, 3-heptyl, 4-heptyl, cycloheptyl, 1-methylcyclohexyl, n-octyl, 2-ethylhexyl, cyclooctyl, 1-bicyclo[2,2,2]octyl, 2-bicyclo[2,2,2]-octyl, 2-(2,6-dimethyl)octyl, 3-(3,7-dimethyl)octyl, adamantyl, 2,2,2-trifluorethyl, 1,1-dimethyl-n-hex-1-yl, 1,1-dimethyl-n-hept-1-yl, 1,1-dimethyl-n-oct-1-yl, 1,1-dimethyl-n-dec-1-yl, 1,1-dimethyl-n-dodec-1-yl, 1,1-dimethyl-n-tetradec-1-yl, 1,1-dimethyl-n-hexadec-1-yl, 1,1-dimethyl-n-octadec-1-yl, 1,1-diethyl-n-hex-1-yl, 1,1-diethyl-n-hept-1-yl, 1,1-diethyl-n-oct-1-yl, 1,1-diethyl-n-dec-1-yl, 1,1-diethyl-n-dodec-1-yl, 1,1-diethyl-n-tetradec-1-yl, 1,1-diethyln-n-hexadec-1-yl,1,1-diethyl-n-octadec-1-yl, 1-(n-propyl)-cyclohex-1-yl, 1-(n-butyl)-cyclohex-1-yl, 1-(n-hexyl)-cyclohex-1-yl, 1-(n-octyl)-cyclohex-1-yl and 1-(n-decyl)-cyclohex-1-yl.

As used throughout the present application the term alkenyl comprises linear, branched, and cyclic alkenyl substituents. The term alkenyl group exemplarily comprises the substituents ethenyl, propenyl, butenyl, pentenyl, cyclopentenyl, hexenyl, cyclohexenyl, heptenyl, cycloheptenyl, octenyl, cyclooctenyl or cyclooctadienyl.

As used throughout the present application the term alkynyl comprises linear, branched, and cyclic alkynyl substituents. The term alkynyl group exemplarily comprises ethynyl, propynyl, butynyl, pentynyl, hexynyl, heptynyl or octynyl.

As used throughout the present application the term alkoxy comprises linear, branched, and cyclic alkoxy substituents. The term alkoxy group exemplarily comprises methoxy, ethoxy, n-propoxy, i-propoxy, n-butoxy, i-butoxy, s-butoxy, t-butoxy and 2-methylbutoxy.

As used throughout the present application the term thioalkoxy comprises linear, branched, and cyclic thioalkoxy substituents, in which the O of the exemplarily alkoxy groups is replaced by S.

As used throughout the present application, the terms "halogen" and "halo" may be understood in the broadest sense as being preferably fluorine, chlorine, bromine or iodine.

Whenever hydrogen (H) is mentioned herein, it could also be replaced by deuterium at each occurrence.

It is understood that when a molecular fragment is described as being a substituent or otherwise attached to another moiety, its name may be written as if it were a fragment (e.g. naphtyl, dibenzofuryl) or as if it were the whole molecule (e.g. naphthalene, dibenzofuran). As used herein, these different ways of designating a substituent or attached fragment are considered to be equivalent.

In one embodiment, the organic molecules according to the invention have an excited state lifetime of not more than 150 $\mu$s, of not more than 100 $\mu$s, in particular of not more than 50 $\mu$s, more preferably of not more than 10 $\mu$s or not more than 7 $\mu$s in a film of poly(methyl methacrylate) (PMMA) with 10 % by weight of organic molecule at room temperature.

In one embodiment of the invention, the organic molecules according to the invention represent thermally-activated delayed fluorescence (TADF) emitters, which exhibit a $\Delta E_{ST}$ value, which corresponds to the energy difference between the first excited singlet state (S1) and the first excited triplet state (T1), of less than 5000 $cm^{-1}$, preferably less than 3000 $cm^{-1}$, more preferably less than 1500 $cm^{-1}$, even more preferably less than 1000 $cm^{-1}$ or even less than 500 $cm^{-1}$.

In a further embodiment of the invention, the organic molecules according to the invention have an emission peak in the visible or nearest ultraviolet range, i.e., in the range of a wavelength of from 380 to 800 nm, with a full width at half maximum of less than 0.50 eV, preferably less than 0.48 eV, more preferably less than 0.45 eV, even more preferably less than 0.43 eV or even less than 0.40 eV in a film of poly(methyl methacrylate) (PMMA) with 10 % by weight of organic molecule at room temperature.

In a further embodiment of the invention, the organic molecules according to the invention have a "blue material index" (BMI), calculated by dividing the photoluminescence quantum yield (PLQY) in % by the CIEy color coordinate of the emitted light, of more than 150, in particular more than 200, preferably more than 250, more preferably of more than 300 or even more than 500.

Orbital and excited state energies can be determined either by means of experimental methods or by calculations employing quantum-chemical methods, in particular density functional theory calculations. The energy of the highest occupied molecular orbital $E^{HOMO}$ is determined by methods known to the person skilled in the art from cyclic voltammetry measurements with an accuracy of 0.1 eV. The energy of the lowest unoccupied molecular orbital $E^{LUMO}$ is calculated as $E^{HOMO} + E^{gap}$, wherein $E^{gap}$ is determined as follows: For host compounds, the onset of the emission spectrum of a film with 10 % by weight of host in poly(methyl methacrylate) (PMMA) is used as $E^{gap}$, unless stated otherwise. For emitter molecules, $E^{gap}$ is determined as the energy at which the excitation and emission spectra of a film with 10 % by weight of emitter in PMMA cross.

The energy of the first excited triplet state T1 is determined from the onset of the emission spectrum at low temperature, typically at 77 K. For host compounds, where the first excited singlet state and the lowest triplet state are energetically separated by > 0.4 eV, the phosphorescence is usually visible in a steady-state spectrum in 2-Me-THF. The triplet energy can thus be determined as the onset of the phosphorescence spectrum. For TADF emitter molecules,

the energy of the first excited triplet state T1 is determined from the onset of the delayed emission spectrum at 77 K, if not otherwise stated measured in a film of PMMA with 10 % by weight of emitter. Both for host and emitter compounds, the energy of the first excited singlet state S1 is determined from the onset of the emission spectrum, if not otherwise stated measured in a film of PMMA with 10 % by weight of host or emitter compound.

The onset of an emission spectrum is determined by computing the intersection of the tangent to the emission spectrum with the x-axis. The tangent to the emission spectrum is set at the high-energy side of the emission band and at the point at half maximum of the maximum intensity of the emission spectrum.

A further aspect of the invention relates to a process for preparing organic molecules (with an optional subsequent reaction) according to the invention, wherein a 2-$Hal^a$-4,6-di-$R^{Tz}$-substituted 1,3,5-triazine (E2) is used as a reactant:

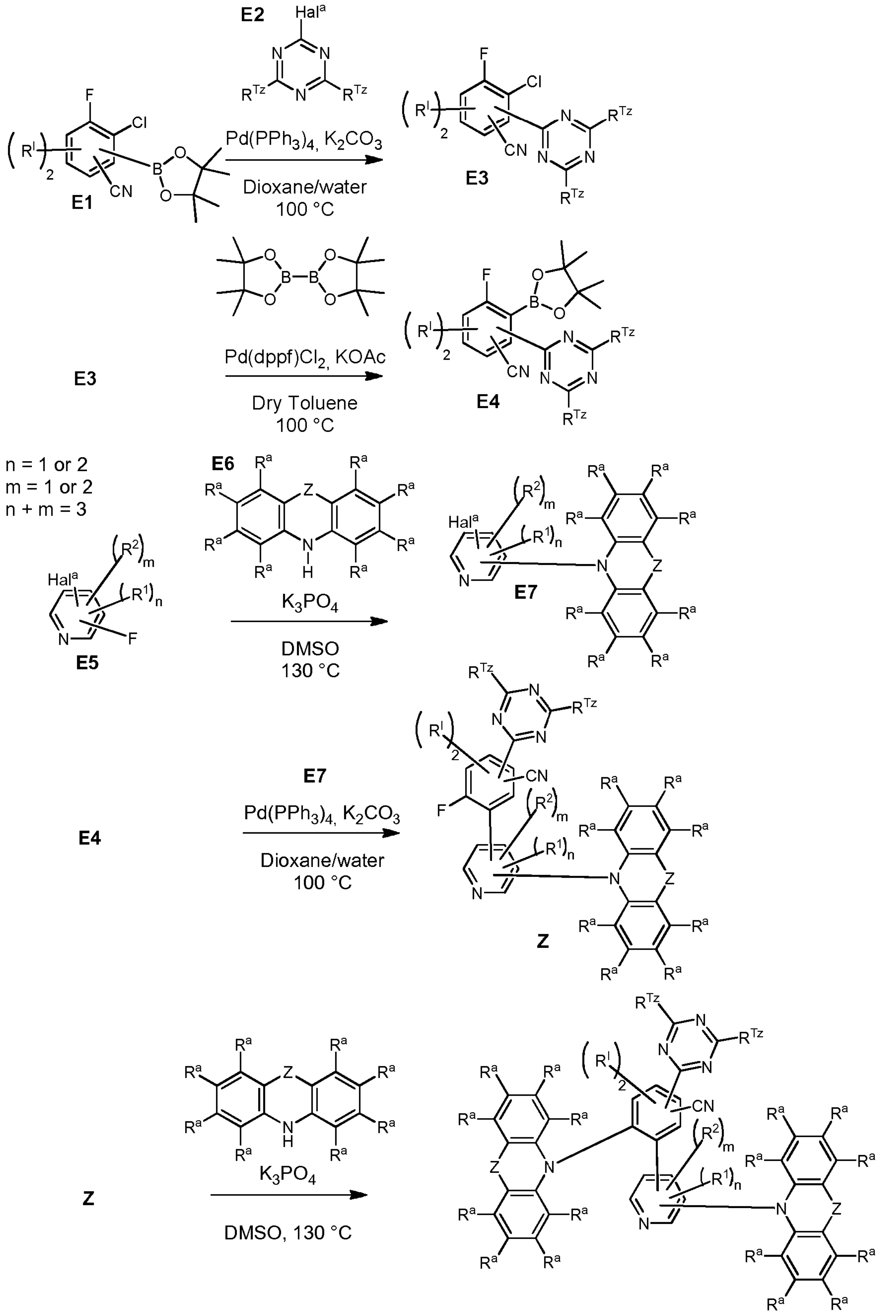


E2 E1 E3 E3 E4 E6 E5 E7 E7 E4 Z Z

Wherein $Hal^a$ at each occurrence independently from each other is selected from the group consisting of Cl, Br and I.

A person of skill in the pertinent art is aware that instead of the *in-situ* generation of a boronic acid ester by

reacting a halo-species with bis-(pinacolato)diboron and then reacting it with another halo-species as described here, alternative synthesis routes can be chosen. For example, one of the two halo-species, i.e. the halo-fluorophenyl-1,3,5-triazine and the halo-fluoro-pyridine, can be replaced by the corresponding boronic acid or boronic acid ester species and reacted with the respective other halo-species in a typical cross-coupling reaction.

For the reaction of **E1** with a nitrogen heterocycle in a nucleophilic aromatic substitution with an aryl halide, preferably an aryl fluoride, typical conditions include the use of a base, such as tribasic potassium phosphate or sodium hydride, for example, in an aprotic polar solvent, such as dimethyl sulfoxide (DMSO) or N,N-dimethylformamide (DMF), for example.

An alternative synthesis route comprises the introduction of a nitrogen heterocycle via copper- or palladium-catalyzed coupling to an aryl halide or aryl pseudohalide, preferably an aryl bromide, an aryl iodide, aryl triflate or an aryl tosylate.

A further aspect of the invention relates to the use of an organic molecule according to the invention as a luminescent emitter or as an absorber, and/or as host material and/or as electron transport material, and/or as hole injection material, and/or as hole blocking material in an organic optoelectronic device.

The optoelectronic device may be understood in the broadest sense as any device based on organic materials that is suitable for emitting light in the visible or nearest ultraviolet (UV) range, i.e., in the range of a wavelength of from 380 to 800 nm. More preferably, the optoelectronic device may be able to emit light in the visible range, i.e., of from 400 to 800 nm.

In the context of such a use, the organic optoelectronic device is more particularly selected from the group consisting of:

- organic light-emitting diodes (OLEDs),
- light-emitting electrochemical cells,
- OLED sensors, especially in gas and vapor sensors not hermetically externally shielded,
- organic diodes,
- organic solar cells,
- organic transistors,
- organic field-effect transistors,
- organic lasers and
- down-conversion elements.

In a preferred embodiment in the context of such use, the optoelectronic device is a device selected from the group consisting of an organic light emitting diode (OLED), a light emitting electrochemical cell (LEC), and a light-emitting transistor.

In the case of the use, the fraction of the organic molecule according to the invention in the emission layer in an organic optoelectronic device, more particularly in OLEDs, is 1 % to 99 % by weight, more particularly 5 % to 80 % by weight. In an alternative embodiment, the proportion of the organic molecule in the emission layer is 100 % by weight.

In one embodiment, the light-emitting layer comprises not only the organic molecules according to the invention but also a host material whose triplet (T1) and singlet (S1) energy levels are energetically higher than the triplet (T1) and singlet (S1) energy levels of the organic molecule.

A further aspect of the invention relates to a composition comprising or consisting of:

(a) at least one organic molecule according to the invention, in particular in the form of an emitter and/or a host, and
(b) one or more emitter and/or host materials, which differ from the organic molecule according to the invention and
(c) optional one or more dyes and/or one or more solvents.

In one embodiment, the light-emitting layer comprises (or (essentially) consists of) a composition comprising or consisting of:

(a) at least one organic molecule according to the invention, in particular in the form of an emitter and/or a host, and
(b) one or more emitter and/or host materials, which differ from the organic molecule according to the invention and
(c) optional one or more dyes and/or one or more solvents.

Particularly preferably the light-emitting layer EML comprises (or (essentially) consists of) a composition comprising or consisting of:

(i) 1-50 % by weight, preferably 5-40 % by weight, in particular 10-30 % by weight, of one or more organic molecules according to the invention E;

(ii) 5-99 % by weight, preferably 30-94.9 % by weight, in particular 40-89% by weight, of at least one host compound H; and
(iii) optionally 0-94 % by weight, preferably 0.1-65 % by weight, in particular 1-50 % by weight, of at least one further host compound D with a structure differing from the structure of the molecules according to the invention; and
(iv) optionally 0-94 % by weight, preferably 0-65 % by weight, in particular 0-50 % by weight, of a solvent; and
(v) optionally 0-30 % by weight, in particular 0-20 % by weight, preferably 0-5 % by weight, of at least one further emitter molecule F with a structure differing from the structure of the molecules according to the invention.

Preferably, energy can be transferred from the host compound H to the one or more organic molecules according to the invention E, in particular transferred from the first excited triplet state T1(H) of the host compound H to the first excited triplet state T1(E) of the one or more organic molecules according to the invention E and/ or from the first excited singlet state S1(H) of the host compound H to the first excited singlet state S1(E) of the one or more organic molecules according to the invention E.
In a further embodiment, the light-emitting layer EML comprises (or (essentially) consists of) a composition comprising or consisting of:

(i) 1-50 % by weight, preferably 5-40 % by weight, in particular 10-30 % by weight, of one organic molecule according to the invention E;
(ii) 5-99 % by weight, preferably 30-94.9 % by weight, in particular 40-89% by weight, of one host compound H; and
(iii) optionally 0-94 % by weight, preferably 0.1-65 % by weight, in particular 1-50 % by weight, of at least one further host compound D with a structure differing from the structure of the molecules according to the invention; and
(iv) optionally 0-94 % by weight, preferably 0-65 % by weight, in particular 0-50 % by weight, of a solvent; and
(v) optionally 0-30 % by weight, in particular 0-20 % by weight, preferably 0-5 % by weight, of at least one further emitter molecule F with a structure differing from the structure of the molecules according to the invention.

In one embodiment, the host compound H has a highest occupied molecular orbital HOMO(H) having an energy $E^{HOMO}(H)$ in the range of from -5 to -6.5 eV and the at least one further host compound D has a highest occupied molecular orbital HOMO(D) having an energy $E^{HOMO}(D)$, wherein $E^{HOMO}(H) > E^{HOMO}(D)$.
In a further embodiment, the host compound H has a lowest unoccupied molecular orbital LUMO(H) having an energy $E^{LUMO}(H)$ and the at least one further host compound D has a lowest unoccupied molecular orbital LUMO(D) having an energy $E^{LUMO}(D)$, wherein $E^{LUMO}(H) > E^{LUMO}(D)$.
In one embodiment, the host compound H has a highest occupied molecular orbital HOMO(H) having an energy $E^{HOMO}(H)$ and a lowest unoccupied molecular orbital LUMO(H) having an energy $E^{LUMO}(H)$, and

the at least one further host compound D has a highest occupied molecular orbital HOMO(D) having an energy $E^{HOMO}(D)$ and a lowest unoccupied molecular orbital LUMO(D) having an energy $E^{LUMO}(D)$,
the organic molecule according to the invention E has a highest occupied molecular orbital HOMO(E) having an energy $E^{HOMO}(E)$ and a lowest unoccupied molecular orbital LUMO(E) having an energy $E^{LUMO}(E)$,

wherein
$E^{HOMO}(H) > E^{HOMO}(D)$ and the difference between the energy level of the highest occupied molecular orbital HOMO(E) of the organic molecule according to the invention E ($E^{HOMO}(E)$) and the energy level of the highest occupied molecular orbital HOMO(H) of the host compound H ($E^{HOMO}(H)$) is between -0.5 eV and 0.5 eV, more preferably between -0.3 eV and 0.3 eV, even more preferably between -0.2 eV and 0.2 eV or even between -0.1 eV and 0.1 eV; and $E^{LUMO}(H) > E^{LUMO}(D)$ and the difference between the energy level of the lowest unoccupied molecular orbital LUMO(E) of the organic molecule according to the invention E ($E^{LUMO}(E)$) and the lowest unoccupied molecular orbital LUMO(D) of the at least one further host compound D ($E^{LUMO}(D)$) is between -0.5 eV and 0.5 eV, more preferably between -0.3 eV and 0.3 eV, even more preferably between -0.2 eV and 0.2 eV or even between -0.1 eV and 0.1 eV.
In a further aspect, the invention relates to an organic optoelectronic device comprising an organic molecule or a composition of the type described here, more particularly in the form of a device selected from the group consisting of organic light-emitting diode (OLED), light-emitting electrochemical cell, OLED sensor, more particularly gas and vapour sensors not hermetically externally shielded, organic diode, organic solar cell, organic transistor, organic field-effect transistor, organic laser and down-conversion element.
In a preferred embodiment, the optoelectronic device is a device selected from the group consisting of an organic light emitting diode (OLED), a light emitting electrochemical cell (LEC), and a light-emitting transistor.
In one embodiment of the organic optoelectronic device of the invention, the organic molecule according to the invention E is used as emission material in a light-emitting layer EML.
In one embodiment of the organic optoelectronic device of the invention the light-emitting layer EML consists

of the composition according to the invention described here.

Exemplarily, when the optoelectronic device is an OLED, it may exhibit the following layer structure:

1. substrate
2. anode layer A
3. hole injection layer, HIL
4. hole transport layer, HTL
5. electron blocking layer, EBL
6. emitting layer, EML
7. hole blocking layer, HBL
8. electron transport layer, ETL
9. electron injection layer, EIL
10. cathode layer,

wherein the OLED comprises each layer only optionally, different layers may be merged and the OLED may comprise more than one layer of each layer type defined above.

Furthermore, the optoelectronic device may optionally comprise one or more protective layers protecting the device from damaging exposure to harmful species in the environment including, exemplarily moisture, vapor and/or gases.

In one embodiment of the invention, the optoelectronic device is an OLED, which exhibits the following inverted layer structure:

1. substrate
2. cathode layer
3. electron injection layer, EIL
4. electron transport layer, ETL
5. hole blocking layer, HBL
6. emitting layer, B
7. electron blocking layer, EBL
8. hole transport layer, HTL
9. hole injection layer, HIL
10. anode layer A

Wherein the OLED with an inverted layer structure comprises each layer only optionally, different layers may be merged and the OLED may comprise more than one layer of each layer types defined above.

In one embodiment of the invention, the optoelectronic device is an OLED, which may exhibit stacked architecture. In this architecture, contrary to the typical arrangement, where the OLEDs are placed side by side, the individual units are stacked on top of each other. Blended light may be generated with OLEDs exhibiting a stacked architecture, in particular white light may be generated by stacking blue, green and red OLEDs. Furthermore, the OLED exhibiting a stacked architecture may optionally comprise a charge generation layer (CGL), which is typically located between two OLED subunits and typically consists of a n-doped and p-doped layer with the n-doped layer of one CGL being typically located closer to the anode layer.

In one embodiment of the invention, the optoelectronic device is an OLED, which comprises two or more emission layers between anode and cathode. In particular, this so-called tandem OLED comprises three emission layers, wherein one emission layer emits red light, one emission layer emits green light and one emission layer emits blue light, and optionally may comprise further layers such as charge generation layers, blocking or transporting layers between the individual emission layers. In a further embodiment, the emission layers are adjacently stacked. In a further embodiment, the tandem OLED comprises a charge generation layer between each two emission layers. In addition, adjacent emission layers or emission layers separated by a charge generation layer may be merged.

The substrate may be formed by any material or composition of materials. Most frequently, glass slides are used as substrates. Alternatively, thin metal layers (e.g., copper, gold, silver or aluminum films) or plastic films or slides may be used. This may allow a higher degree of flexibility. The anode layer A is mostly composed of materials allowing to obtain an (essentially) transparent film. As at least one of both electrodes should be (essentially) transparent in order to allow light emission from the OLED, either the anode layer A or the cathode layer C is transparent. Preferably, the anode layer A comprises a large content or even consists of transparent conductive oxides (TCOs). Such anode layer A may exemplarily comprise indium tin oxide, aluminum zinc oxide, fluorine doped tin oxide, indium zinc oxide, PbO, SnO, zirconium oxide, molybdenum oxide, vanadium oxide, wolfram oxide, graphite, doped Si, doped Ge, doped GaAs, doped polyaniline, doped polypyrrol and/or doped polythiophene. Particularly preferably, the anode layer A (essentially)

consists of indium tin oxide (ITO) (e.g., $(InO_3)0.9(SnO_2)0.1$). The roughness of the anode layer A caused by the transparent conductive oxides (TCOs) may be compensated by using a hole injection layer (HIL). Further, the HIL may facilitate the injection of quasi charge carriers (i.e., holes) in that the transport of the quasi charge carriers from the TCO to the hole transport layer (HTL) is facilitated. The hole injection layer (HIL) may comprise poly-3,4-ethylendioxy thiophene (PEDOT), polystyrene sulfonate (PSS), $MoO_2$, $V_2O_5$, CuPC or CuI, in particular a mixture of PEDOT and PSS. The hole injection layer (HIL) may also prevent the diffusion of metals from the anode layer A into the hole transport layer (HTL). The HIL may exemplarily comprise PEDOT:PSS (poly-3,4-ethylendioxy thiophene: polystyrene sulfonate), PEDOT (poly-3,4-ethylendioxy thiophene), mMTDATA (4,4',4"-tris[phenyl(m-tolyl)amino]triphenylamine), Spiro-TAD (2,2',7,7'-tetrakis(n,n-diphenylamino)-9,9'-spirobifluorene), DNTPD (N1,N1'-(biphenyl-4,4'-diyl)bis(N1-phenyl-N4,N4-di-m-tolylbenzene-1,4-diamine), NPB (N,N'-nis-(1-naphthalenyl)-N,N'-bisphenyl-(1,1'-biphenyl)-4,4'-diamine), NPNPB (N,N'-diphenyl-N,N'-di-[4-(N,N-diphenyl-amino)phenyl]benzidine), MeO-TPD (N,N,N',N'-tetrakis(4-methoxyphenyl)benzidine), HAT-CN (1,4,5,8,9,11-hexaazatriphenylen-hexacarbonitrile) and/or Spiro-NPD (N,N'-diphenyl-N,N'-bis-(1-naphthyl)-9,9'-spirobifluorene-2,7-diamine).

Adjacent to the anode layer A or hole injection layer (HIL) typically a hole transport layer (HTL) is located. Herein, any hole transport compound may be used. Exemplarily, electron-rich heteroaromatic compounds such as triarylamines and/or carbazoles may be used as hole transport compound. The HTL may decrease the energy barrier between the anode layer A and the light-emitting layer EML. The hole transport layer (HTL) may also be an electron blocking layer (EBL). Preferably, hole transport compounds bear comparably high energy levels of their triplet states T1. Exemplarily the hole transport layer (HTL) may comprise a star-shaped heterocycle such as tris(4-carbazoyl-9-ylphenyl)amine (TCTA), poly-TPD (poly(4-butylphenyl-diphenyl-amine)), [alpha]-NPD (poly(4-butylphenyl-diphenyl-amine)), TAPC (4,4'-cyclohexyliden-bis[N,N-bis(4-methylphenyl)benzenamine]), 2-TNATA (4,4',4"-tris[2-naphthyl(phenyl)amino]triphenylamine), Spiro-TAD, DNTPD, NPB, NPNPB, MeO-TPD, HAT-CN and/or TrisPcz (9,9'-diphenyl-6-(9-phenyl-9H-carbazol-3-yl)-9H,9'H-3,3'-bicarbazole). In addition, the HTL may comprise a p-doped layer, which may be composed of an inorganic or organic dopant in an organic hole-transporting matrix. Transition metal oxides such as vanadium oxide, molybdenum oxide or tungsten oxide may exemplarily be used as inorganic dopant. Tetrafluorotetracyanoquinodimethane ($F_4$-TCNQ), copper-pentafluorobenzoate (Cu(I)pFBz) or transition metal complexes may exemplarily be used as organic dopant.

The EBL may exemplarily comprise mCP (1,3-bis(carbazol-9-yl)benzene), TCTA, 2-TNATA, mCBP (3,3-di(9H-carbazol-9-yl)biphenyl), tris-Pcz, CzSi (9-(4-tert-Butylphenyl)-3,6-bis(triphenylsilyl)-9H-carbazole), and/or DCB (N,N'-dicarbazolyl-1,4-dimethylbenzene).

Adjacent to the hole transport layer (HTL), typically, the light-emitting layer EML is located. The light-emitting layer EML comprises at least one light emitting molecule. Particularly, the EML comprises at least one light emitting molecule according to the invention E. In one embodiment, the light-emitting layer comprises only the organic molecules according to the invention E. Typically, the EML additionally comprises one or more host materials H. Exemplarily, the host material H is selected from CBP (4,4'-Bis-(N-carbazolyl)-biphenyl), mCP, mCBP Sif87 (dibenzo[b,d]thiophen-2-yltriphenylsilane), CzSi, Sif88 (dibenzo[b,d]thiophen-2-yl)diphenylsilane), DPEPO (bis[2-(diphenylphosphino)phenyl] ether oxide), 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole, 9-[3,5-bis(2-dibenzothiophenyl)phenyl]-9H-carbazole, T2T (2,4,6-tris(biphenyl-3-yl)-1,3,5-triazine), T3T (2,4,6-tris(triphenyl-3-yl)-1,3,5-triazine) and/or TST (2,4,6-tris(9,9'-spirobifluorene-2-yl)-1,3,5-triazine). The host material H typically should be selected to exhibit first triplet (T1) and first singlet (S1) energy levels, which are energetically higher than the first triplet (T1) and first singlet (S1) energy levels of the organic molecule.

In one embodiment of the invention, the EML comprises a so-called mixed-host system with at least one hole-dominant host and one electron-dominant host. In a particular embodiment, the EML comprises exactly one light emitting molecule according to the invention E and a mixed-host system comprising T2T as electron-dominant host and a host selected from CBP, mCP, mCBP, 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole and 9-[3,5-bis(2-dibenzothiophenyl)phenyl]-9H-carbazole as hole-dominant host. In a further embodiment the EML comprises 50-80 % by weight, preferably 60-75 % by weight of a host selected from CBP, mCP, mCBP, 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole and 9-[3,5-bis(2-dibenzothiophenyl)phenyl]-9H-carbazole; 10-45 % by weight, preferably 15-30 % by weight of T2T and 5-40 % by weight, preferably 10-30 % by weight of light emitting molecule according to the invention.

Adjacent to the light-emitting layer EML an electron transport layer (ETL) may be located. Herein, any electron transporter may be used. Exemplarily, electron-poor compounds such as, e.g., benzimidazoles, pyridines, triazoles, oxadiazoles (e.g., 1,3,4-oxadiazole), phosphinoxides and sulfone, may be used. An electron transporter may also be a star-shaped heterocycle such as 1,3,5-tri(1-phenyl-1H-benzo[d]imidazol-2-yl)phenyl (TPBi). The ETL may comprise NBphen (2,9-bis(naphthalen-2-yl)-4,7-diphenyl-1,10-phenanthroline), $Alq_3$ (Aluminum-tris(8-hydroxyquinoline)), TSPO1

(diphenyl-4-triphenylsilylphenyl-phosphinoxide), BPyTP2 (2,7-di(2,2'-bipyridin-5-yl)triphenyle), Sif87 (dibenzo[b,d]thiophen-2-yltriphenylsilane), Sif88 (dibenzo[b,d]thiophen-2-yl)diphenylsilane), BmPyPhB (1,3-bis[3,5-di(pyridin-3-yl)phenyl]benzene) and/or BTB (4,4'-bis-[2-(4,6-diphenyl-1,3,5-triazinyl)]-1,1'-biphenyl). Optionally, the ETL may be doped with materials such as Liq. The electron transport layer (ETL) may also block holes or a holeblocking layer (HBL) is introduced.

The HBL may exemplarily comprise BCP (2,9-dimethyl-4,7-diphenyl-1,10-phenanthroline = Bathocuproine), BAlq (bis(8-hydroxy-2-methylquinoline)-(4-phenylphenoxy)aluminum), NBphen (2,9-bis(naphthalen-2-yl)-4,7-diphenyl-1,10-phenanthroline), $Alq_3$ (Aluminum-tris(8-hydroxyquinoline)), TSPO1 (diphenyl-4-triphenylsilylphenyl-phosphinoxide), T2T (2,4,6-tris(biphenyl-3-yl)-1,3,5-triazine), T3T (2,4,6-tris(triphenyl-3-yl)-1,3,5-triazine), TST (2,4,6-tris(9,9'-spirobifluorene-2-yl)-1,3,5-triazine), and/or TCB/TCP (1,3,5-tris(N-carbazolyl)benzol/ 1,3,5-tris(carbazol)-9-yl)benzene).

Adjacent to the electron transport layer (ETL), a cathode layer C may be located. Exemplarily, the cathode layer C may comprise or may consist of a metal (e.g., Al, Au, Ag, Pt, Cu, Zn, Ni, Fe, Pb, LiF, Ca, Ba, Mg, In, W, or Pd) or a metal alloy. For practical reasons, the cathode layer may also consist of (essentially) intransparent metals such as Mg, Ca or Al. Alternatively or additionally, the cathode layer C may also comprise graphite and or carbon nanotubes (CNTs). Alternatively, the cathode layer C may also consist of nanoscalic silver wires.

An OLED may optionally further, comprise a protection layer between the electron transport layer (ETL) and the cathode layer C (which may be designated as electron injection layer (EIL)). This layer may comprise lithium fluoride, cesium fluoride, silver, Liq (8-hydroxyquinolinolatolithium), $Li_2O$, $BaF_2$, MgO and/or NaF.

Optionally, also the electron transport layer (ETL) and/or a hole blocking layer (HBL) may comprise one or more host compounds H.

In order to modify the emission spectrum and/or the absorption spectrum of the light-emitting layer EML further, the light-emitting layer EML may further comprise one or more further emitter molecules F. Such an emitter molecule F may be any emitter molecule known in the art. Preferably such an emitter molecule F is a molecule with a structure differing from the structure of the molecules according to the invention E. The emitter molecule F may optionally be a TADF emitter. Alternatively, the emitter molecule F may optionally be a fluorescent and/or phosphorescent emitter molecule which is able to shift the emission spectrum and/or the absorption spectrum of the light-emitting layer EML. Exemplarily, the triplet and/or singlet excitons may be transferred from the emitter molecule according to the invention E to the emitter molecule F before relaxing to the ground state S0 by emitting light typically red-shifted in comparison to the light emitted by emitter molecule E. Optionally, the emitter molecule F may also provoke two-photon effects (i.e., the absorption of two photons of half the energy of the absorption maximum).

Optionally, an optoelectronic device (e.g., an OLED) may, for example, be an essentially white optoelectronic device. Exemplarily such white optoelectronic device may comprise at least one (deep) blue emitter molecule and one or more emitter molecules emitting green and/or red light. Then, there may also optionally be energy transmittance between two or more molecules as described above.

As used herein, if not defined more specifically in the particular context, the designation of the colors of emitted and/or absorbed light is as follows:

| | |
|---|---|
| violet: | wavelength range of >380-420 nm; |
| deep blue: | wavelength range of >420-480 nm; |
| sky blue: | wavelength range of >480-500 nm; |
| green: | wavelength range of >500-560 nm; |
| yellow: | wavelength range of >560-580 nm; |
| orange: | wavelength range of >580-620 nm; |
| red: | wavelength range of >620-800 nm. |

With respect to emitter molecules, such colors refer to the emission maximum. Therefore, exemplarily, a deep blue emitter has an emission maximum in the range of from >420 to 480 nm, a sky blue emitter has an emission maximum in the range of from >480 to 500 nm, a green emitter has an emission maximum in a range of from >500 to 560 nm, a red emitter has an emission maximum in a range of from >620 to 800 nm.

A deep blue emitter may preferably have an emission maximum of below 480 nm, more preferably below 470 nm, even more preferably below 465 nm or even below 460 nm. It will typically be above 420 nm, preferably above 430 nm, more preferably above 440 nm or even above 450 nm.

Accordingly, a further aspect of the present invention relates to an OLED, which exhibits an external quantum efficiency at 1000 cd/$m^2$ of more than 8 %, more preferably of more than 10 %, more preferably of more than 13 %, even more preferably of more than 15 % or even more than 20 % and/or exhibits an emission maximum between 420 nm and 500 nm, preferably between 430 nm and 490 nm, more preferably between 440 nm and 480 nm, even more

preferably between 450 nm and 470 nm and/or exhibits a LT80 value at 500 cd/$m^2$ of more than 100 h, preferably more than 200 h, more preferably more than 400 h, even more preferably more than 750 h or even more than 1000 h. Accordingly, a further aspect of the present invention relates to an OLED, whose emission exhibits a CIEy color coordinate of less than 0.45, preferably less than 0.30, more preferably less than 0.20 or even more preferably less than 0.15 or even less than 0.10.

A further aspect of the present invention relates to an OLED, which emits light at a distinct color point. According to the present invention, the OLED emits light with a narrow emission band (small full width at half maximum (FWHM)). In one aspect, the OLED according to the invention emits light with a FWHM of the main emission peak of less than 0.50 eV, preferably less than 0.48 eV, more preferably less than 0.45 eV, even more preferably less than 0.43 eV or even less than 0.40 eV.

A further aspect of the present invention relates to an OLED, which emits light with CIEx and CIEy color coordinates close to the CIEx (= 0.131) and CIEy (= 0.046) color coordinates of the primary color blue (CIEx = 0.131 and CIEy = 0.046) as defined by ITU-R Recommendation BT.2020 (Rec. 2020) and thus is suited for the use in Ultra High Definition (UHD) displays, e.g. UHD-TVs. Accordingly, a further aspect of the present invention relates to an OLED, whose emission exhibits a CIEx color coordinate of between 0.02 and 0.30, preferably between 0.03 and 0.25, more preferably between 0.05 and 0.20 or even more preferably between 0.08 and 0.18 or even between 0.10 and 0.15 and/ or a a CIEy color coordinate of between 0.00 and 0.45, preferably between 0.01 and 0.30, more preferably between 0.02 and 0.20 or even more preferably between 0.03 and 0.15 or even between 0.04 and 0.10.

In a further aspect, the invention relates to a method for producing an optoelectronic component. In this case an organic molecule of the invention is used.

The optoelectronic device, in particular the OLED according to the present invention, can be fabricated by any means of vapor deposition and/ or liquid processing. Accordingly, at least one layer is

- prepared by means of a sublimation process,
- prepared by means of an organic vapor phase deposition process,
- prepared by means of a carrier gas sublimation process,
- solution processed or printed.

The methods used to fabricate the optoelectronic device, in particular the OLED according to the present invention, are known in the art. The different layers are individually and successively deposited on a suitable substrate by means of subsequent deposition processes. The individual layers may be deposited using the same or differing deposition methods.

Vapor deposition processes, for example, comprise thermal (co)evaporation, chemical vapor deposition and physical vapor deposition. For active matrix OLED display, an AMOLED backplane is used as substrate. The individual layer may be processed from solutions or dispersions employing adequate solvents. Solution deposition process exemplarily comprise spin coating, dip coating and jet printing. Liquid processing may optionally be carried out in an inert atmosphere (e.g., in a nitrogen atmosphere) and the solvent may optionally be completely or partially removed by means known in the state of the art.

## Examples

General synthesis scheme I

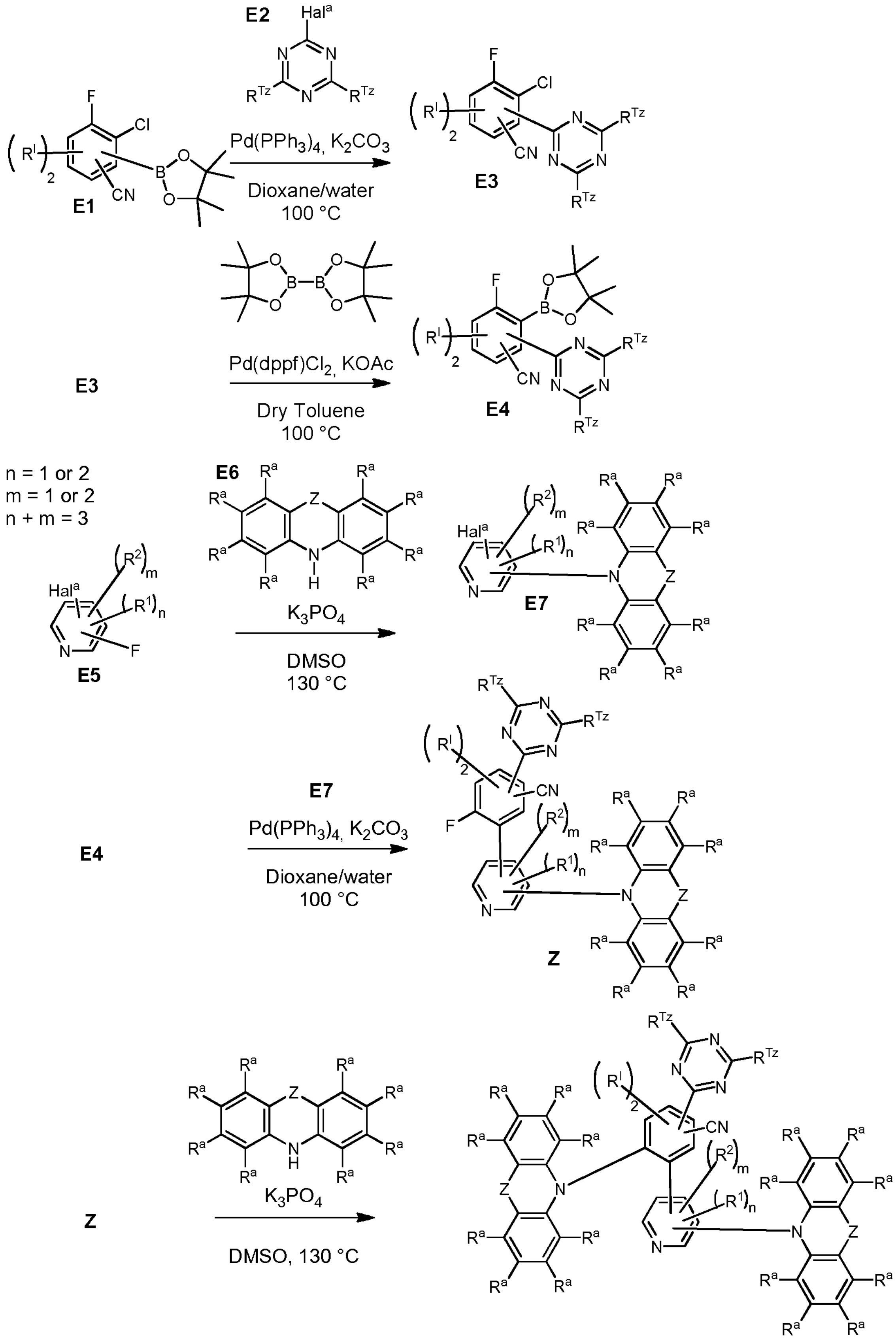


*General procedure for synthesis **AAV1**:*

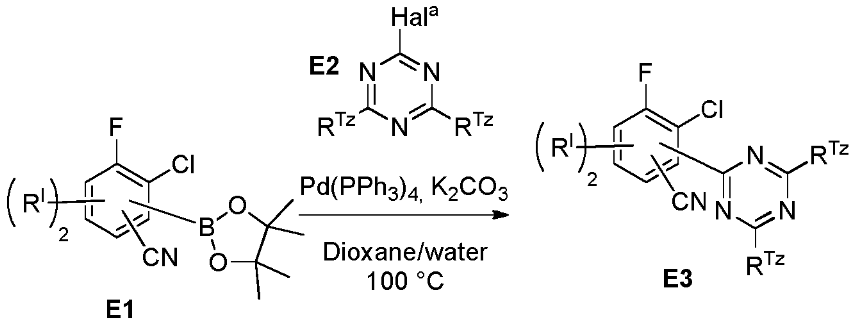


E1 E2 E3

A solution **E1** (1.0 equivalent), **E2** (1.3 equivalent) (CAS 3842-55-5), potassium carbonate (2.0 equivalent; CAS 584-08-7) in dioxane/water was degassed with nitrogen, tetrakis(triphenylphosphine)palladium(0) ($Pd(PPh_3)_4$, 0.05 equivalent; CAS 14221-01-3) was added and the solution was again degassed with nitrogen. The reaction mixture was stirred at 100 °C for 12 h. After cooling down to room temperature (RT), the reaction mixture was extracted with ethyl acetate. The organic phases were collected, washed with brine and dried over $MgSO_4$. The organic solvent was removed, the crude product was purified by column chromatography and the pure compound **E3** was obtained.

*General procedure for synthesis **AAV2**:*

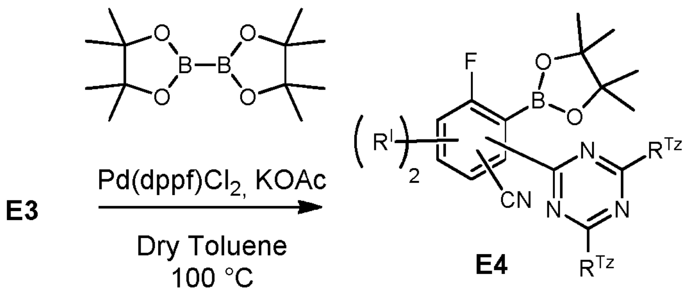


E3 E4

A solution of **E3** (1.0 equivalent), bis(pinacolato)diboron (1.5 equivalent; CAS 73183-34-3), potassium acetate (2.5 equivalent; CAS 127-08-2), [1,1'-bis(diphenylphosphino)ferrocene]palladium (II) dichloride (0.1 equivalent, CAS 72287-26-4, $Pd(dppf)Cl_2$) in dry toluene was degassed with nitrogen. The reaction mixture was stirred at 100 °C for 12 h. After cooling down to room temperature (RT), the reaction mixture was extracted with ethyl acetate. The organic phases were collected, washed with brine and dried over $MgSO_4$. The organic solvent was removed, the crude product was purified by hot wash with ethanol to obtain **E4.**

*General procedure for synthesis **AAV3**:*

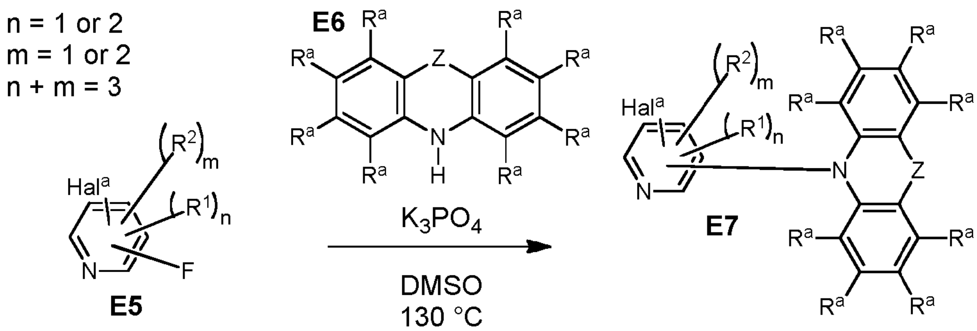


E5 E6 E7

**E5** (1.0 equivalent), **E6** (1.0 equivalent), and potassium phosphate tribasic (2.0 equivalent; CAS 7778-53-2)

were added to dimethyl sulfoxide. The reaction mixture was stirred at 130 °C for 12 h. After cooling down to room temperature (RT), the reaction mixture was extracted with ethyl acetate. The organic phases were collected, washed with brine and dried over $MgSO_4$. The organic solvent was removed, the crude product was purified by hot wash with ethanol and the pure compound **E7** was isolated.

*General procedure for synthesis* ***AAV4*****:**

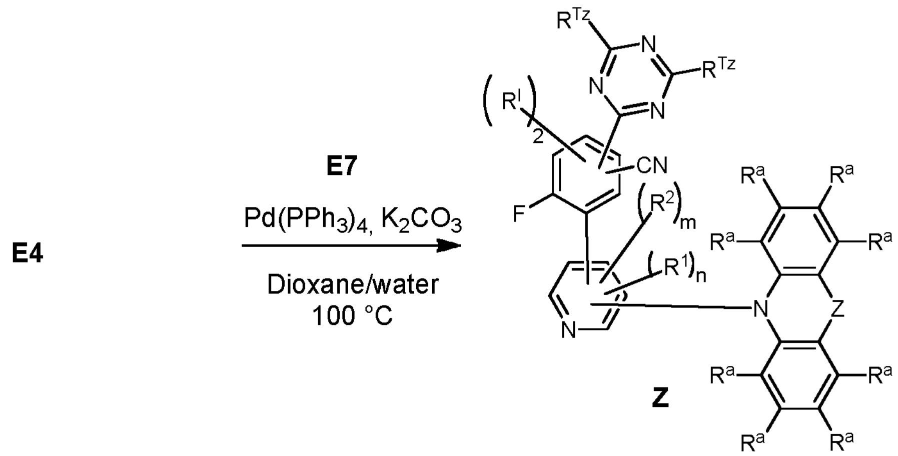


A solution **of E4** (1.0 equivalent), **E7** (1.5 equivalent), potassium carbonate (2.0 equivalent) (CAS 584-08-7) in dioxane/water was degassed with nitrogen, tetrakis(triphenylphosphine)palladium(0) (0.05 equivalent; CAS 14221-01-3) was added and the solution again degassed with nitrogen. The reaction mixture was stirred at 100 °C for 12 h. After cooling down to room temperature (RT), the reaction mixture was extracted with ethyl acetate. The organic phases were collected, washed with brine and dried over $MgSO_4$. The organic solvent was removed, the crude product was purified by column chromatography and the pure product **Z** was obtained.

*General procedure for synthesis* ***AAV5*****:**

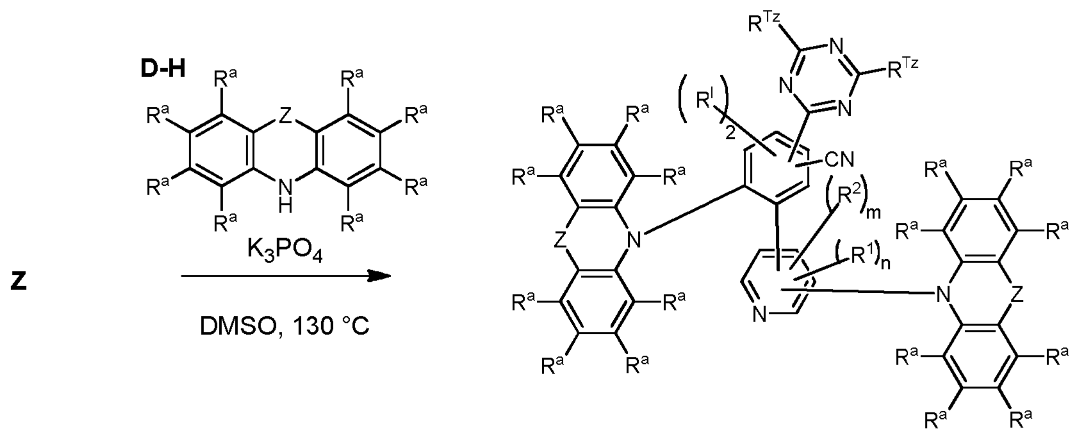


**Z** (1.00 equivalents), the corresponding donor molecule **D-H** (1.20 equivalents) and tribasic potassium phosphate (2.50 equivalents) are suspended under nitrogen atmosphere in DMSO and stirred at 120 °C (12-72 h). Subsequently, the reaction mixture is poured into an excess of brine in order to precipitate the product. The precipitate is filtered off, washed with water and dried under vacuum. The crude product is purified by recrystallization or by flash chromatography. The product is obtained as a solid.

In particular, the donor molecule D-H is a 3,6-substituted carbazole (e.g., 3,6-dimethylcarbazole, 3,6-diphenylcarbazole, 3,6-di-tert-butylcarbazole), a 2,7-substituted carbazole (e.g., 2,7-dimethylcarbazole, 2,7-diphenylcarbazole,

2,7-di-tert-butylcarbazole), a 1,8-substituted carbazole (e.g., 1,8-dimethylcarbazole, 1,8-diphenylcarbazole, 1,8-di-tert-butylcarbazole), a 1-substituted carbazole (e.g., 1-methylcarbazole, 1-phenylcarbazole, 1-tert-butylcarbazole), a 2-substituted carbazole (e.g., 2-methylcarbazole, 2-phenylcarbazole, 2-tert-butylcarbazole), or a 3-substituted carbazole (e.g., 3-methylcarbazole, 3-phenylcarbazole, 3-tert-butylcarbazole).

For example, a halogen-substituted carbazole, particularly 3-bromocarbazole, can be used as D-H.

In a subsequent reaction, a boronic acid ester functional group or boronic acid functional group may, for example, be introduced at the position of the one or more halogen substituents, which was introduced via D-H, to yield the corresponding carbazol-3-ylboronic acid ester or carbazol-3-ylboronic acid, e.g., via the reaction with bis(pinacolato)diboron (CAS No. 73183-34-3). Subsequently, one or more substituents $R^a$ may be introduced in place of the boronic acid ester group or the boronic acid group via a coupling reaction with the corresponding halogenated reactant $R^a$-Hal, preferably $R^a$-Cl and $R^a$-Br.

Alternatively, one or more substituents $R^a$ may be introduced at the position of the one or more halogen substituents, which was introduced via D-H, via the reaction with a boronic acid of the substituent $R^a$[$R^a$-$B(OH)_2$] or a corresponding boronic acid ester.

*Cyclic voltammetry*

Cyclic voltammograms are measured from solutions having concentration of $10^{-3}$ mol/L of the organic molecules in dichloromethane or a suitable solvent and a suitable supporting electrolyte (e.g. 0.1 mol/L of tetrabutylammonium hexafluorophosphate). The measurements are conducted at room temperature under nitrogen atmosphere with a three-electrode assembly (Working and counter electrodes: Pt wire, reference electrode: Pt wire) and calibrated using $FeCp_2/FeC_{p2}^+$ as internal standard. The HOMO data was corrected using ferrocene as internal standard against SCE (saturated calomel electrode).

*Density functional theory calculation*

Molecular structures are optimized employing the BP86 functional and the resolution of identity approach (RI). Excitation energies are calculated using the (BP86) optimized structures employing Time-Dependent DFT (TD-DFT) methods. Orbital and excited state energies are calculated with the B3LYP functional. Def2-SVP basis sets (and a m4-grid for numerical integration are used. The Turbomole program package is used for all calculations.

*Photophysical measurements*

Sample pretreatment: Spin-coating
Apparatus: Spin150, SPS euro.
The sample concentration is 10 mg/ml, dissolved in a suitable solvent.
Program: 1) 3 s at 400 U/min; 20 s at 1000 U/min at 1000 Upm/s. 3) 10 s at 4000 U/min at 1000 Upm/s. After coating, the films are dried at 70 °C for 1 min.

Photoluminescence spectroscopy and TCSPC (*Time-correlated single-photon counting*) Steady-state emission spectroscopy is measured by a Horiba Scientific, Modell FluoroMax-4 equipped with a 150 W Xenon-Arc lamp, excitation- and emissions monochromators and a Hamamatsu R928 photomultiplier and a time-correlated single-photon counting option. Emissions and excitation spectra are corrected using standard correction fits.

Excited state lifetimes are determined employing the same system using the TCSPC method with FM-2013 equipment and a Horiba Yvon TCSPC hub.
Excitation sources:

NanoLED 370 (wavelength: 371 nm, puls duration: 1,1 ns)
NanoLED 290 (wavelength: 294 nm, puls duration: <1 ns)
SpectraLED 310 (wavelength: 314 nm)
SpectraLED 355 (wavelength: 355 nm).

Data analysis (exponential fit) is done using the software suite DataStation and DAS6 analysis software. The fit is specified using the chi-squared-test.

Photoluminescence quantum yield measurements

For photoluminescence quantum yield (PLQY) measurements an *Absolute PL Quantum Yield Measurement C9920-03G* system (*Hamamatsu Photonics*) is used. Quantum yields and CIE coordinates are determined using the

software U6039-05 version 3.6.0.
Emission maxima are given in nm, quantum yields $\phi$ in % and CIE coordinates as x,y values. PLQY is determined using the following protocol:

1) Quality assurance: Anthracene in ethanol (known concentration) is used as reference
2) Excitation wavelength: the absorption maximum of the organic molecule is determined and the molecule is excited using this wavelength
3) Measurement
Quantum yields are measured for sample of solutions or films under nitrogen atmosphere. The yield is calculated using the equation:

$$\Phi_{PL} = \frac{n_{photon}, emited}{n_{photon}, absorbed} = \frac{\int \frac{\lambda}{hc}\left[Int_{emitted}^{sample}(\lambda) - Int_{absorbed}^{sample}(\lambda)\right]d\lambda}{\int \frac{\lambda}{hc}\left[Int_{emitted}^{reference}(\lambda) - Int_{absorbed}^{reference}(\lambda)\right]d\lambda}$$

wherein $n_{photon}$ denotes the photon count and Int. the intensity.

**Production and characterization of optoelectronic devices**

Optoelectronic devices, such as OLED devices, comprising organic molecules according to the invention can be produced via vacuum-deposition methods. If a layer contains more than one compound, the weight-percentage of one or more compounds is given in %. The total weight-percentage values amount to 100 %, thus if a value is not given, the fraction of this compound equals to the difference between the given values and 100 %.
The not fully optimized OLEDs are characterized using standard methods and measuring electroluminescence spectra, the external quantum efficiency (in %) in dependency on the intensity, calculated using the light detected by the photodiode, and the current. The OLED device lifetime is extracted from the change of the luminance during operation at constant current density. The LT50 value corresponds to the time, where the measured luminance decreased to 50 % of the initial luminance, analogously LT80 corresponds to the time point, at which the measured luminance decreased to 80 % of the initial luminance, LT 95 to the time point, at which the measured luminance decreased to 95 % of the initial luminance etc. Accelerated lifetime measurements are performed (e.g. applying increased current densities). Exemplarily LT80 values at 500 cd/m$^2$ are determined using the following equation:

$$\mathrm{LT80}\left(500\frac{cd^2}{m^2}\right) = \mathrm{LT80}(L_0)\left(\frac{L_0}{500\frac{cd^2}{m^2}}\right)^{1.6}$$

wherein $L_0$ denotes the initial luminance at the applied current density.
The values correspond to the average of several pixels (typically two to eight), the standard deviation between these pixels is given.

**HPLC-MS**

HPLC-MS analysis is performed on an HPLC by Agilent (1100 series) with MS-detector (Thermo LTQ XL). Exemplary a typical HPLC method is as follows: a reverse phase column 4,6mm x 150mm, particle size 3,5 µm from Agilent (*ZORBAX Eclipse Plus 95Å C18,* 4.6 x *150 mm, 3.5 µm HPLC column*) is used in the HPLC. The HPLC-MS measurements are performed at room temperature (rt) following gradients

| Flow rate [ml/min] | time [min] | A[%] | B[%] | C[%] |
|---|---|---|---|---|
| 2.5 | 0 | 40 | 50 | 10 |
| 2.5 | 5 | 40 | 50 | 10 |
| 2.5 | 25 | 10 | 20 | 70 |
| 2.5 | 35 | 10 | 20 | 70 |
| 2.5 | 35.01 | 40 | 50 | 10 |

(continued)

| Flow rate [ml/min] | time [min] | A[%] | B[%] | C[%] |
|---|---|---|---|---|
| 2.5 | 40.01 | 40 | 50 | 10 |
| 2.5 | 41.01 | 40 | 50 | 10 |

using the following solvent mixtures:

| | | |
|---|---|---|
| solvent A: | $H_2O$ (90%) | MeCN (10%) |
| solvent B: | $H_2O$ (10%) | MeCN (90%) |
| solvent C: | THF (50%) | MeCN (50%) |

An injection volume of 5 µL from a solution with a concentration of 0.5 mg/mL of the analyte is taken for the measurements.

Ionization of the probe is performed using an APCI (atmospheric pressure chemical ionization) source either in positive (APCI +) or negative (APCI -) ionization mode.

**Example 1**

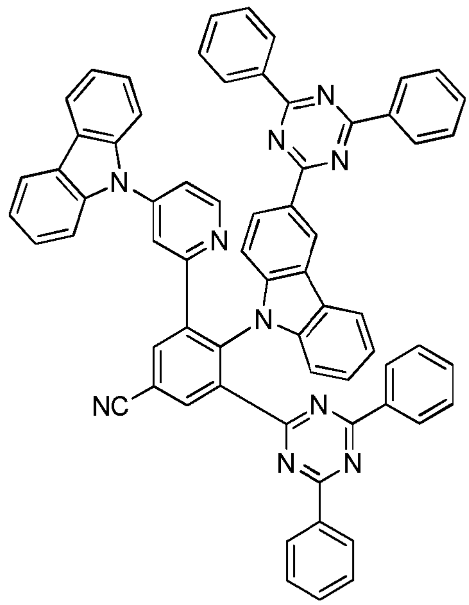

Example **1** was synthesized according to

***AAV1*** (yield 56%), wherein 3-chloro-4-fluoro-5-(4,4,5,5-tetramethyl-1,3,2-dioxaborolan-2-yl)benzonitrile **(E1),** 2-chloro-4,6-diphenyl-1,3,5-triazine (**E2;** CAS 3842-55-5) were used as a reactants,
***AAV2*** (yield 65%), wherein 3-chloro-4-fluoro-5-triazinylbenzonitrile **(E3),** bis(pinacolato)diboron (CAS 73183-34-3) were used as a reactants,
***AAV3*** (yield 41%), wherein 2-Bromo-4-fluoropyridine **(E5;** CAS 357927-50-5), carbazole (**E6;** CAS 86-74-8) were used as a reactants,
***AAV4*** (yield 80%), wherein 3-(4,4,5,5-tetramethyl-1,3,2-dioxaborolan-2-yl)-4-fluoro-5-triazinylbenzonitrile **(E4),** 1-bromo-3-carbazolylpyridine (**E7;** CAS 3842-55-5) were used as reactants, and
***AAV5*** (yield 2%), wherein 3-(3-carbazolylpyridin-1-yl)-4-fluoro-5-triazinylbenzonitrile **(Z),** 3-triazinylcarbazole **(D-H)** were used as reactants, wherein 3-triazinylcarbazole was synthesized according to:

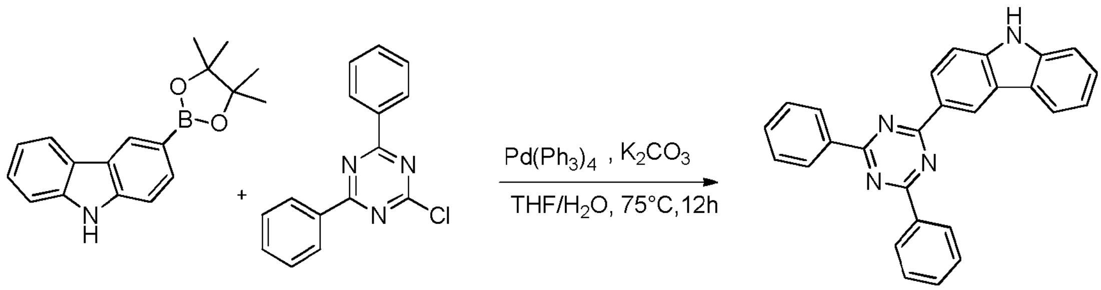


wherein a solution of 3-(4,4,5,5-tetramethyl-1,3,2-dioxaborolan-2-yl)carbazole(1.0 equivalent; CAS 855738-89-5), 2-chloro-4,6-diphenyl-1,3,5-triazine (1.3 equivalent; CAS:3842-55-5), potassium carbonate (3.0equivalent) (CAS: 584-08-7) in tetrahydrofuran and water was degassed with nitrogen, tetrakis(triphenylphosphine)palladium(0) (0.05 equivalent) (CAS 14221-01-3) was added and the solution was again degassed with nitrogen. The reaction mixture was stirred at 75 °C for 12 h. After cooling down to room temperature (RT), the reaction mixture was extracted with ethyl acetate. The organic phases were collected, washed with brine and dried over $MgSO_4$. The organic solvent was removed, the crude product was purified by column chromatography and the pure compound was isolated as a white yellowish solid (yield: 40 %).

HPLC-MS (example **1**): 972.47 m/z (100 %).

Figure 1 depicts the emission spectrum of example **1** (10 % by weight in PMMA). The emission maximum ($\lambda_{max}$) is at 467 nm. The photoluminescence quantum yield (PLQY) is 55 %, the full width at half maximum (FWHM) is 0.45 eV and the excited-state lifetime was determined at 9 μs. The resulting $CIE_x$ coordinate is determined at 0.17 and the $CIE_y$ coordinate at 0.21.

**Additional Examples of Organic Molecules of the Invention**

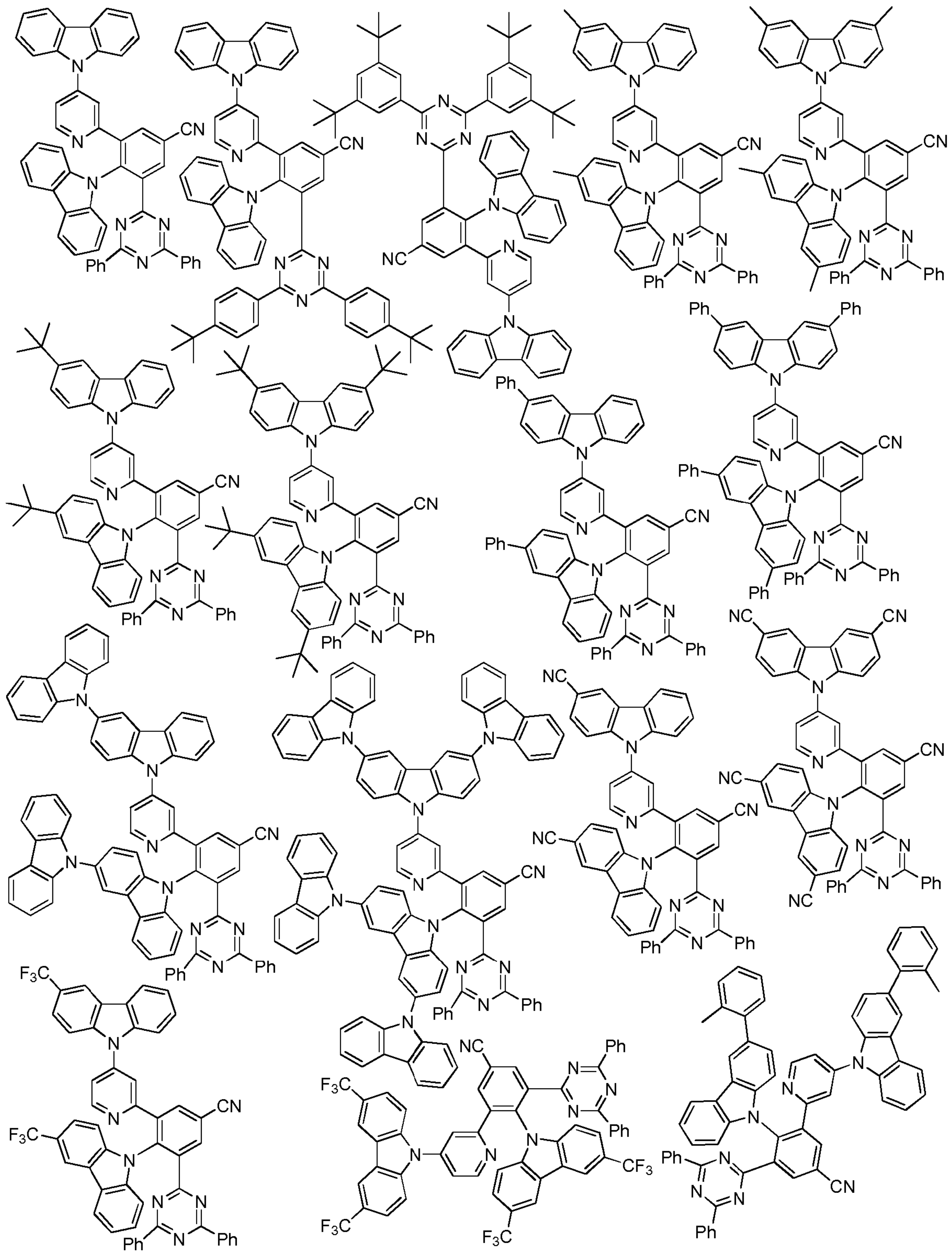

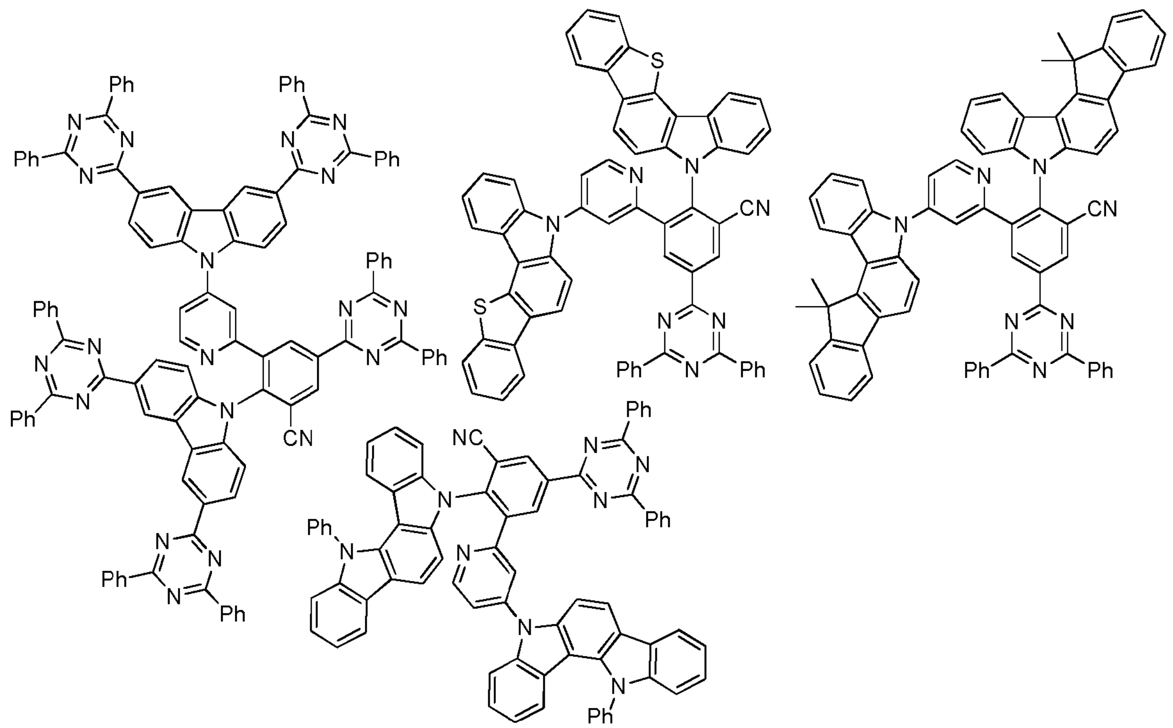

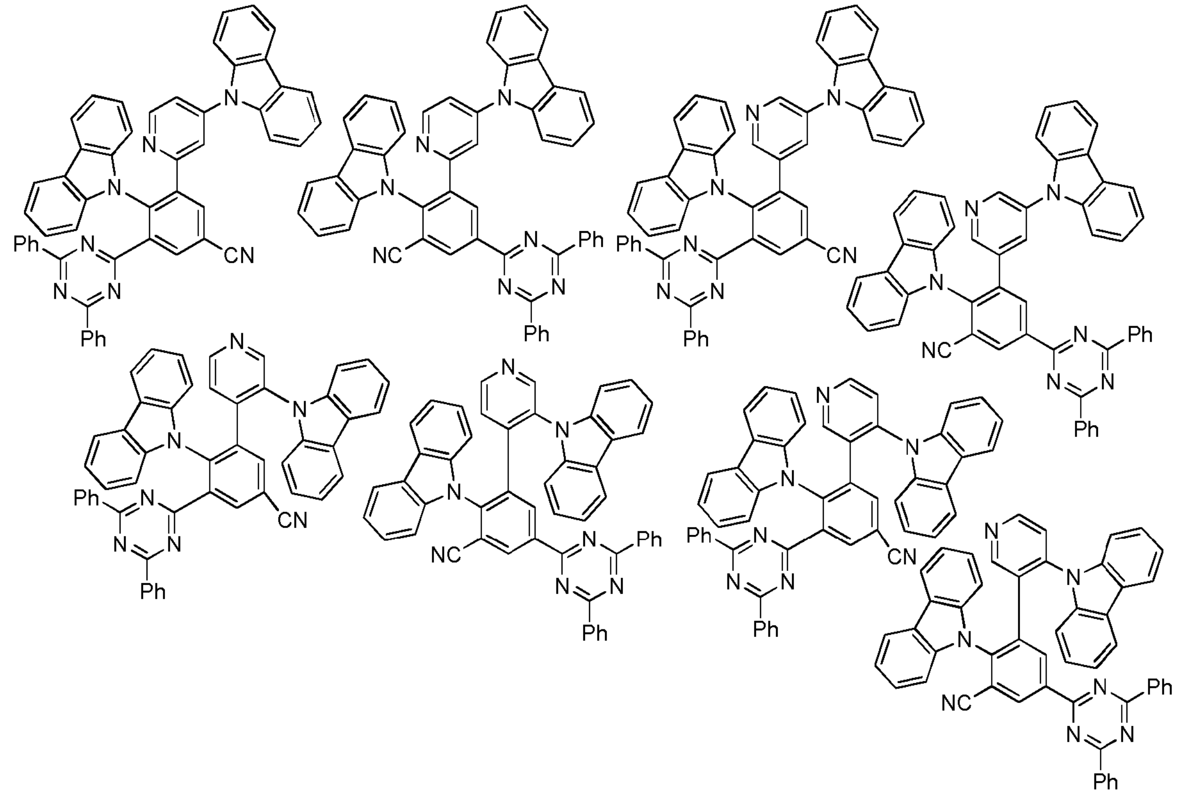

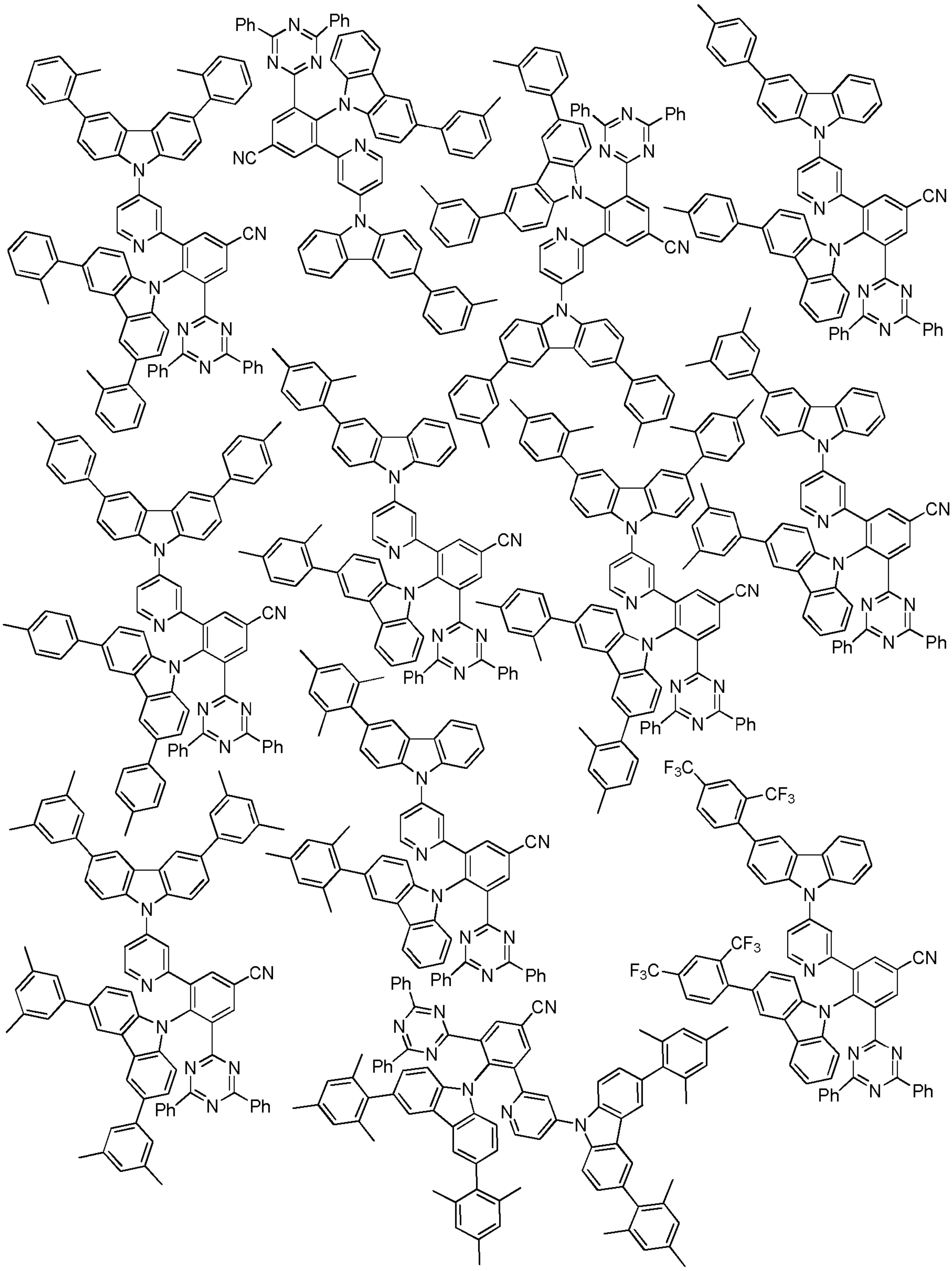

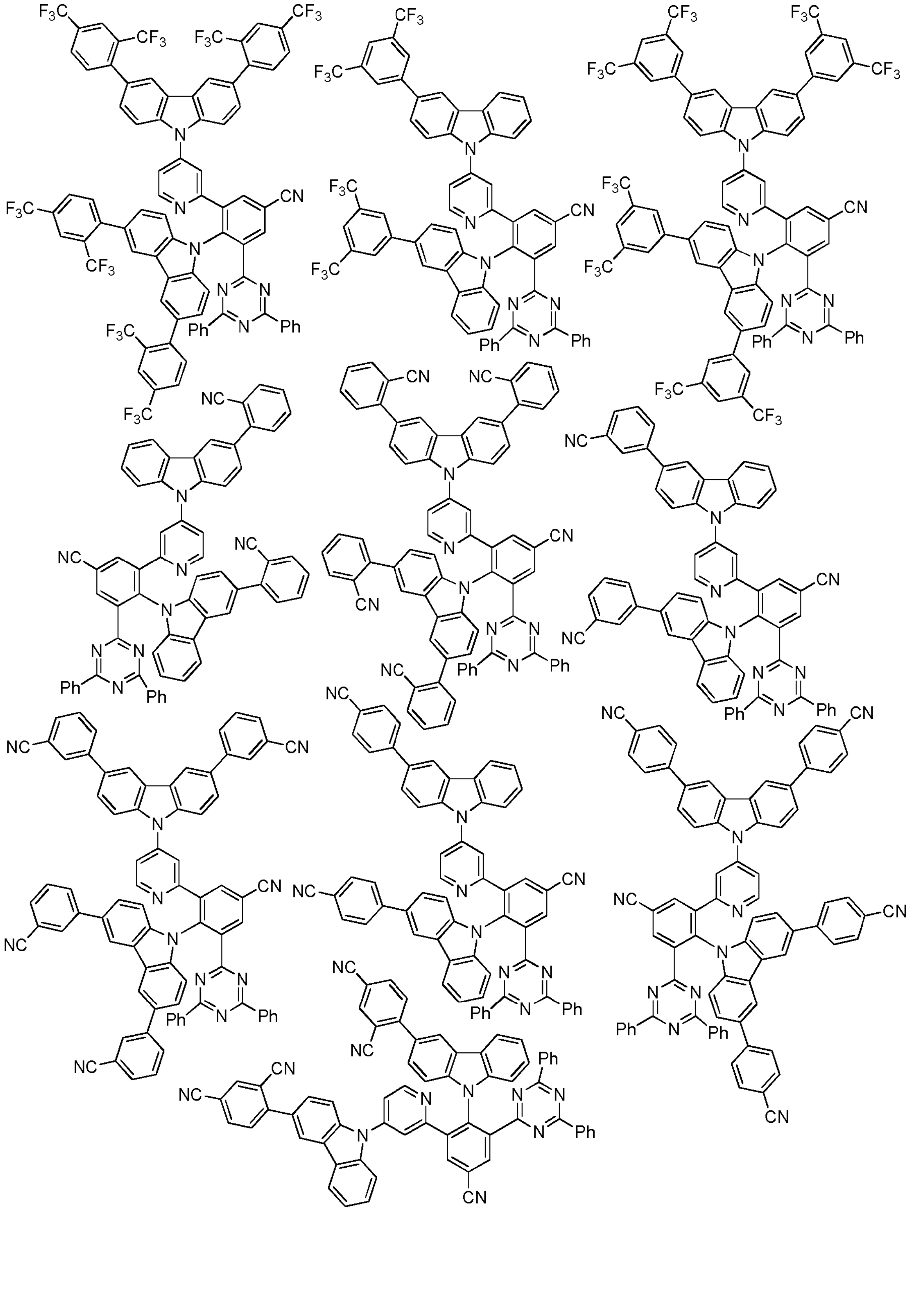

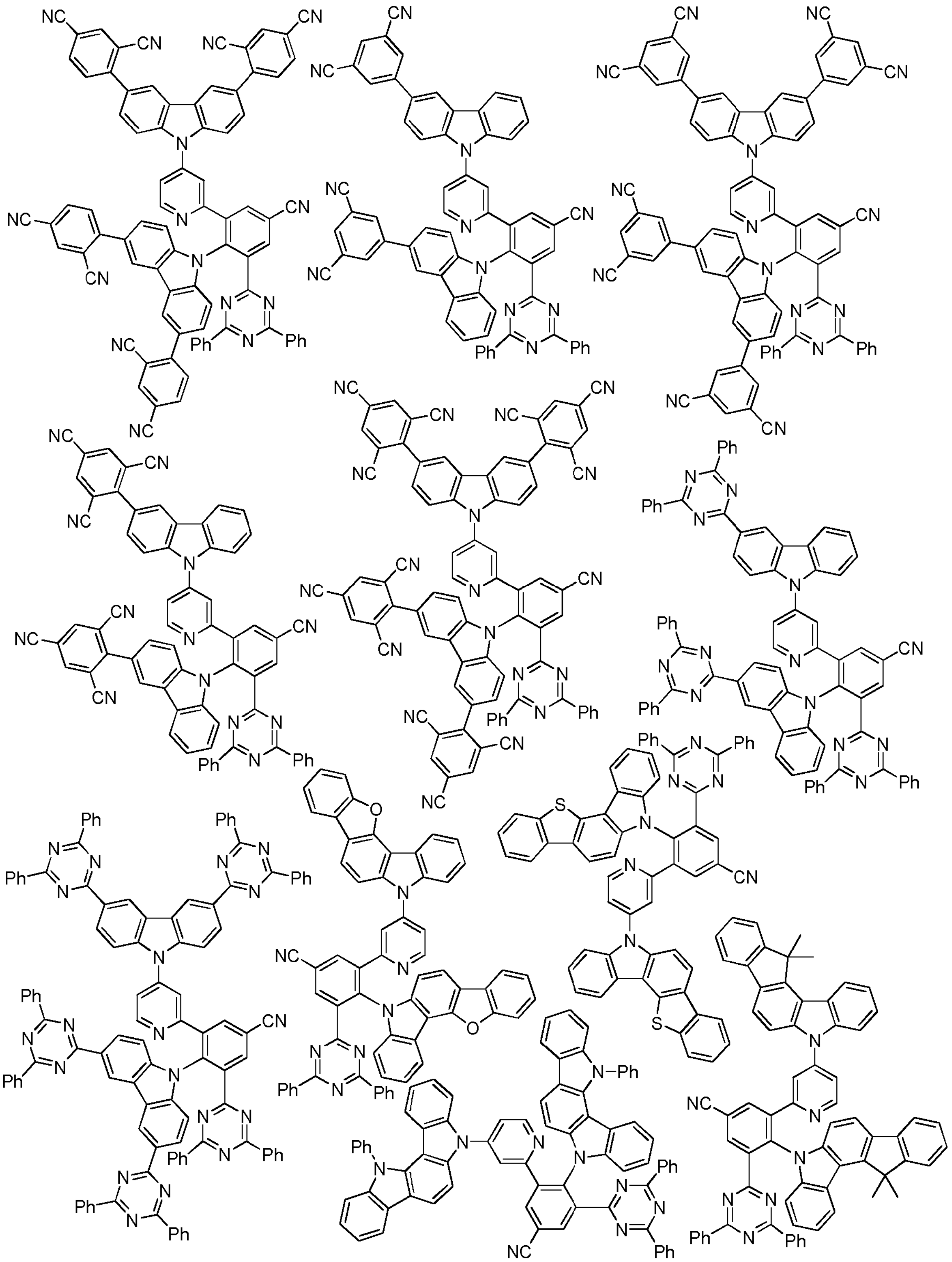

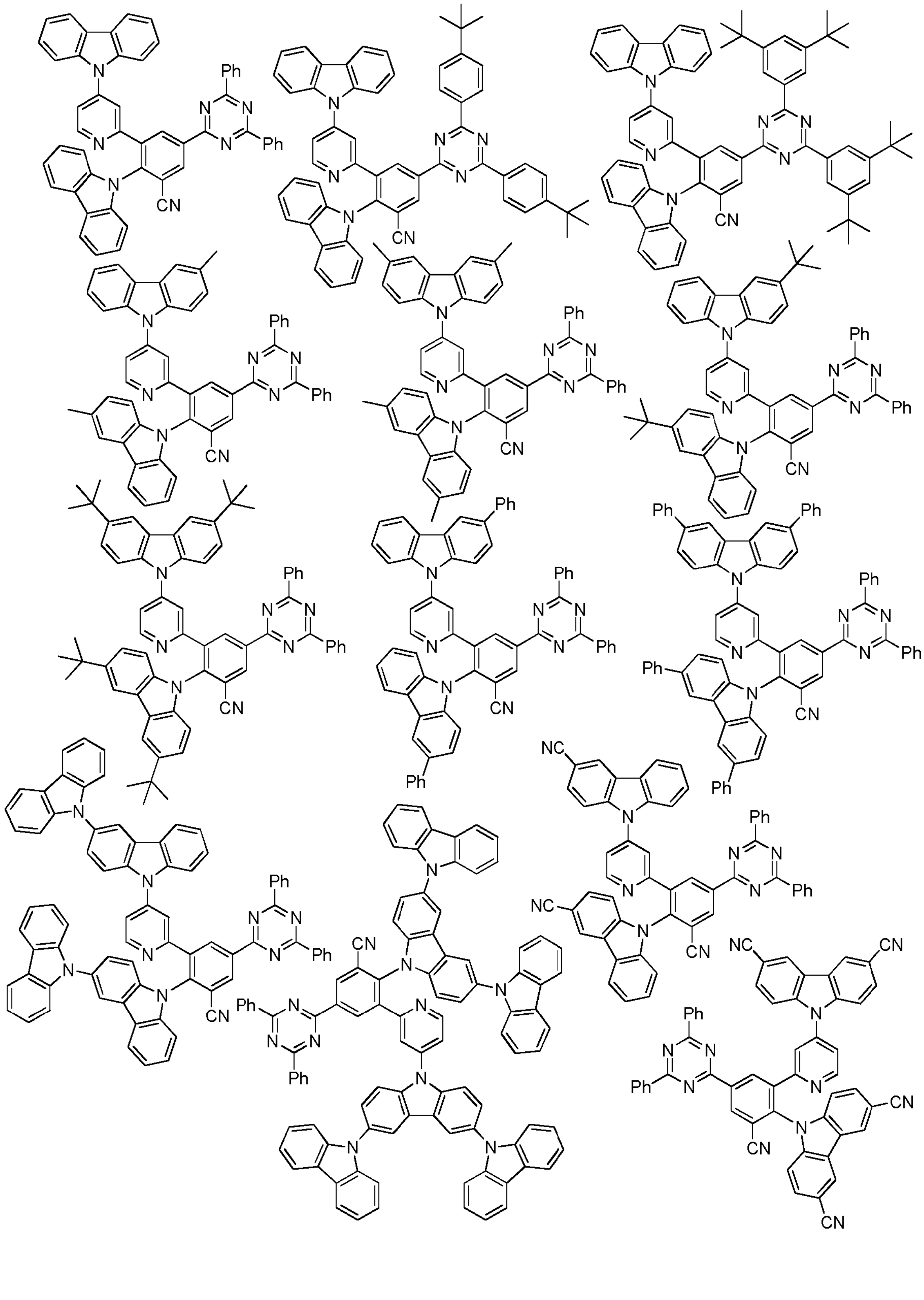

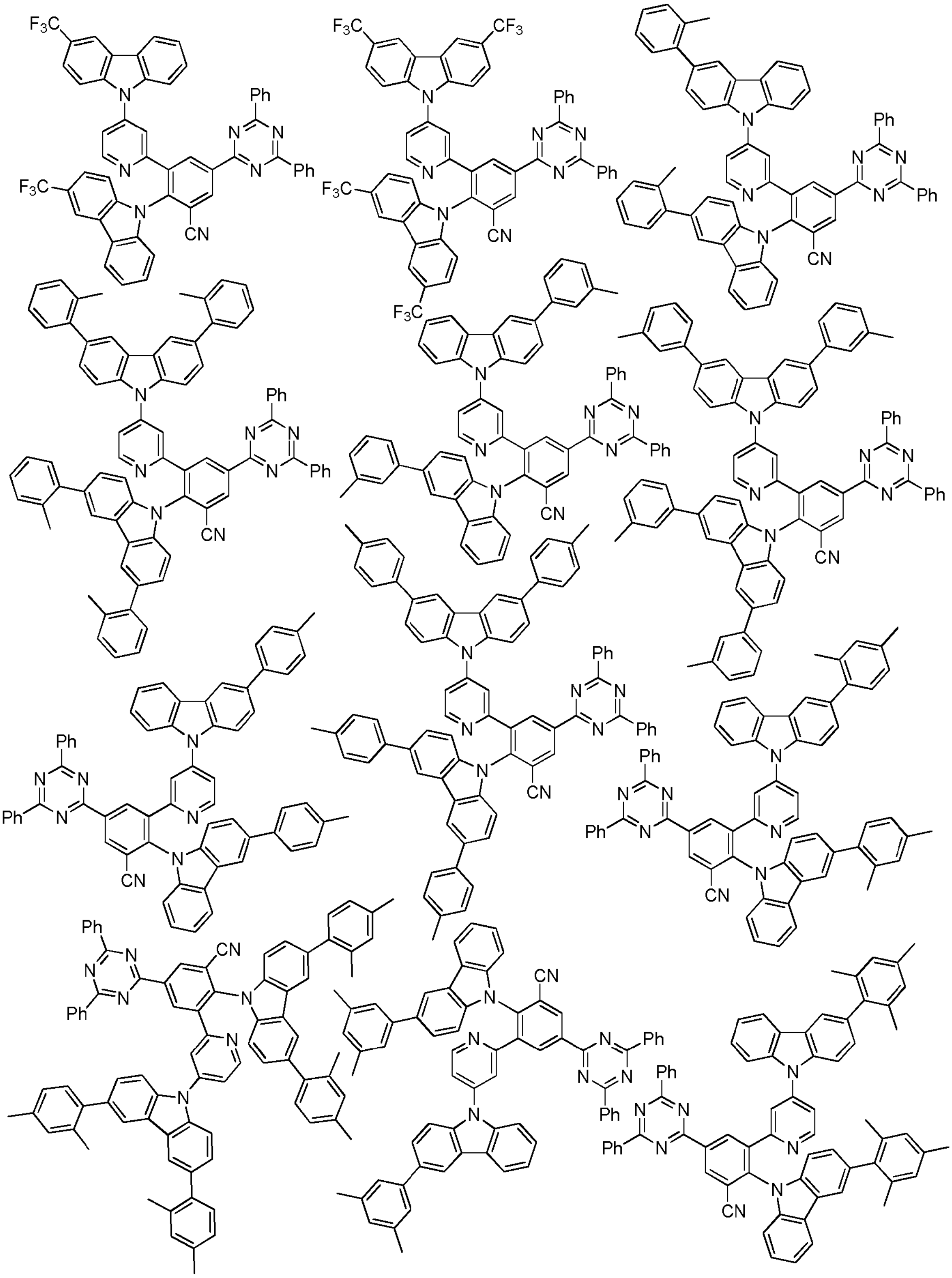

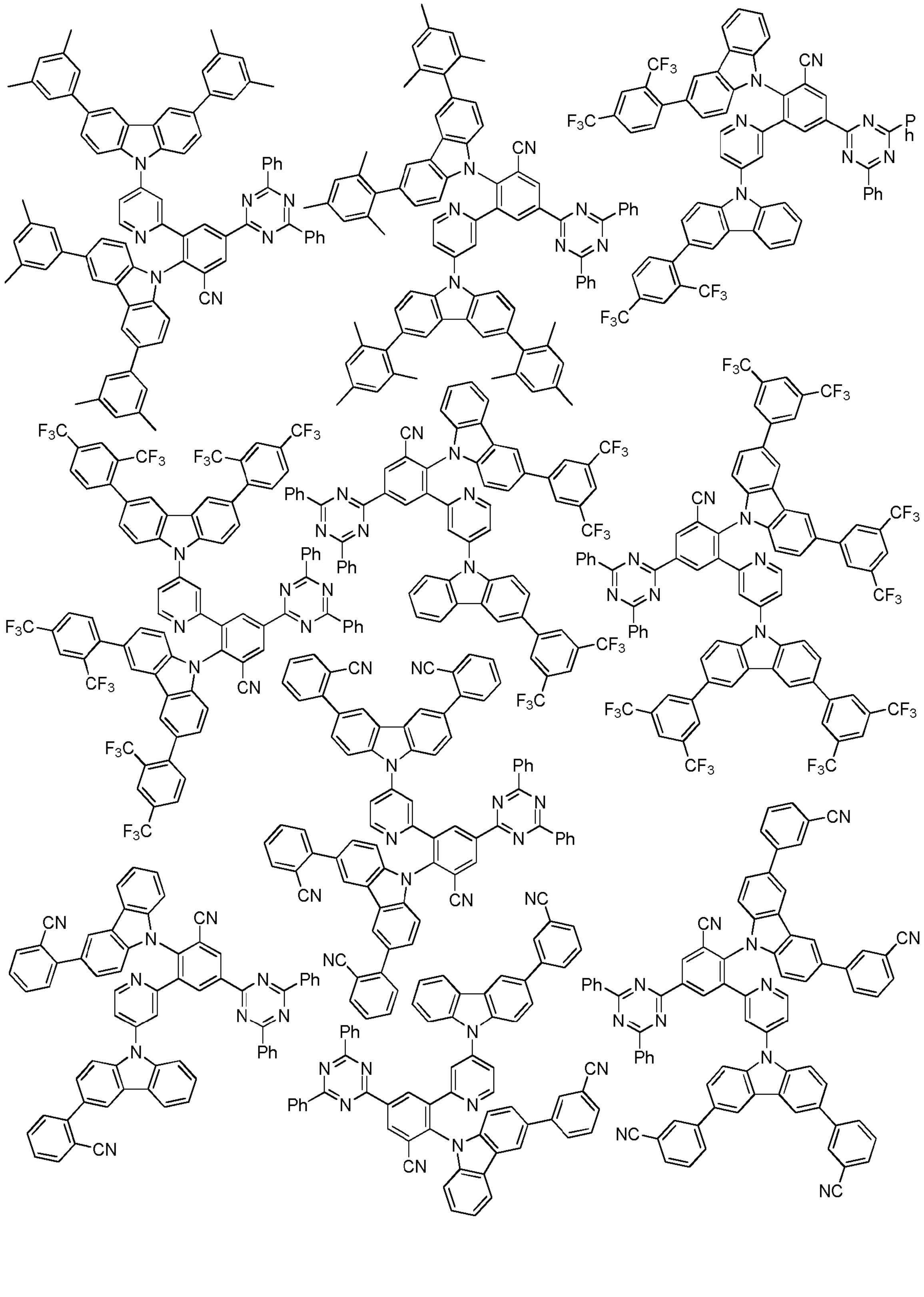

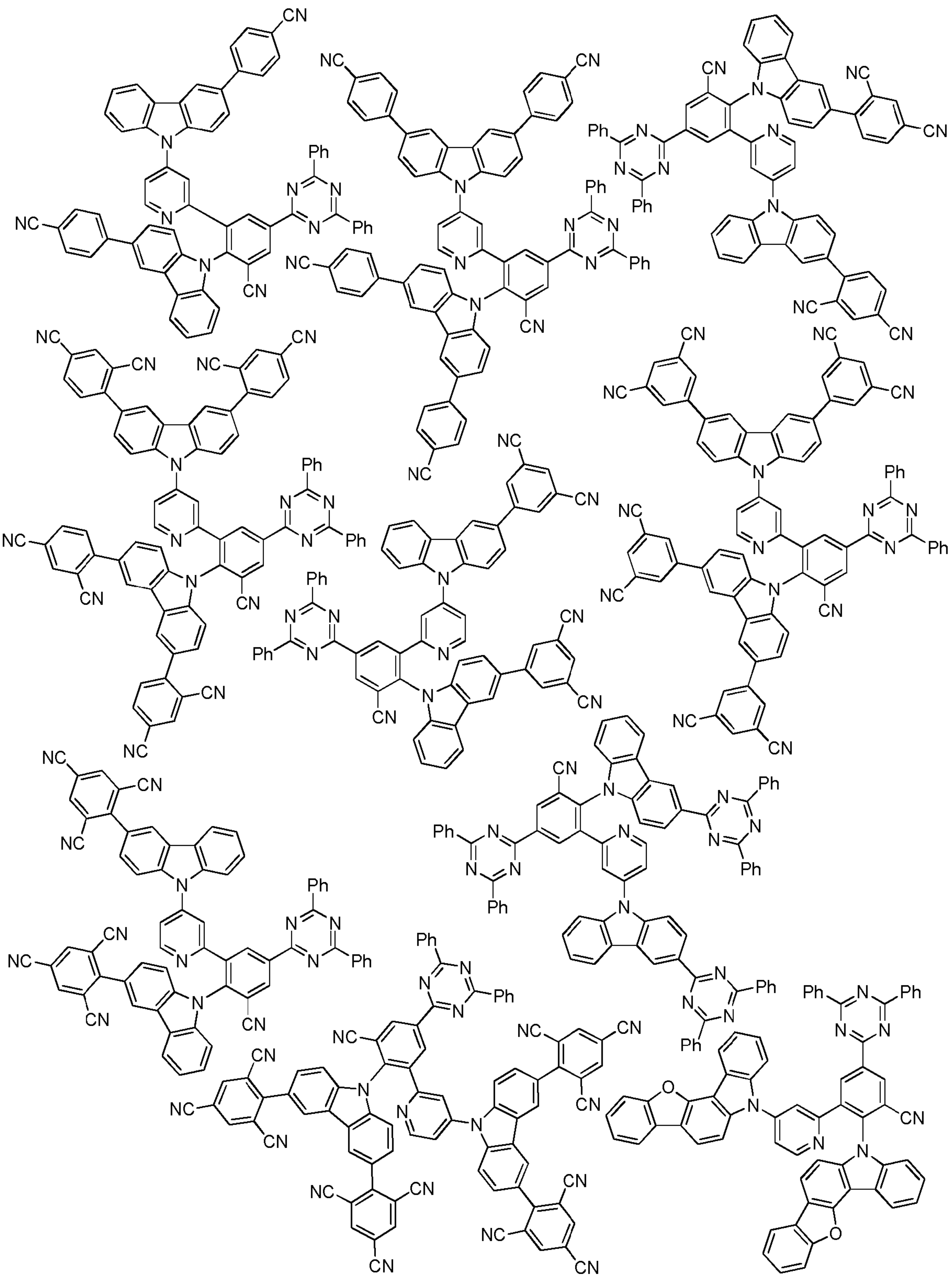

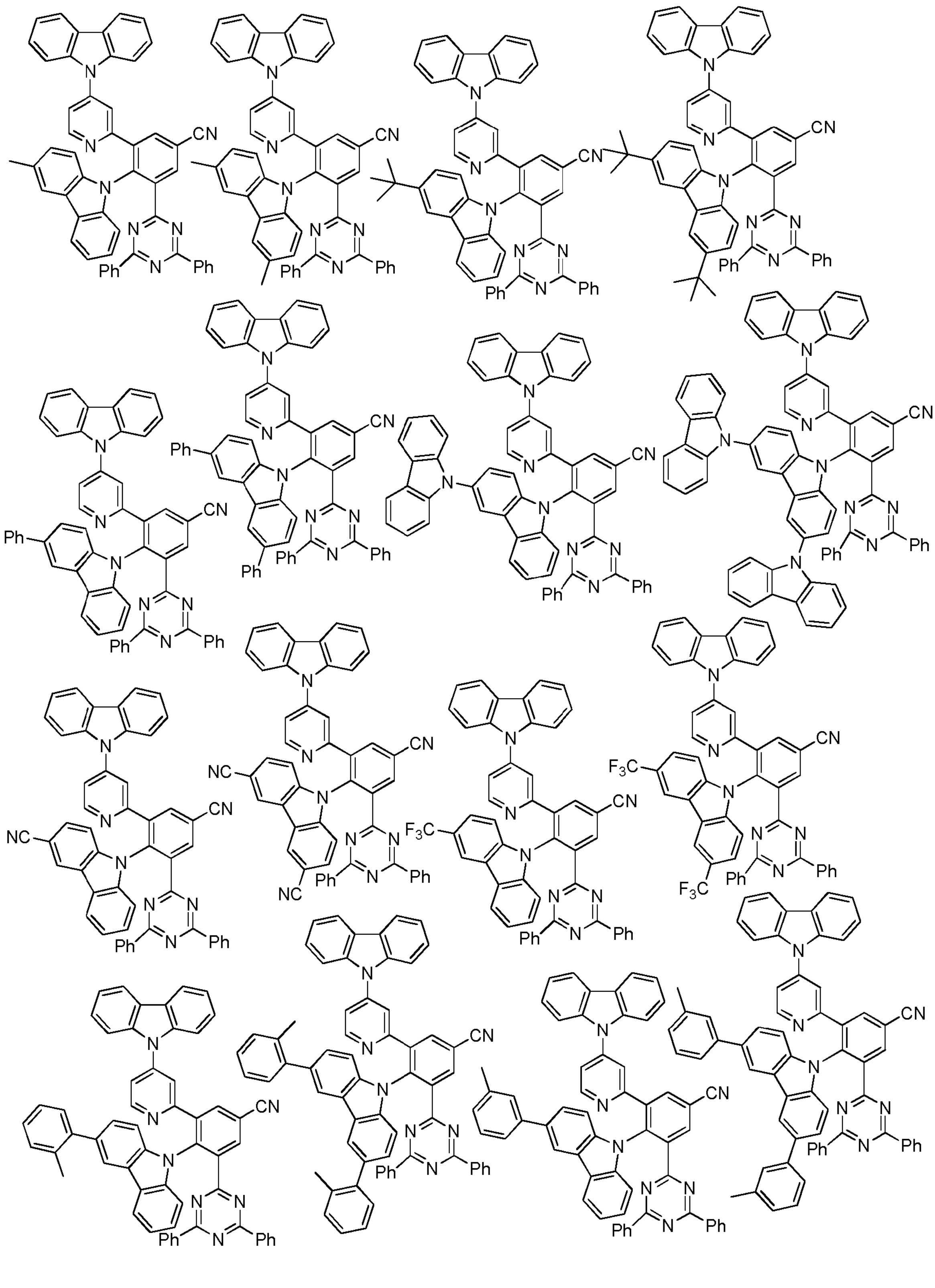

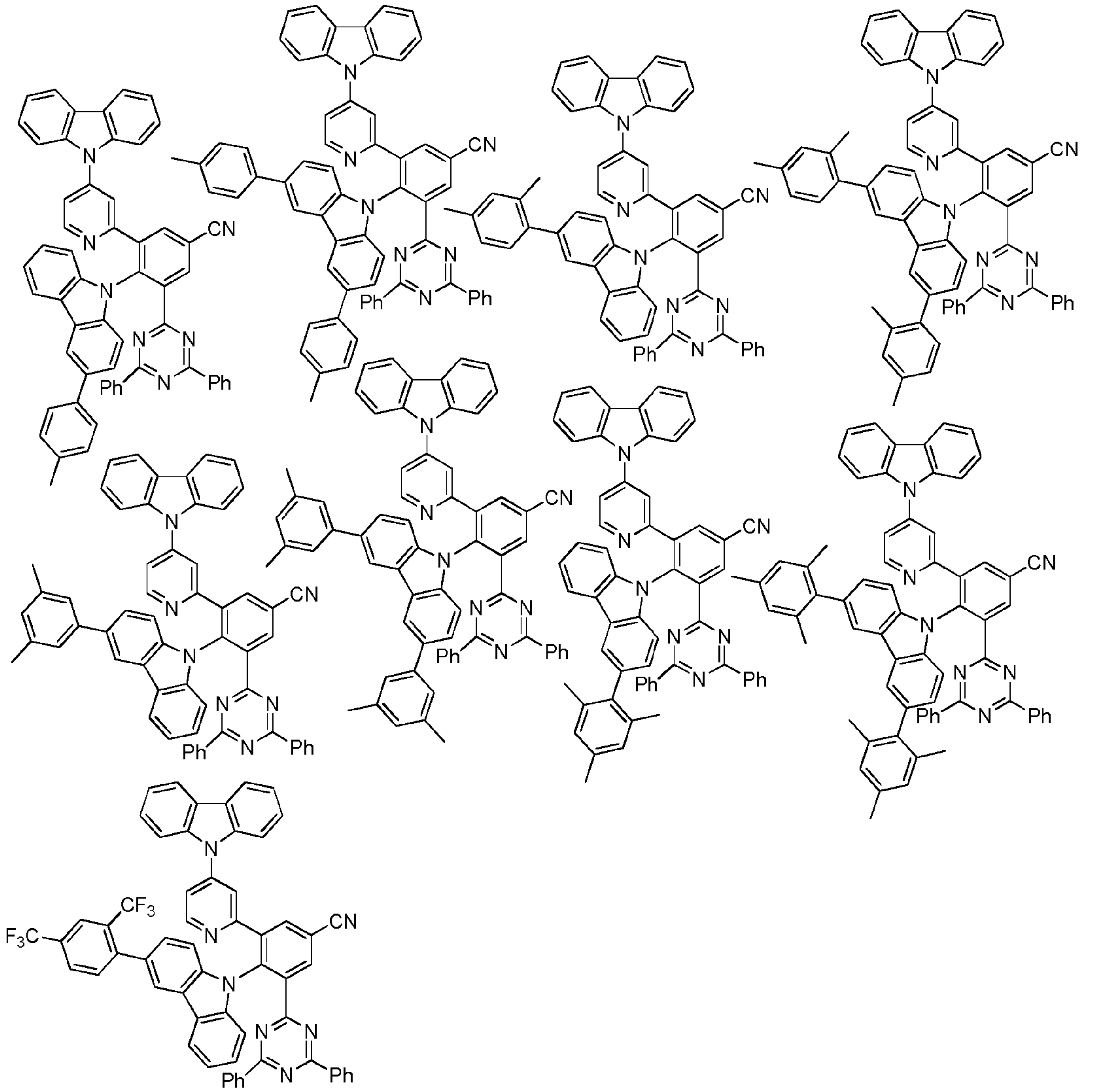

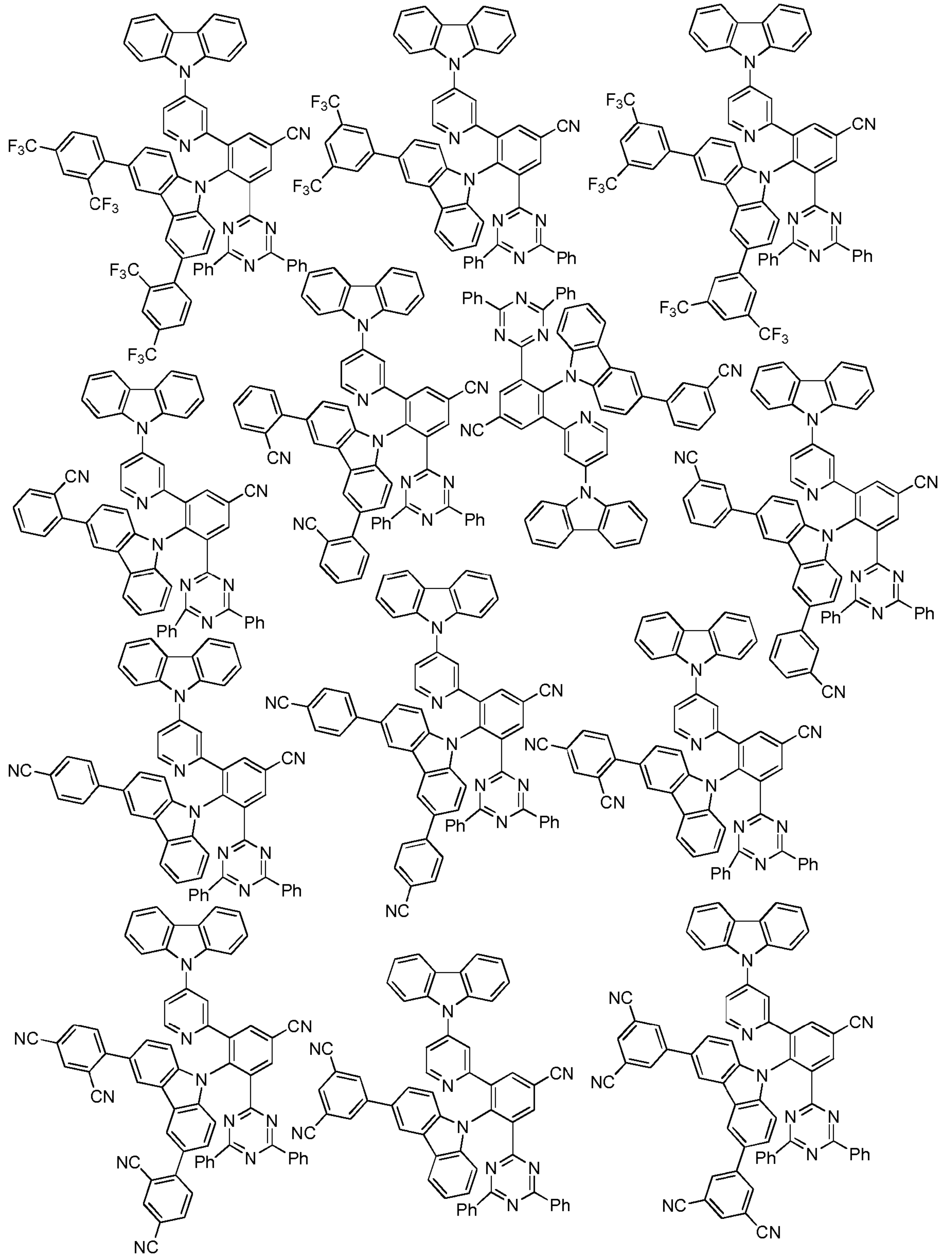

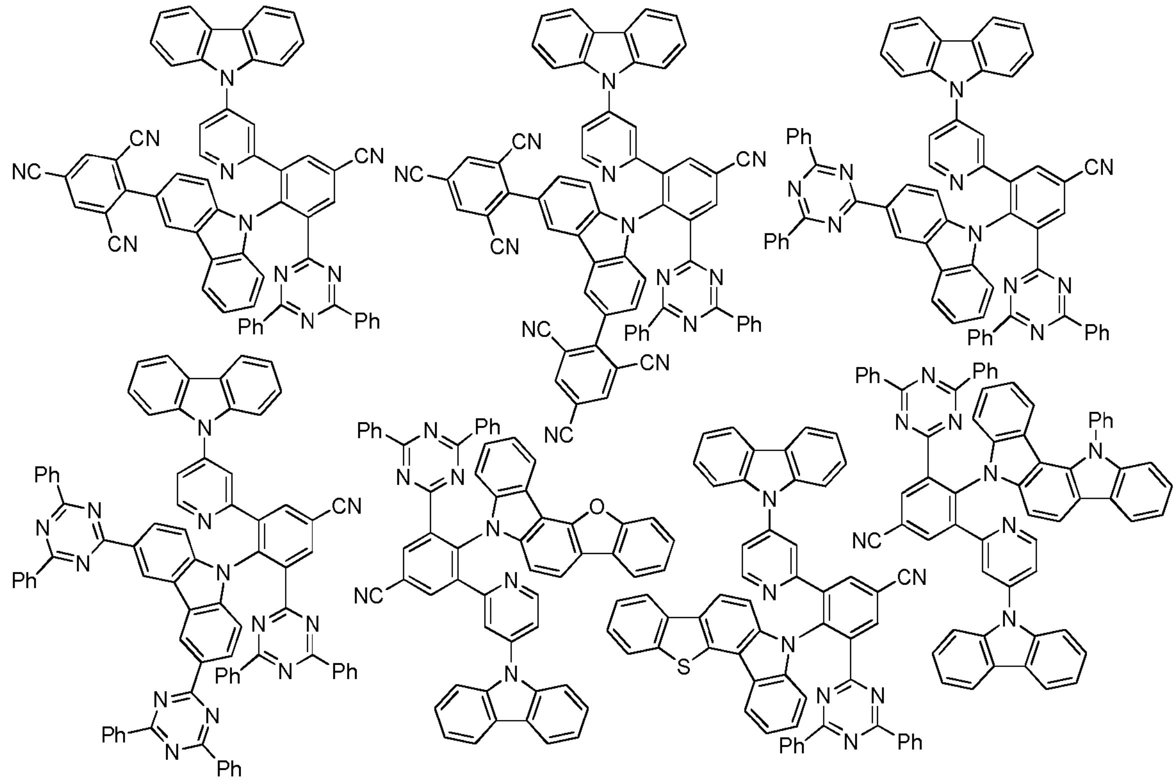

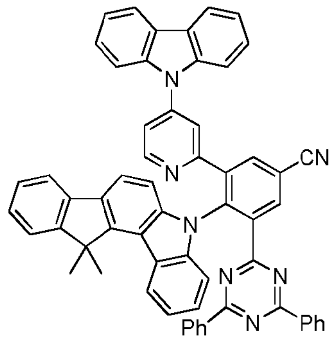

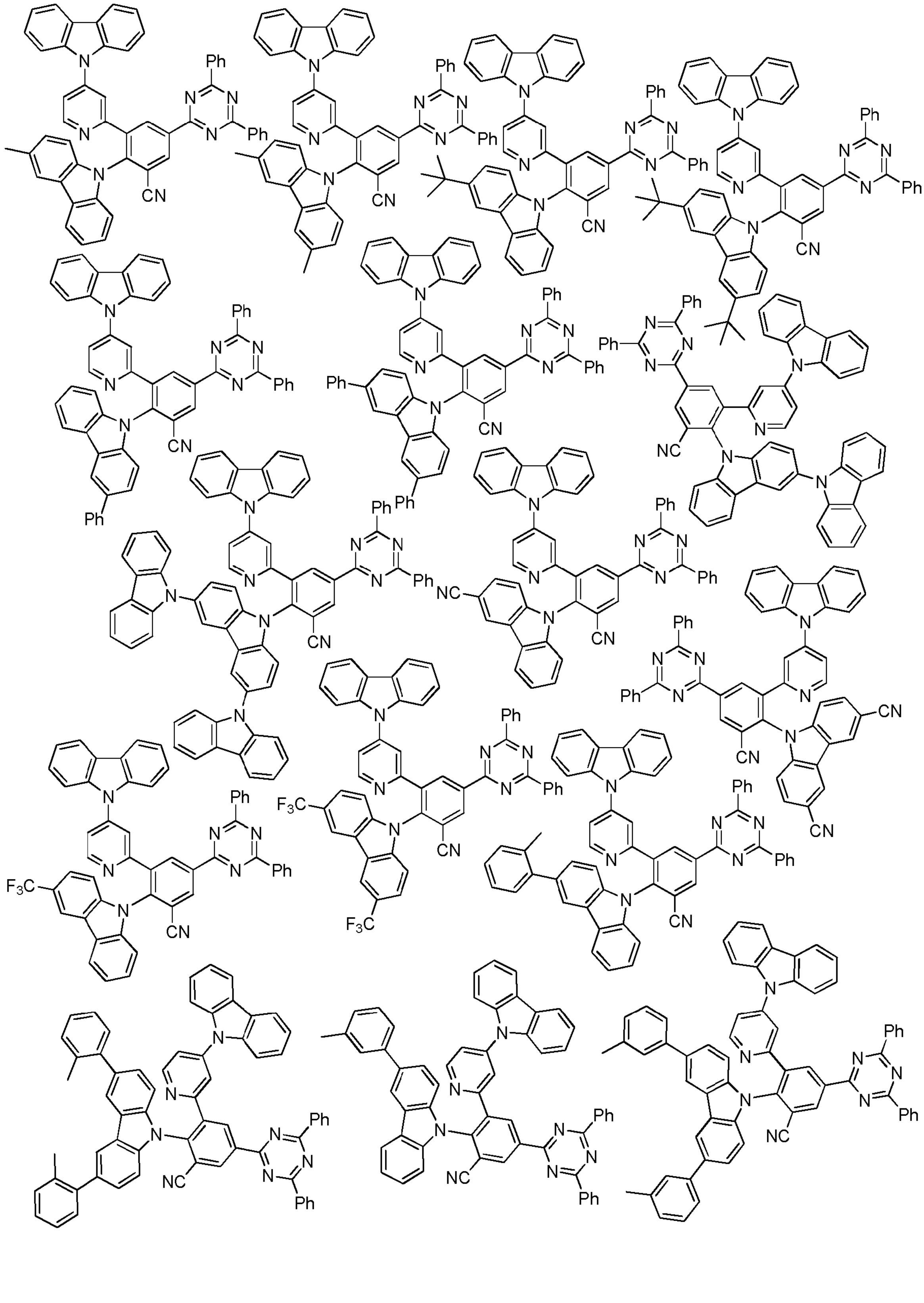

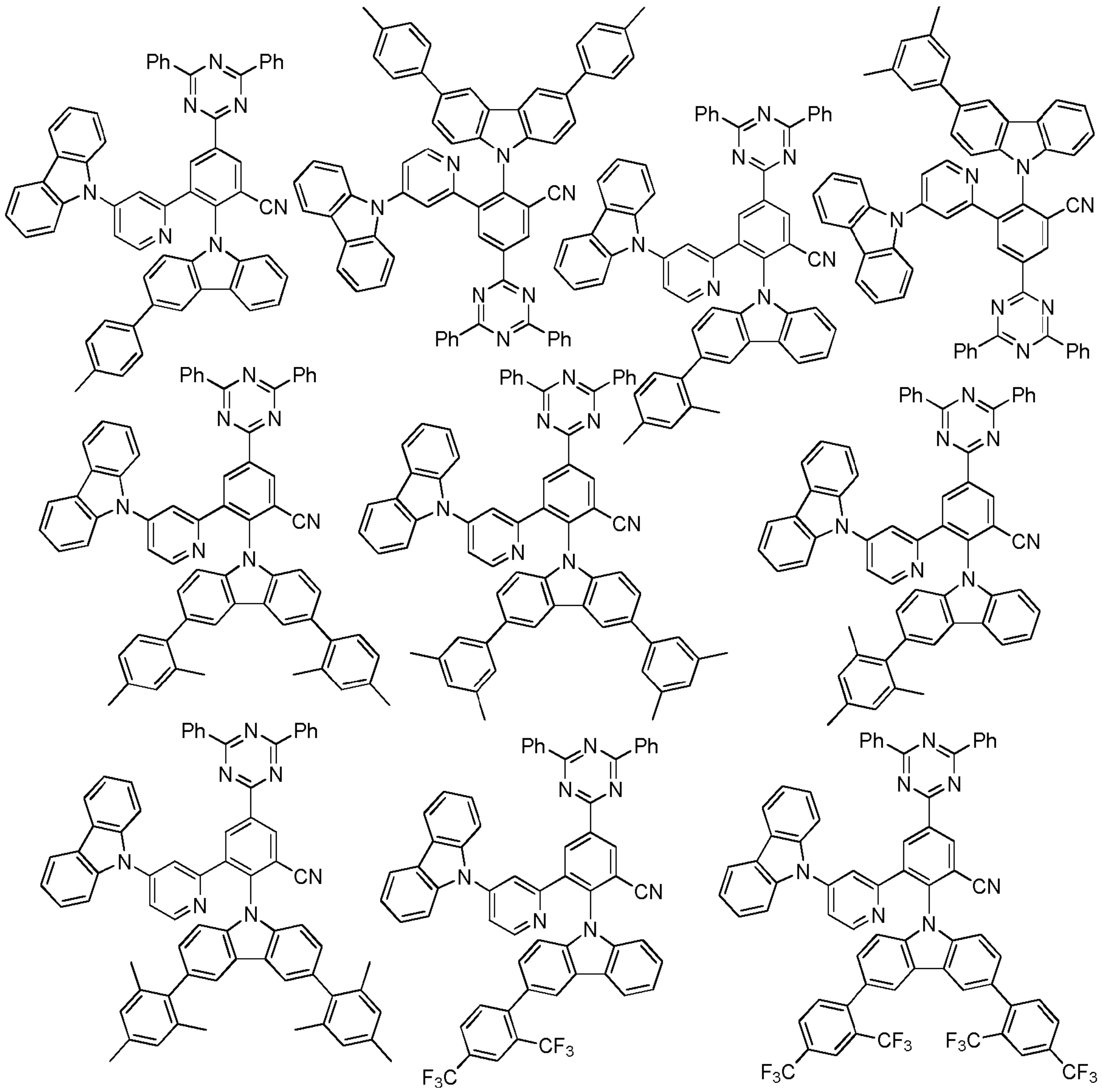

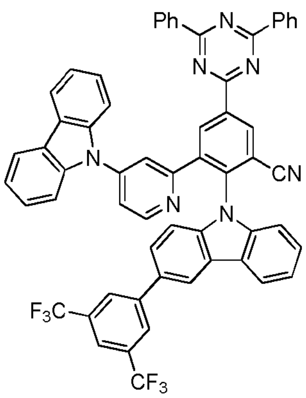

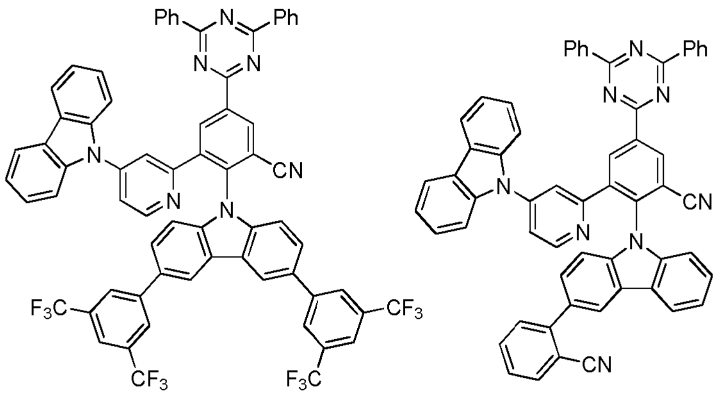

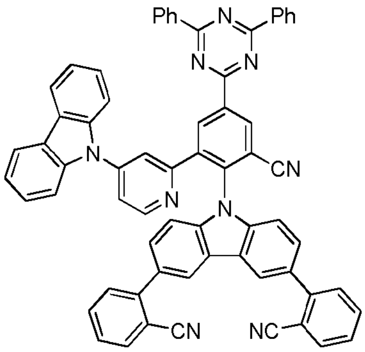

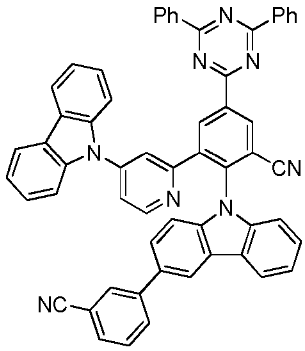

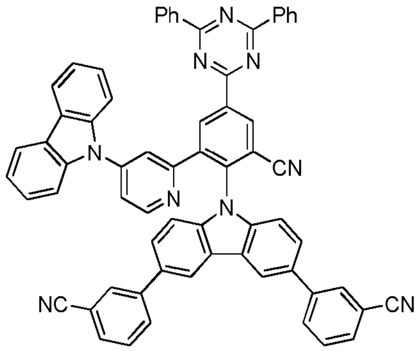

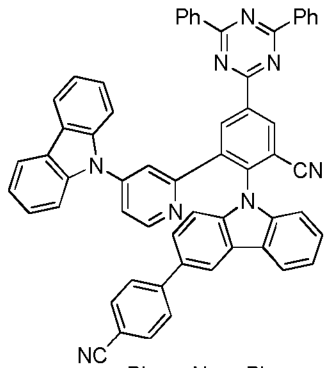

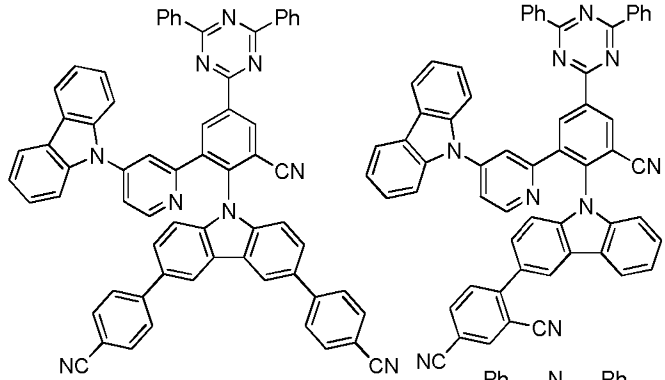

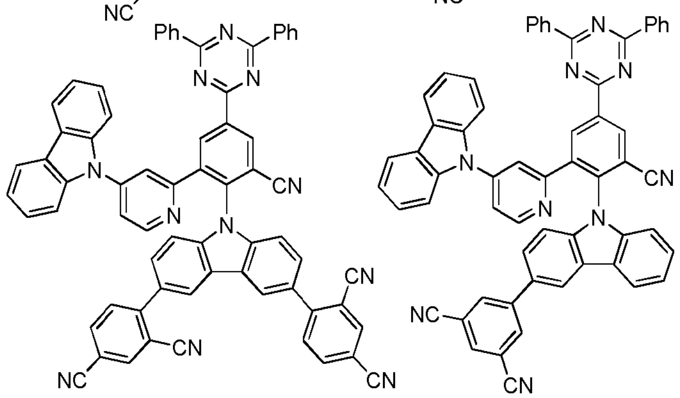

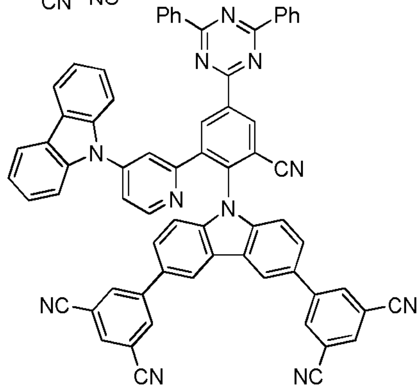

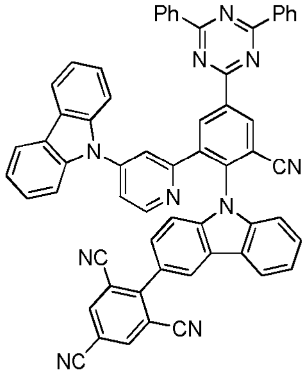

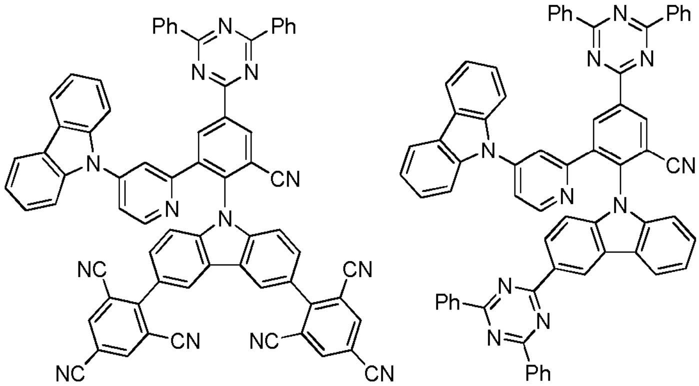

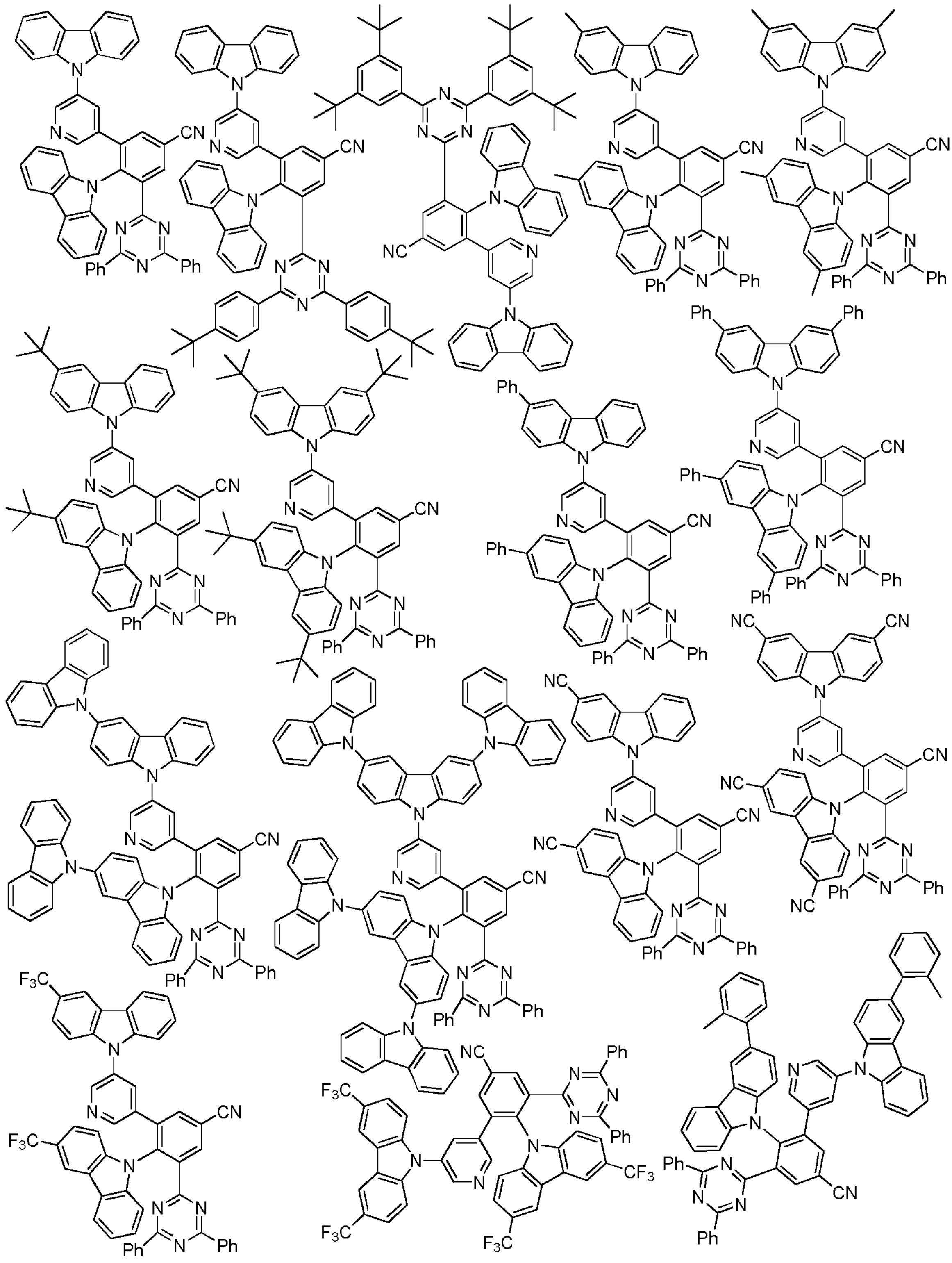

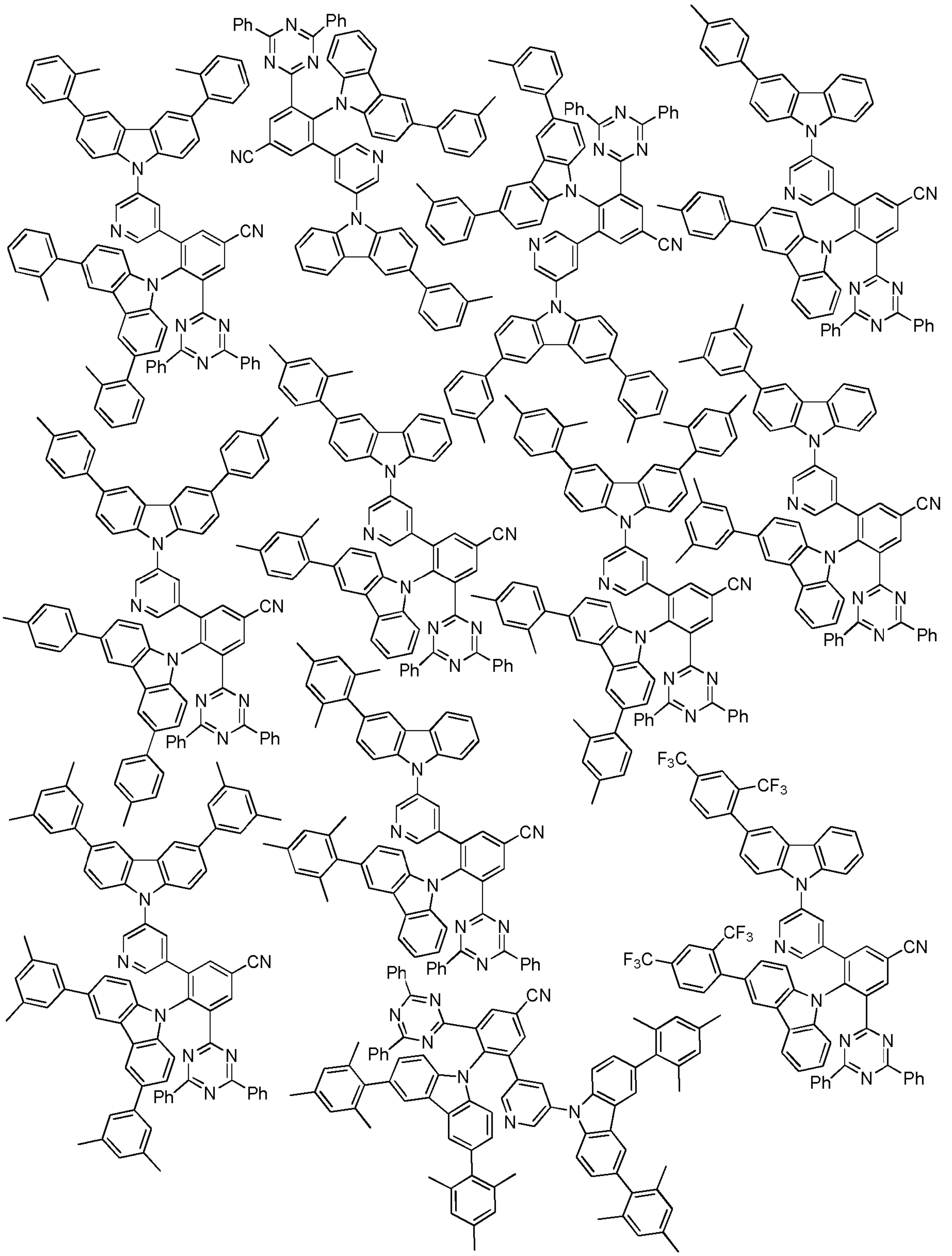

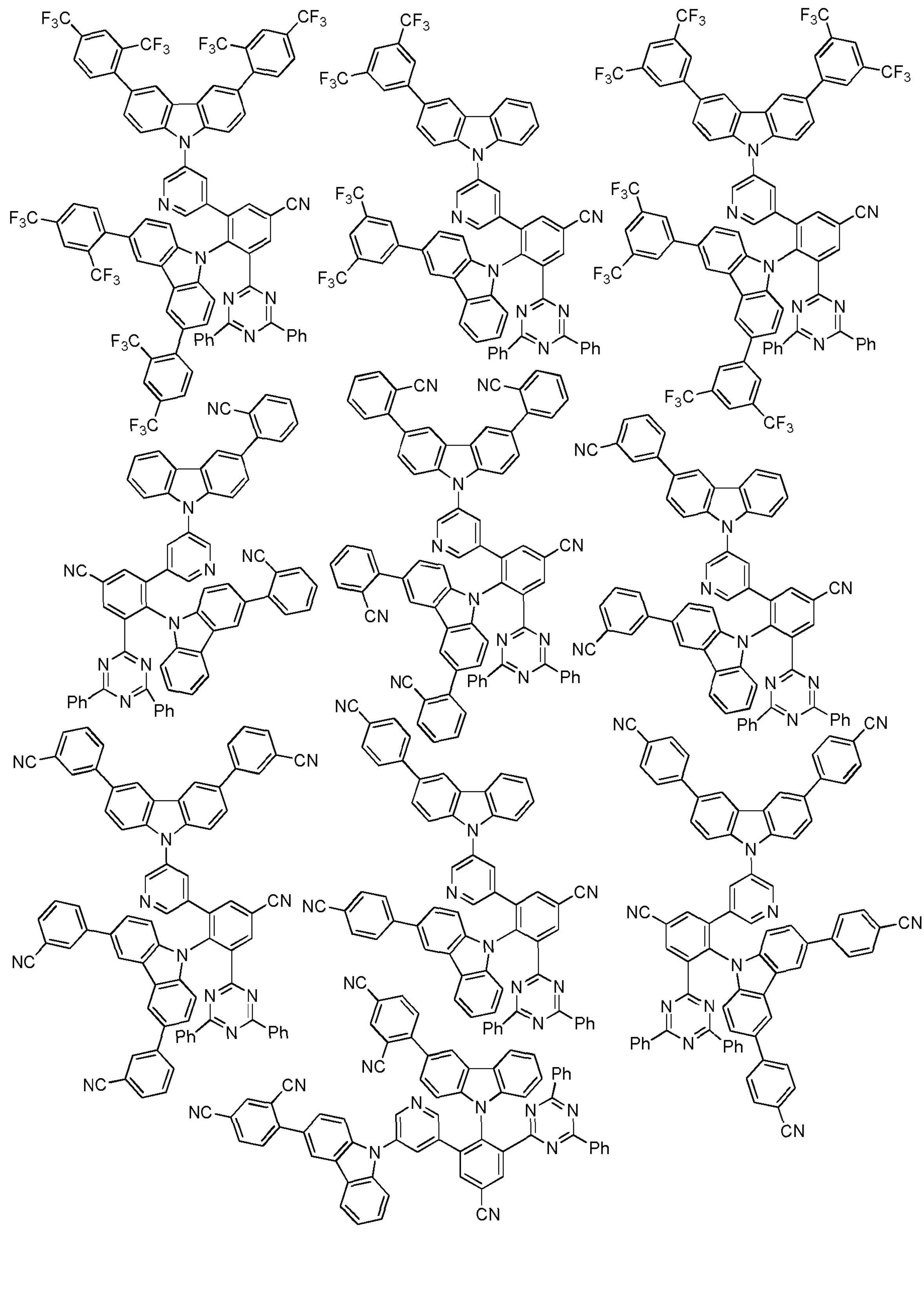

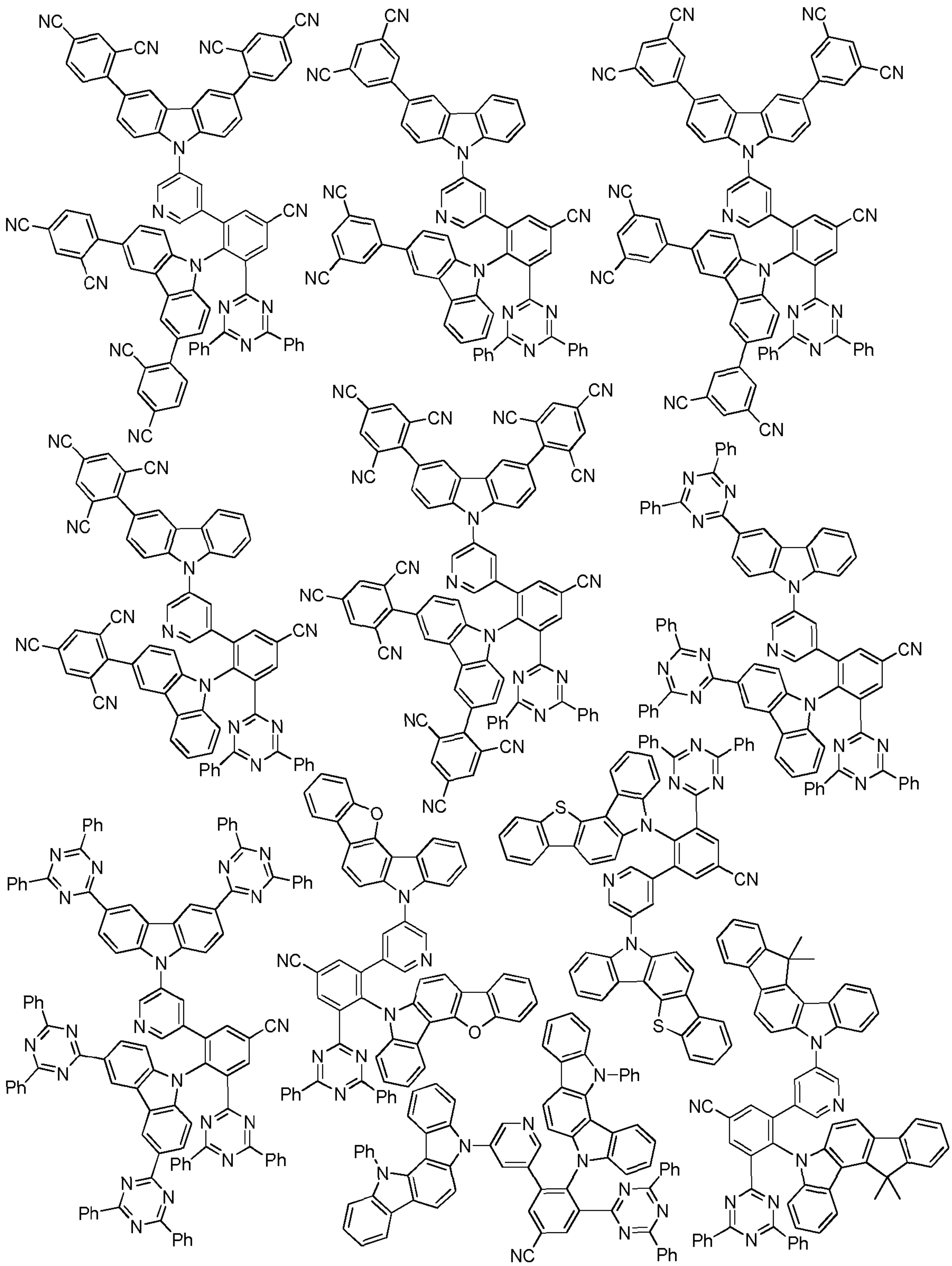

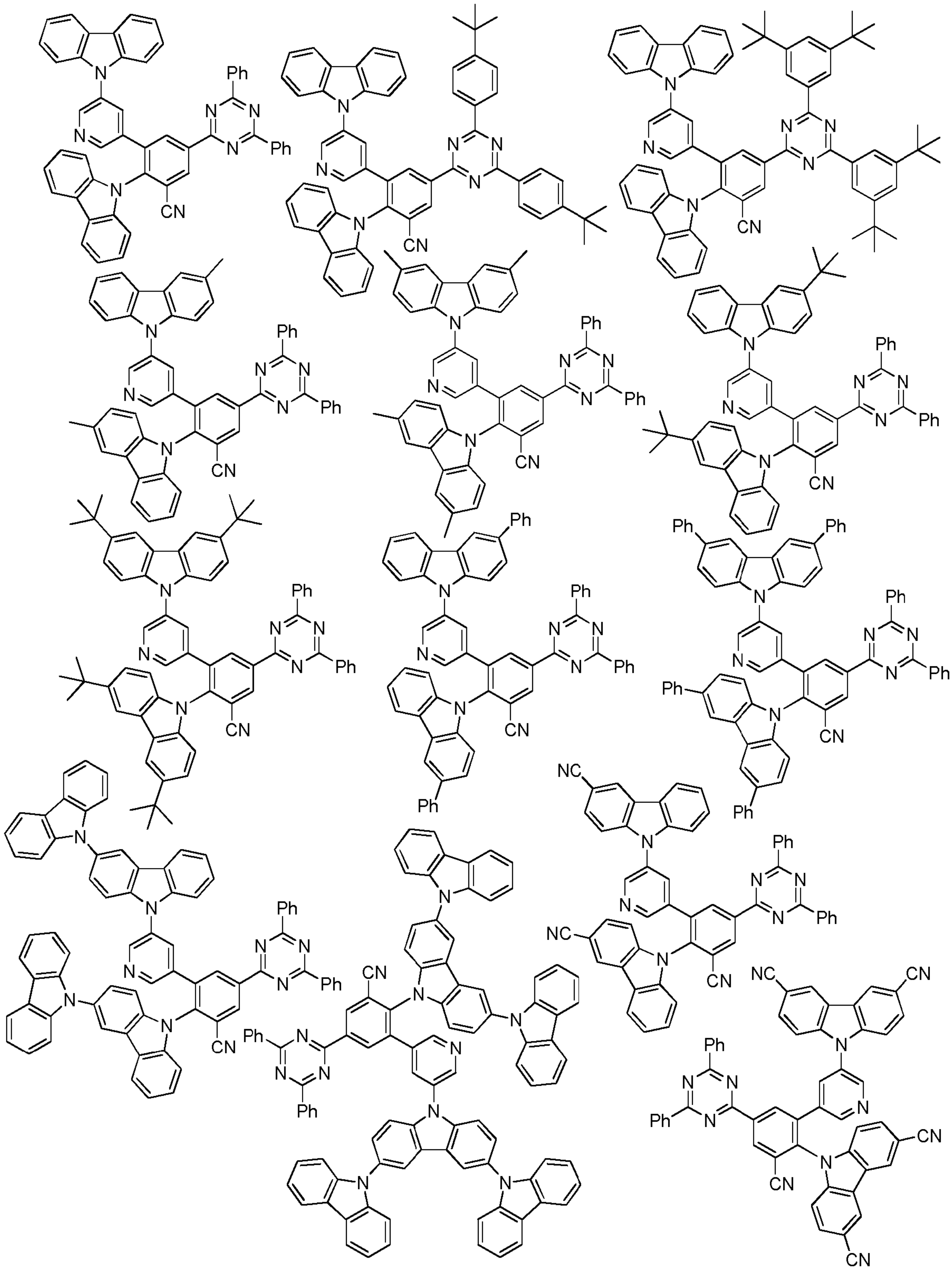

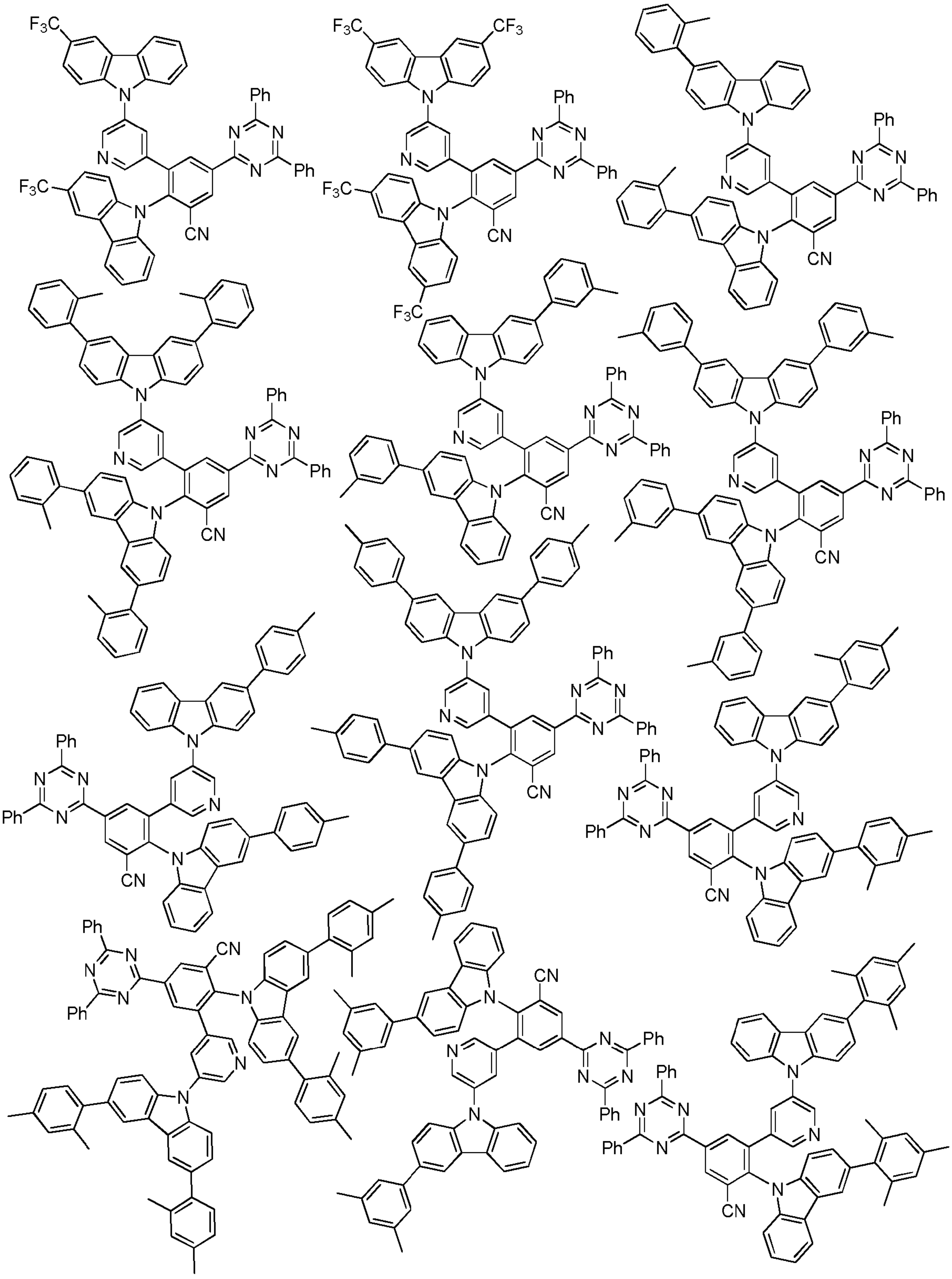

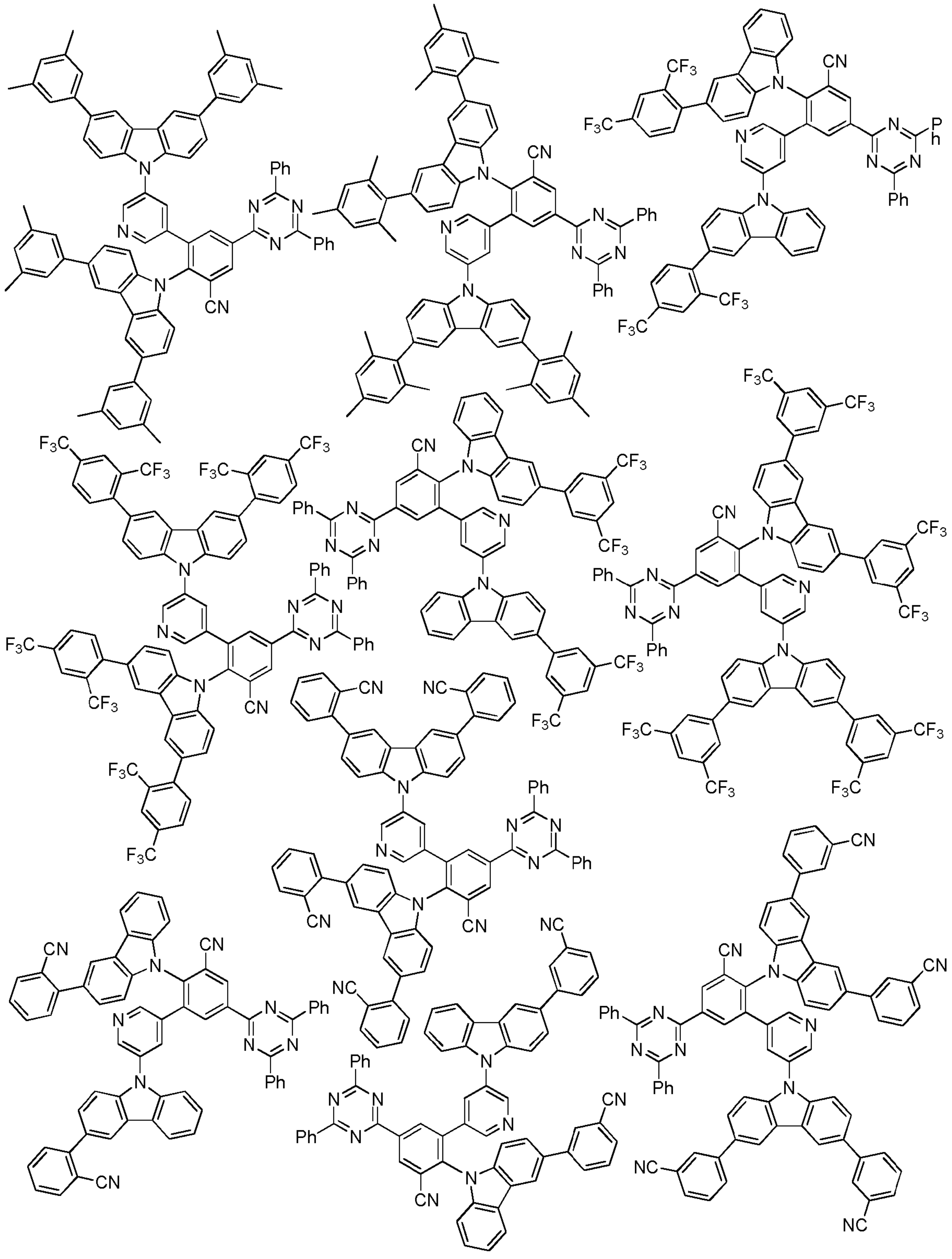

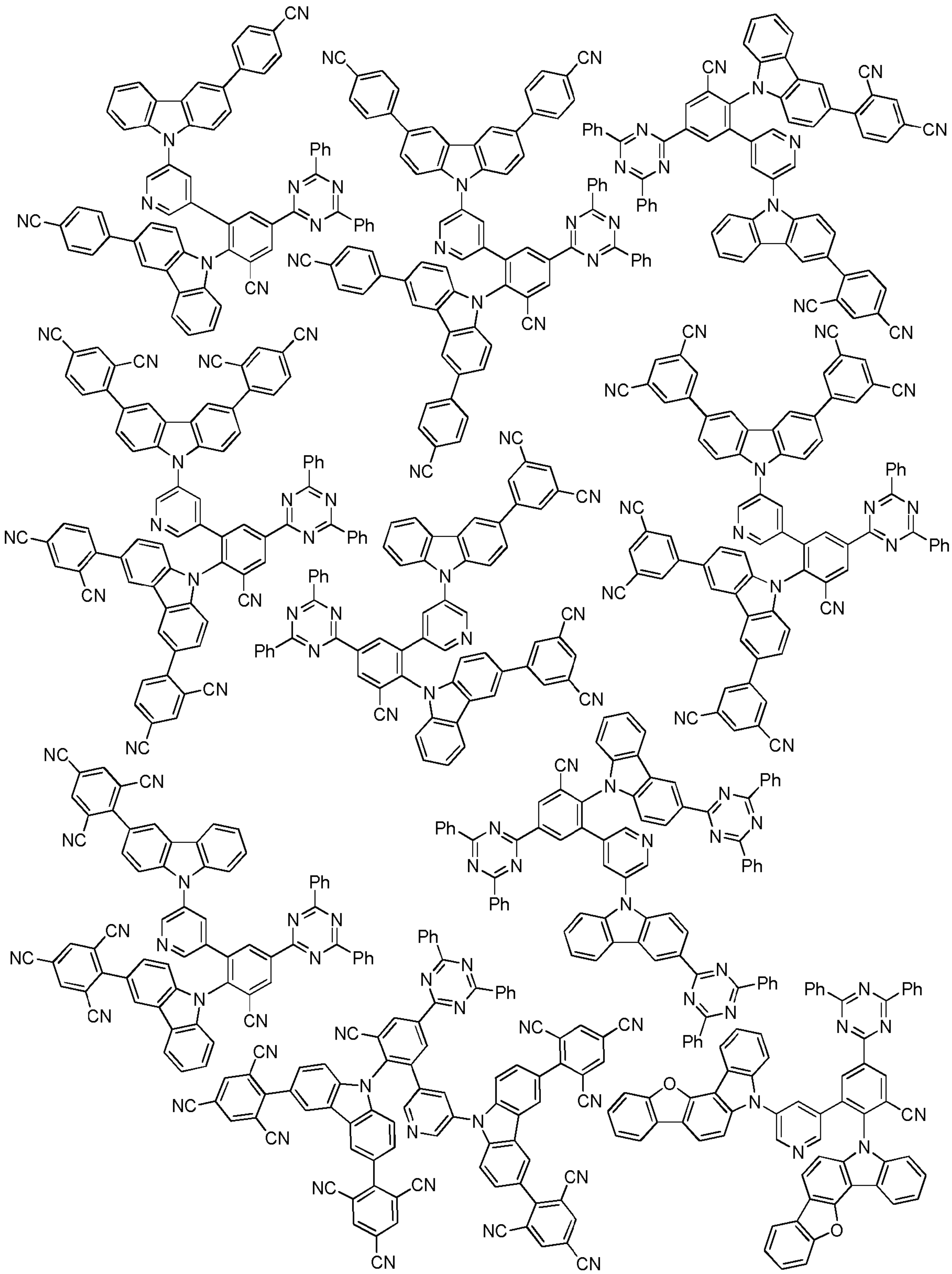

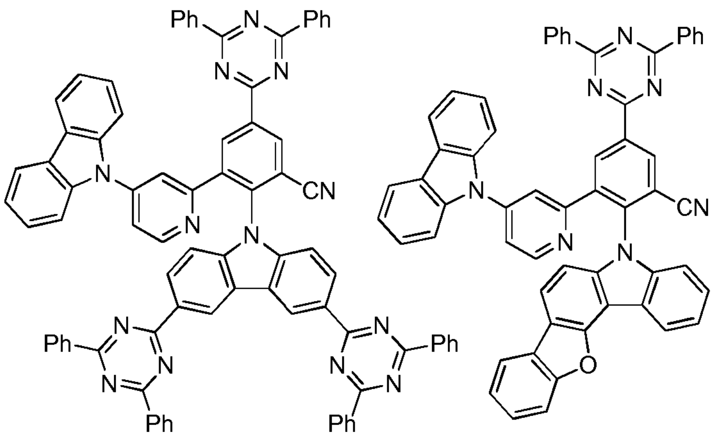

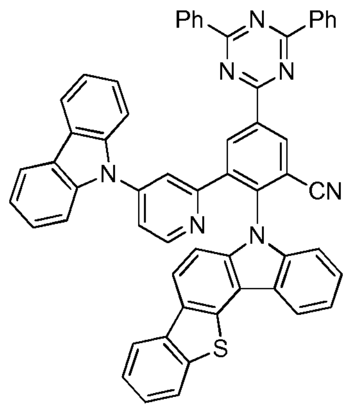

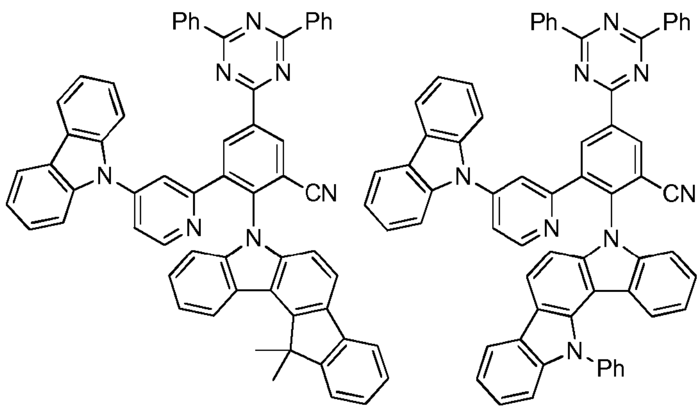

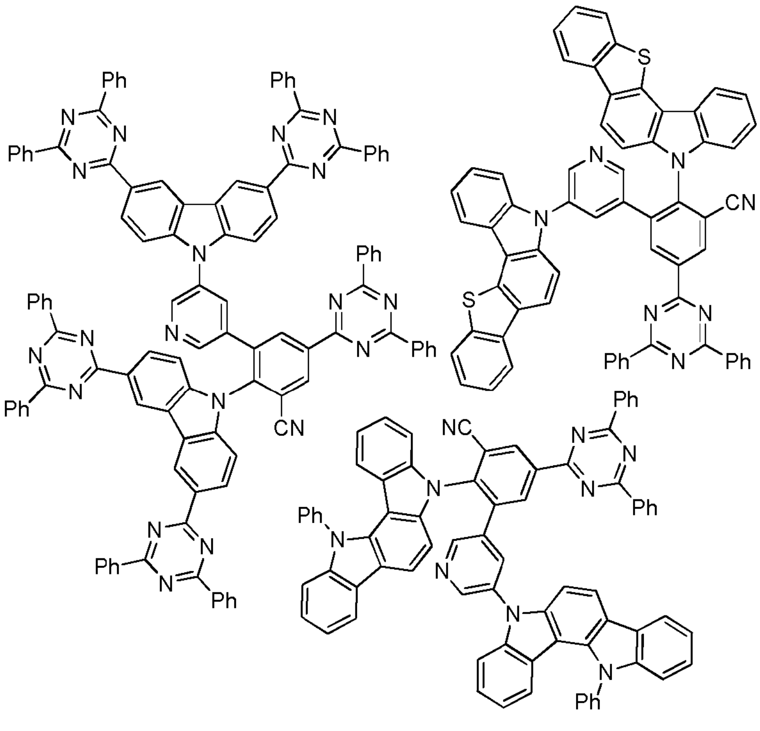

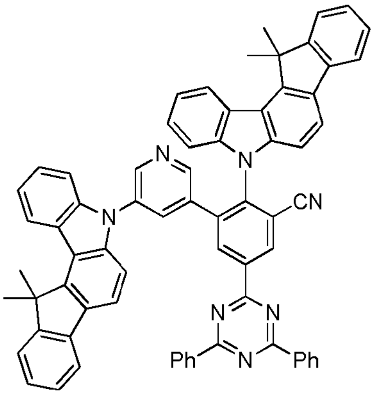

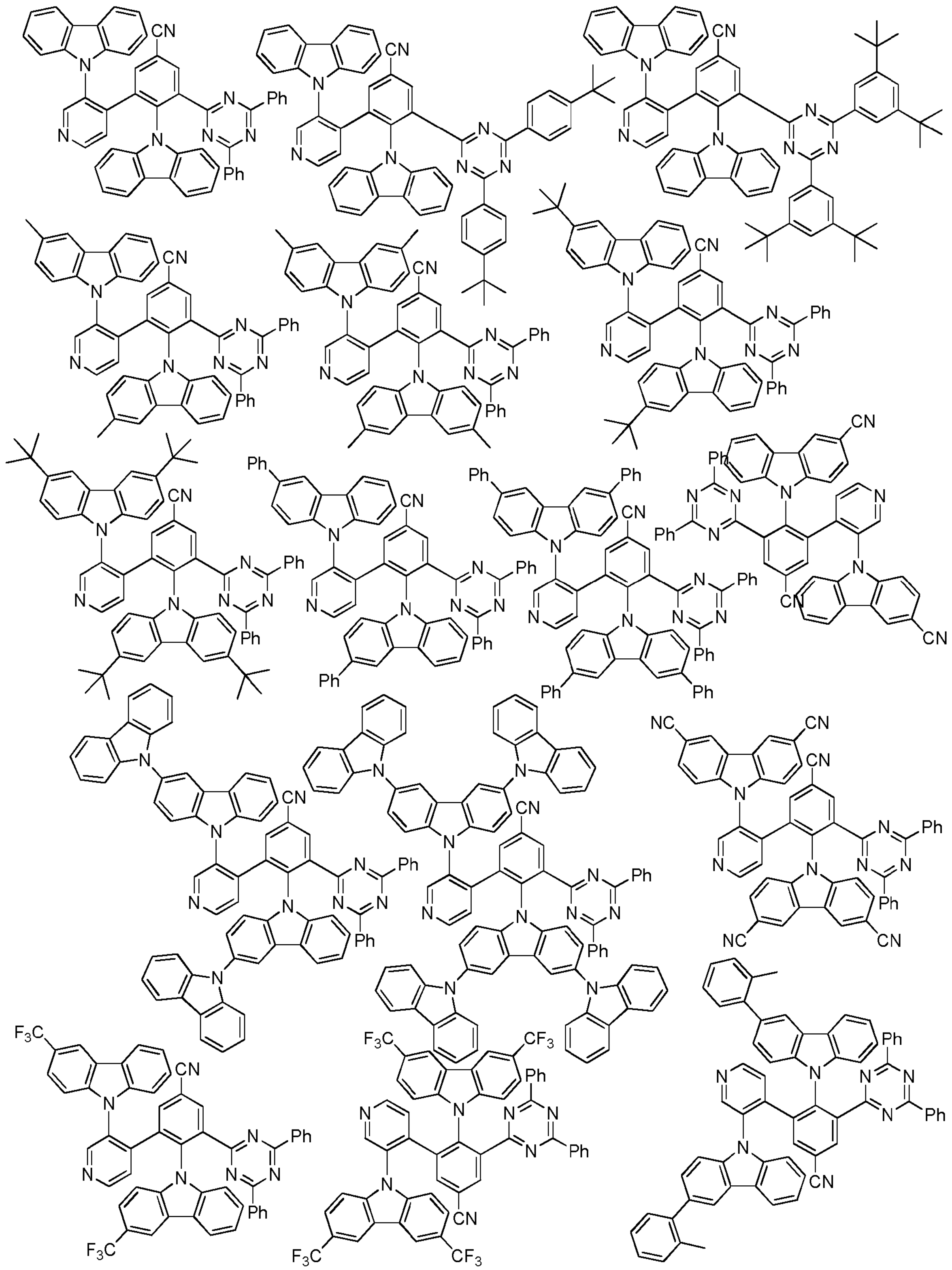

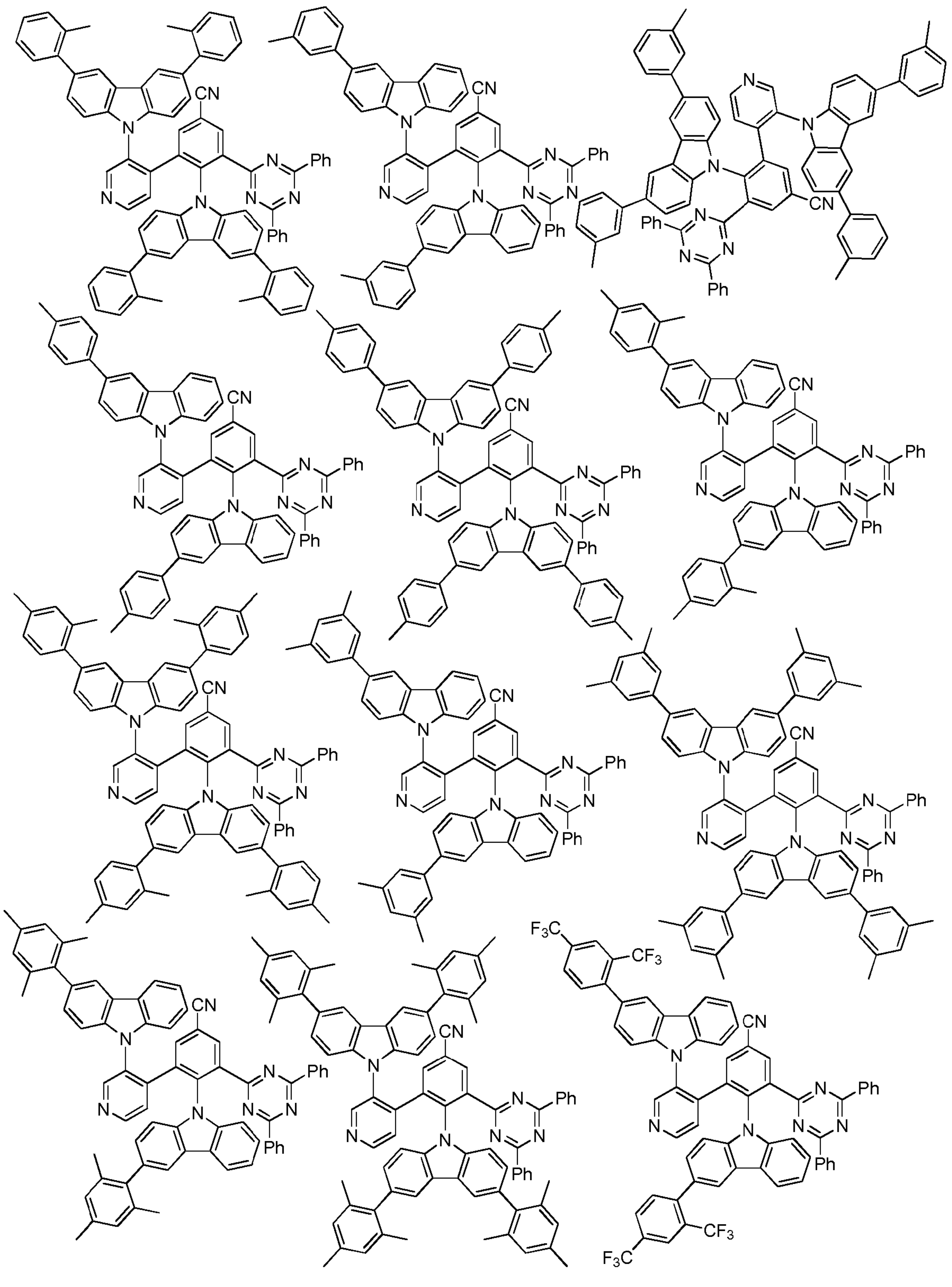

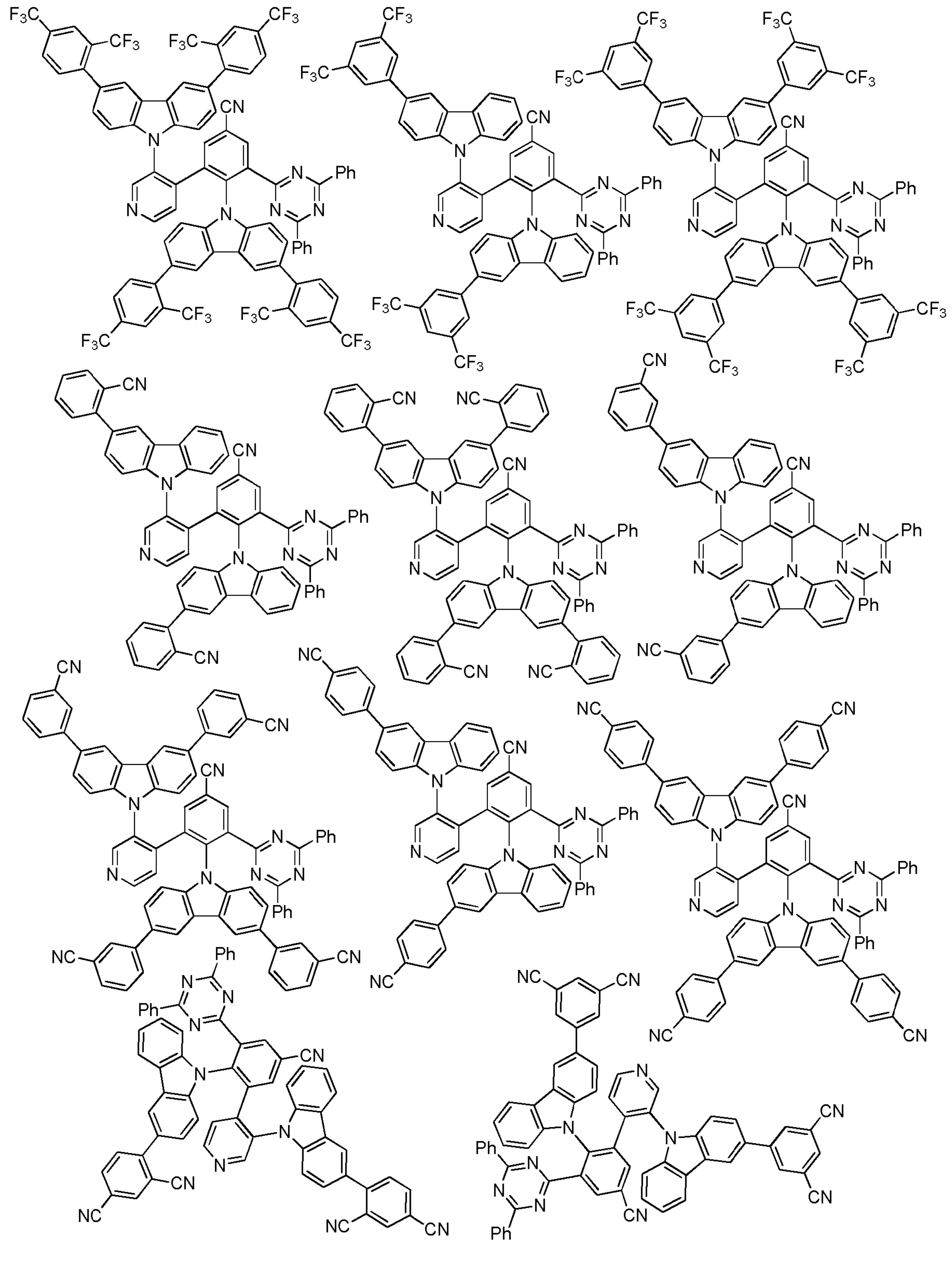

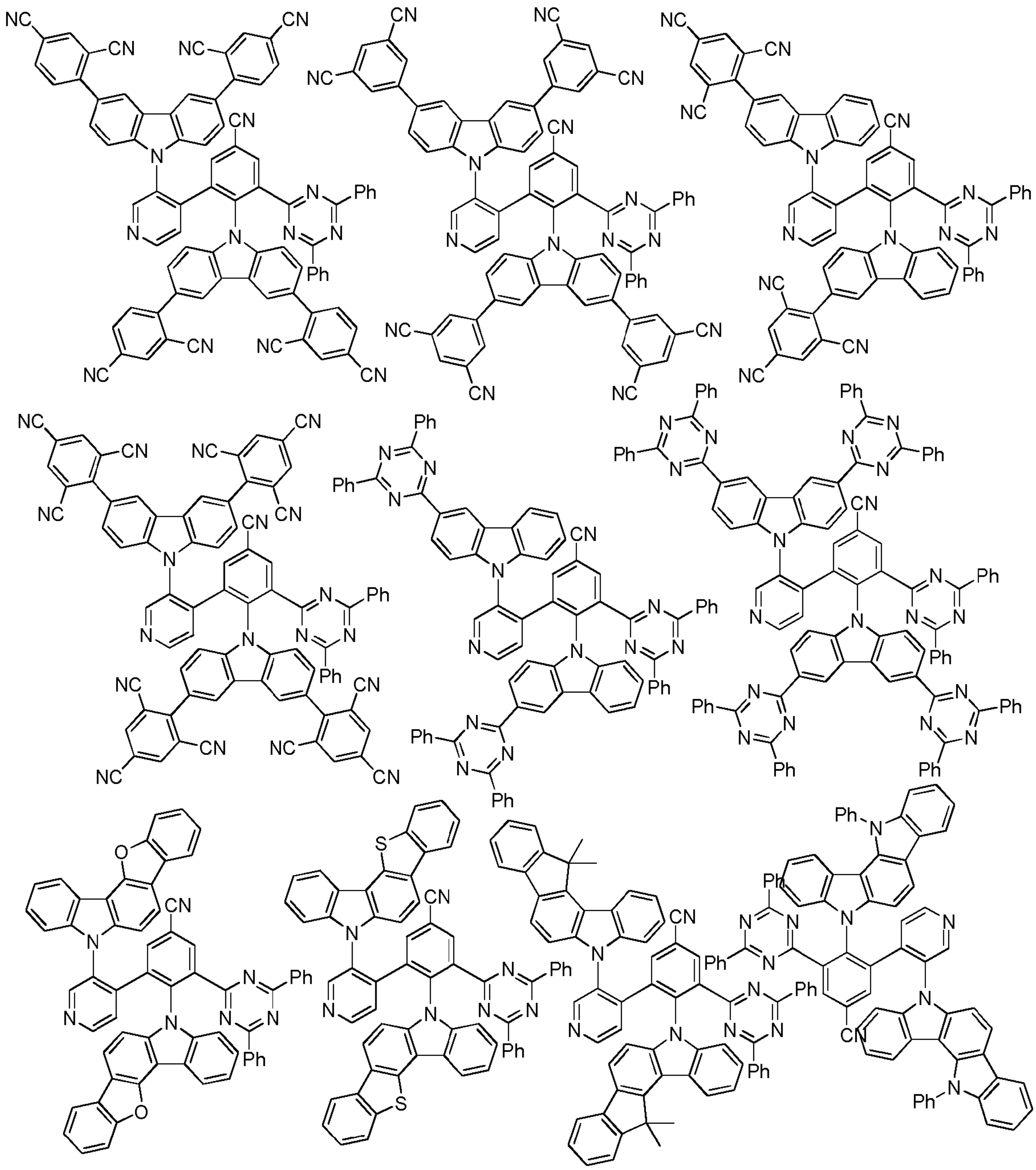

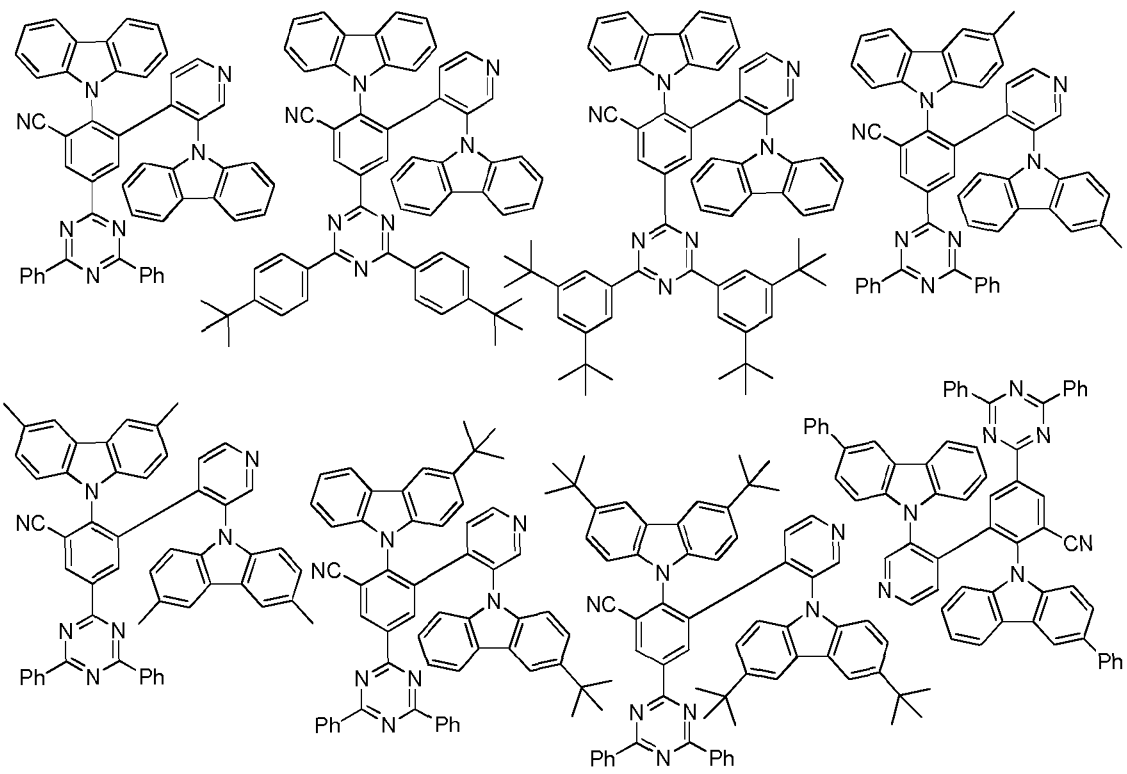

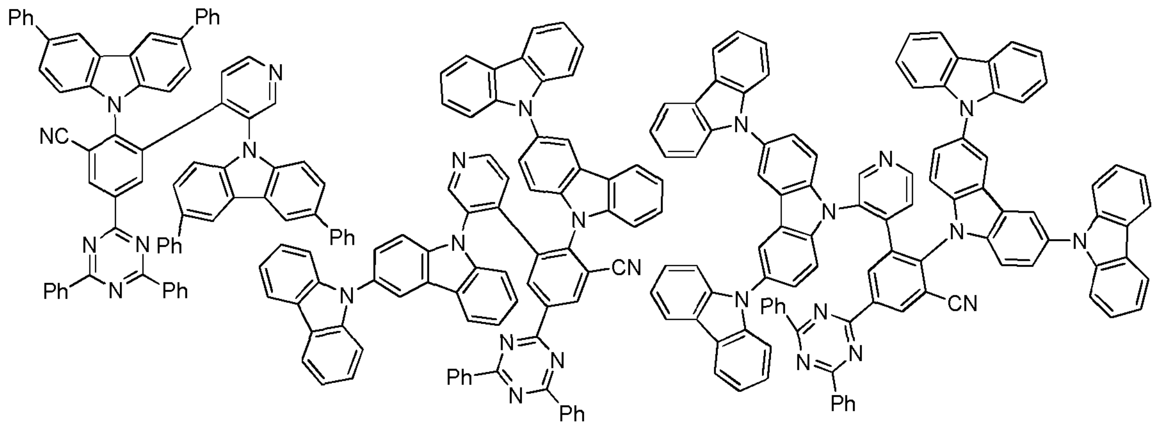

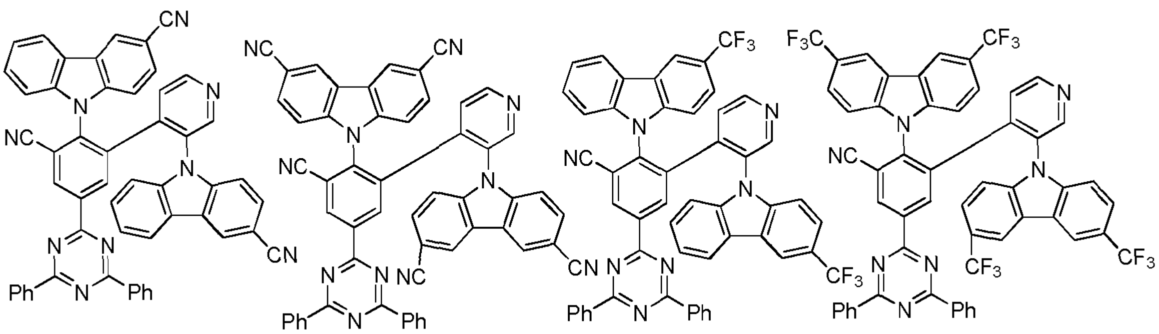

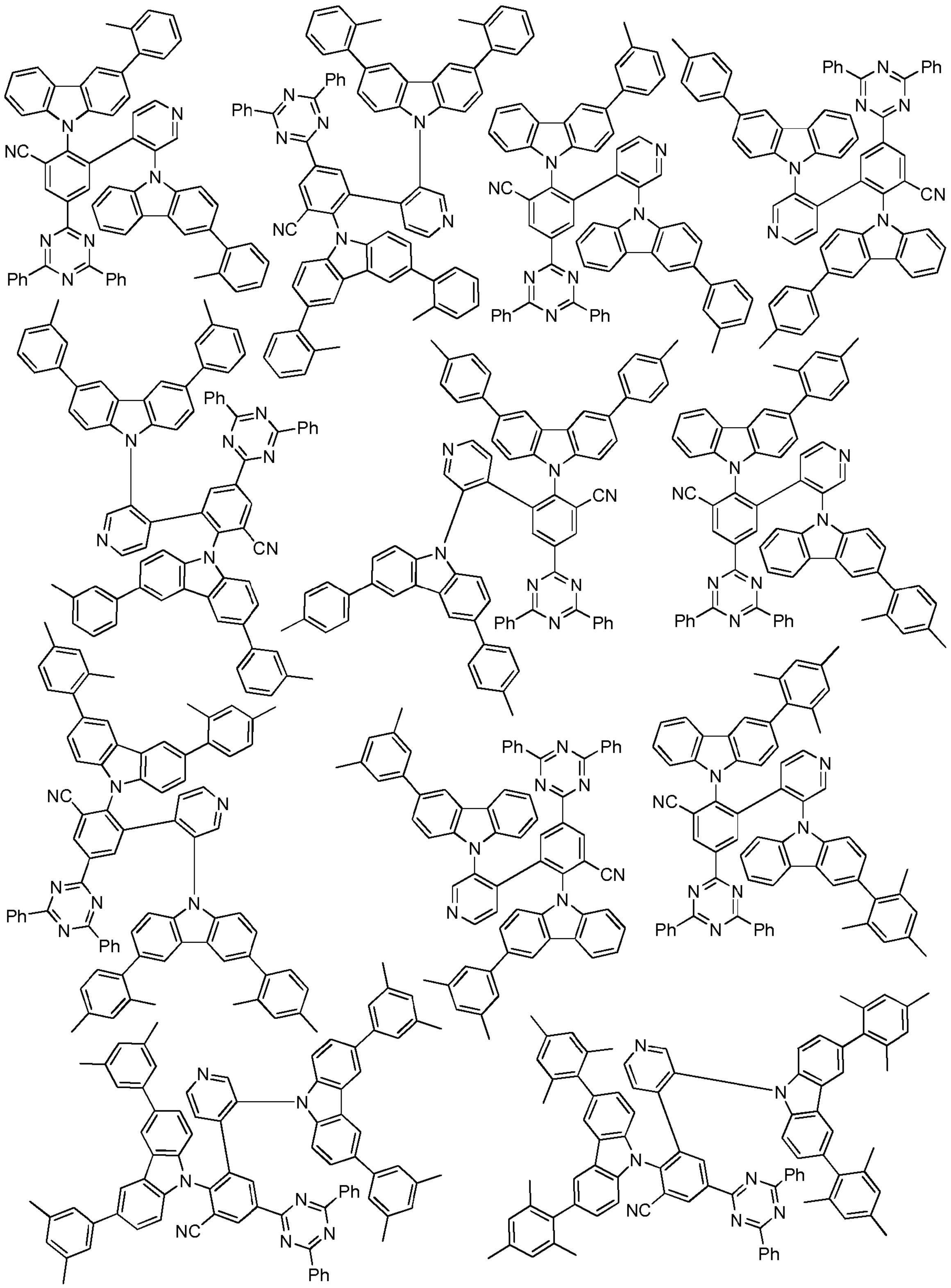

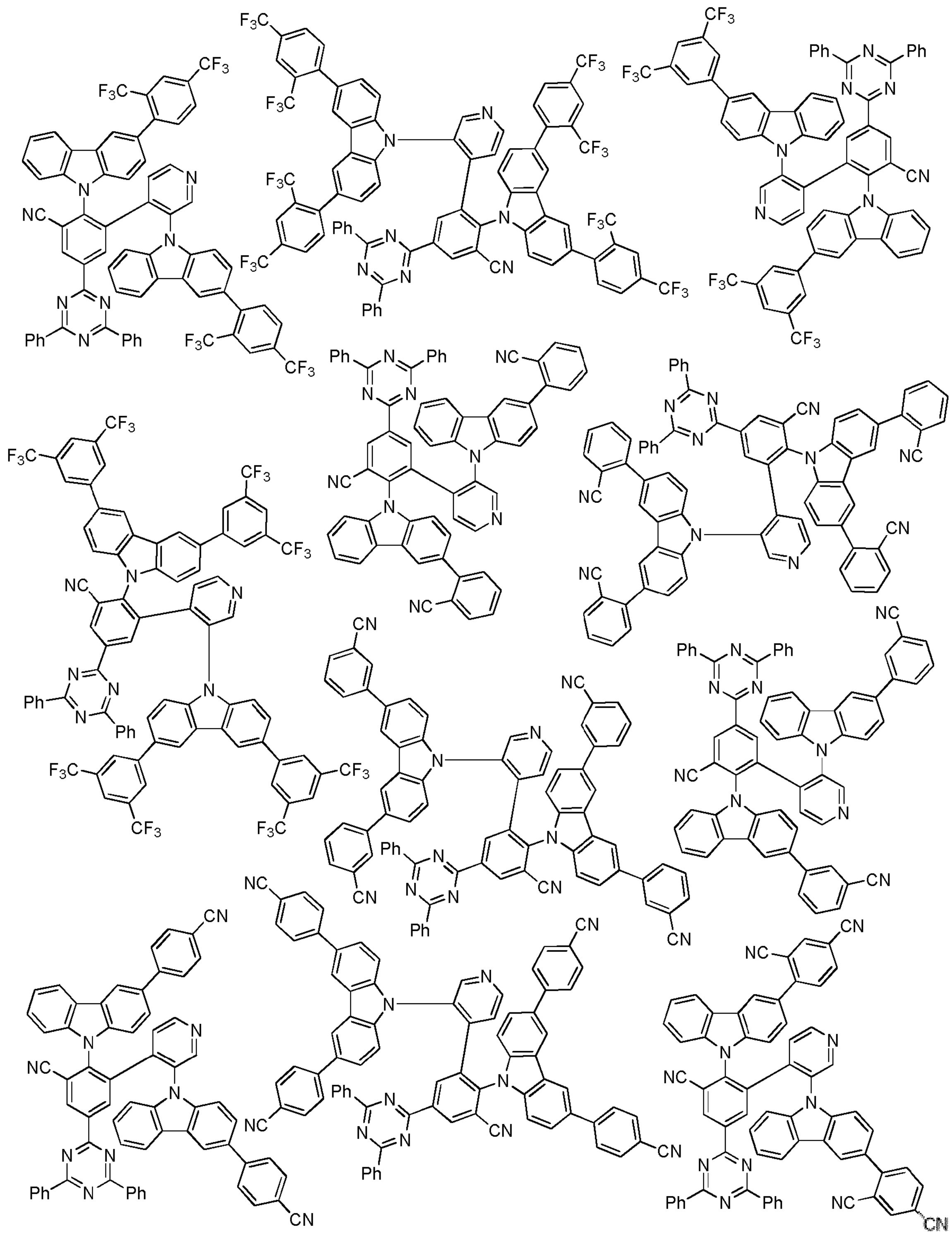

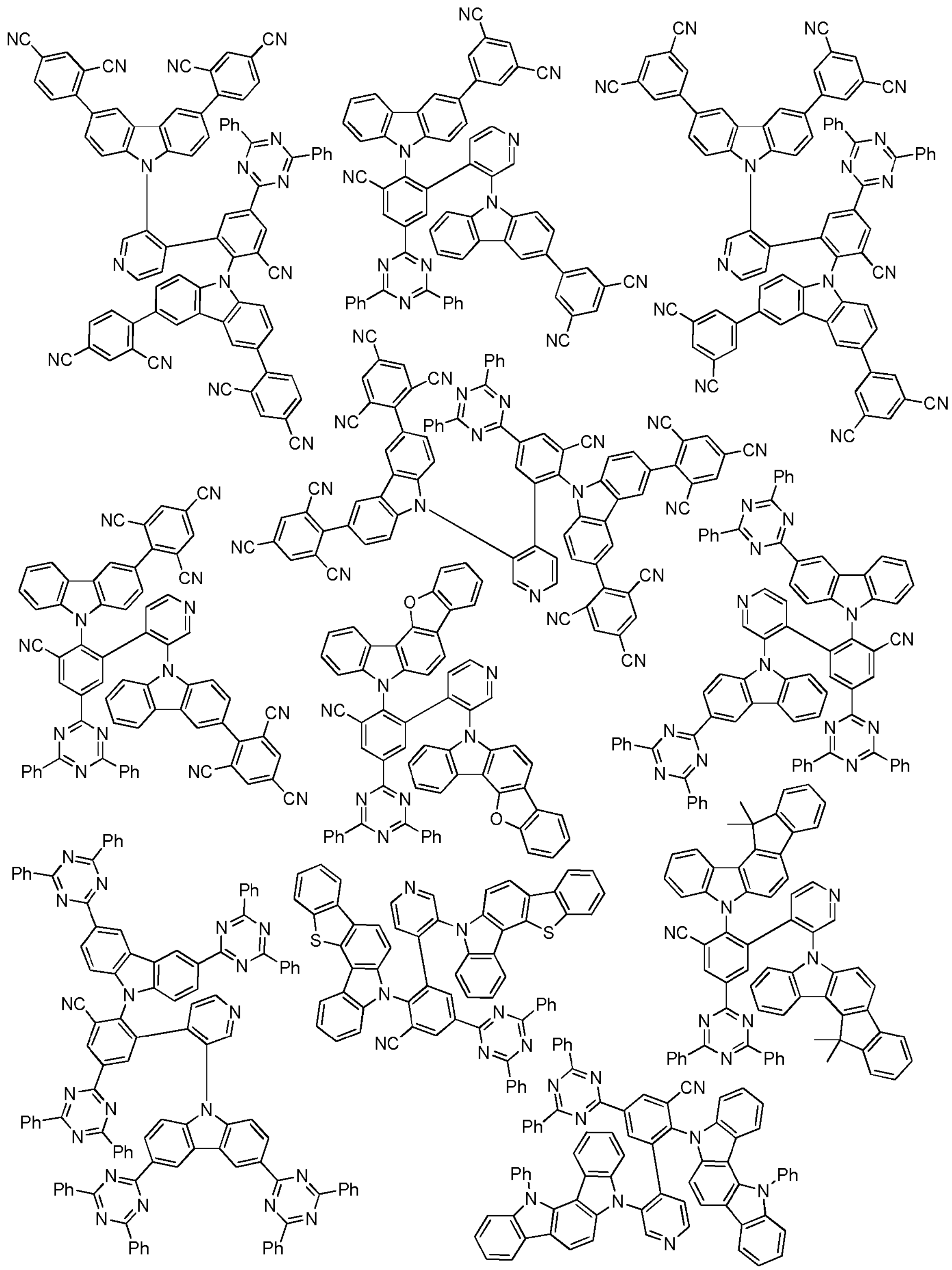

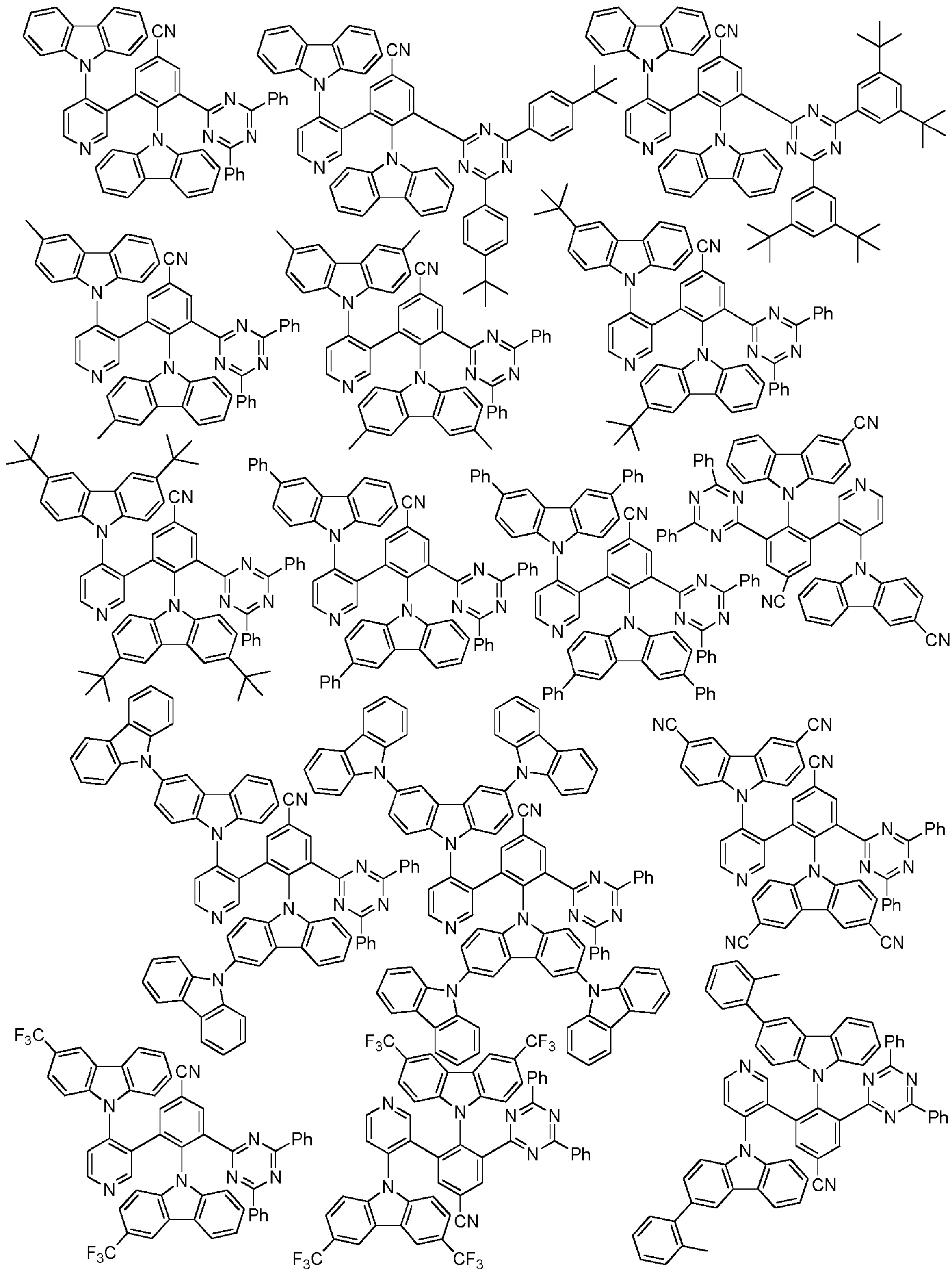

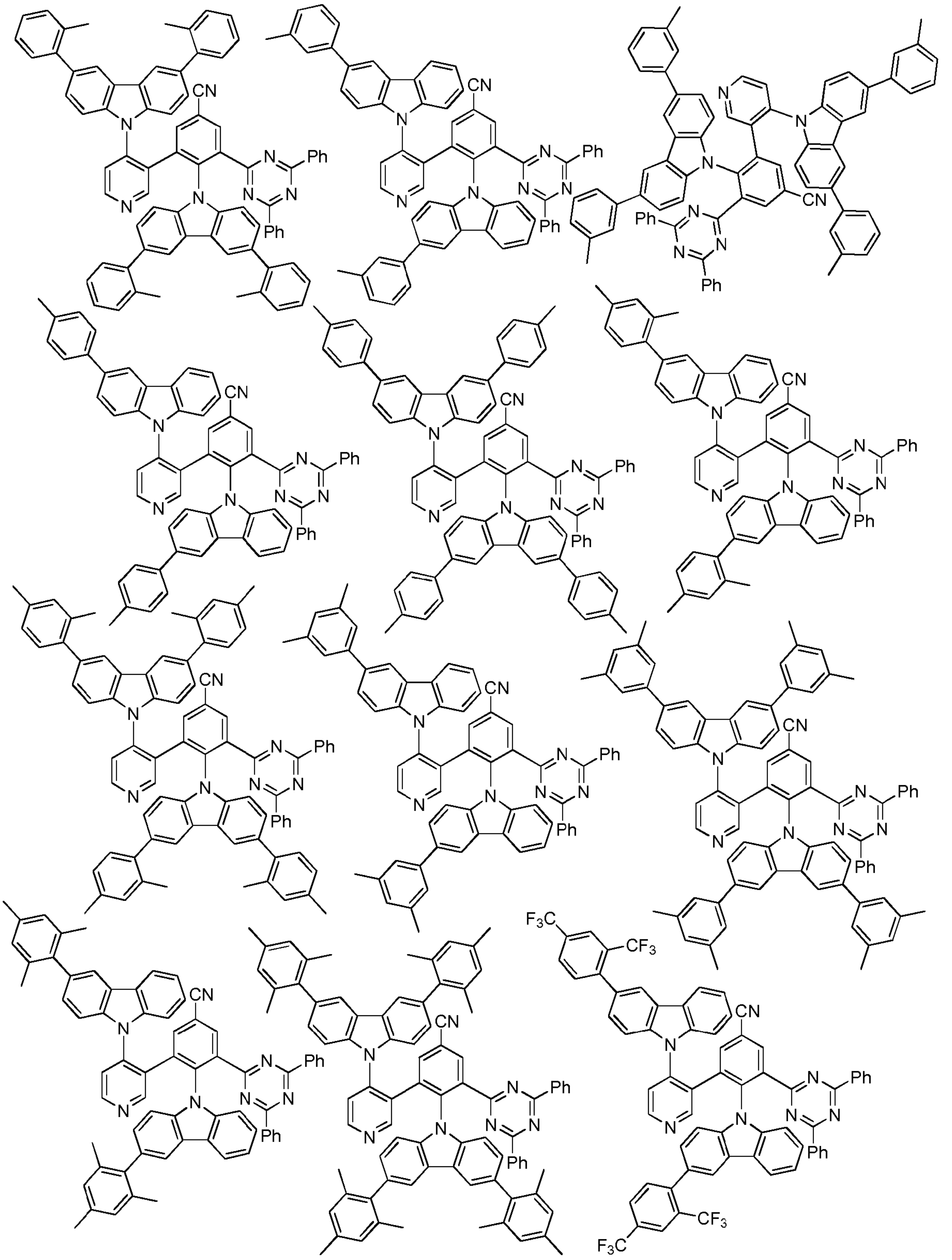

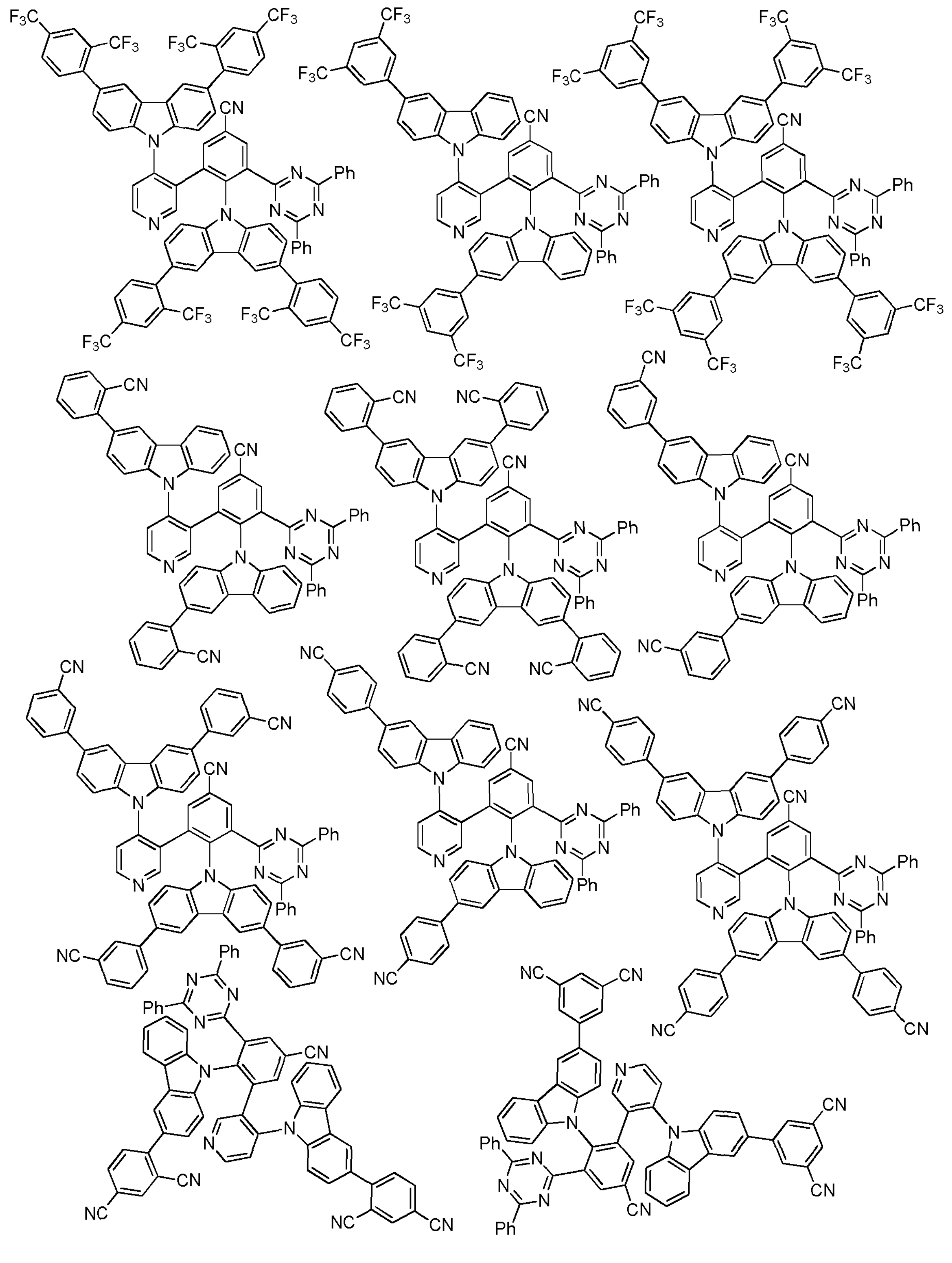

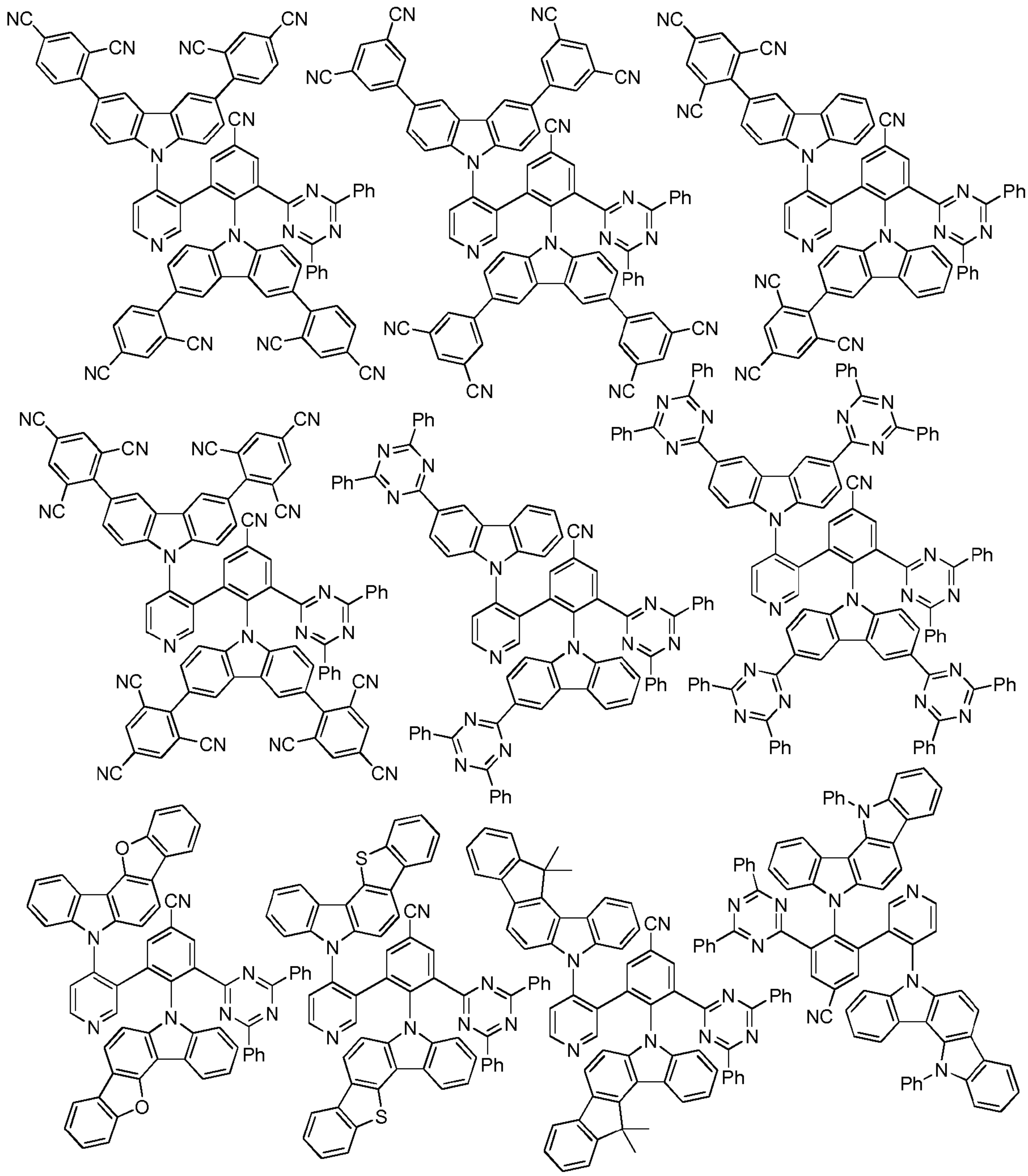

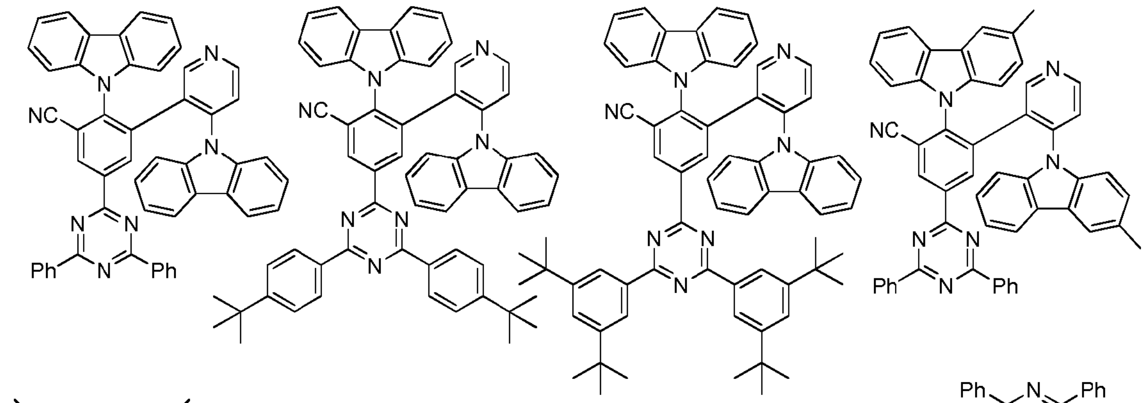

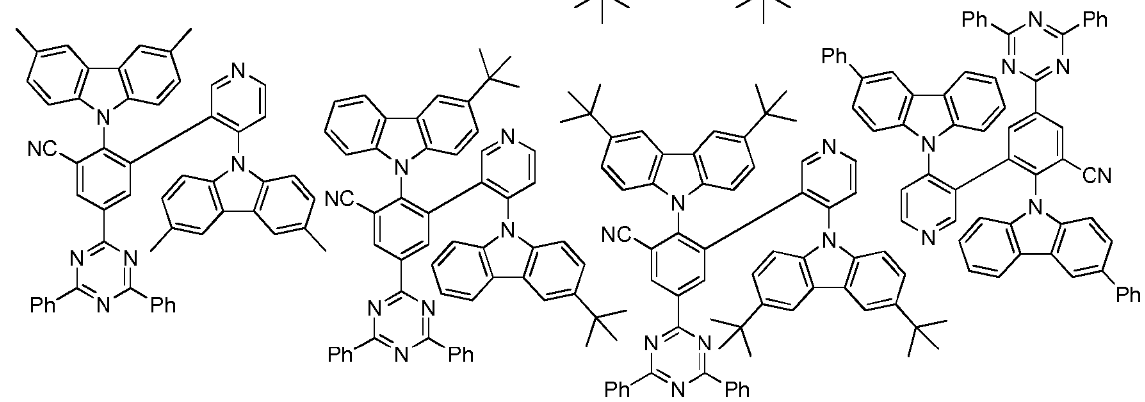

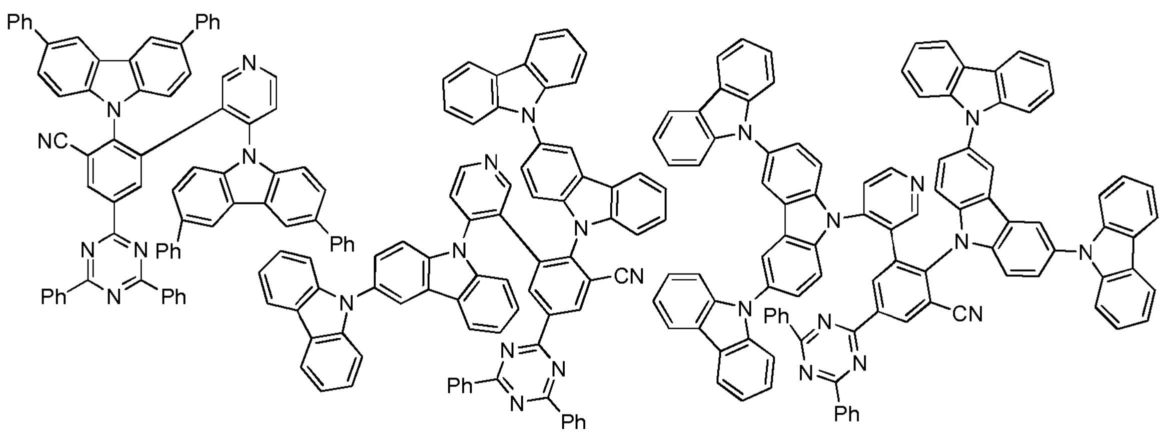

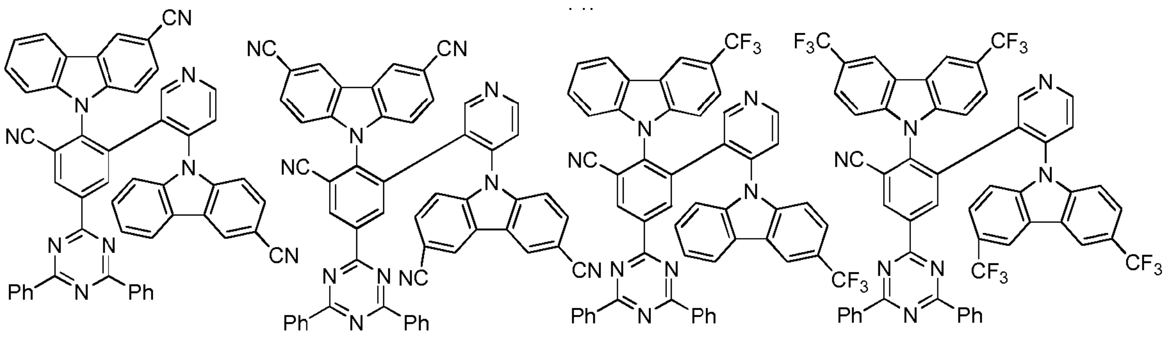

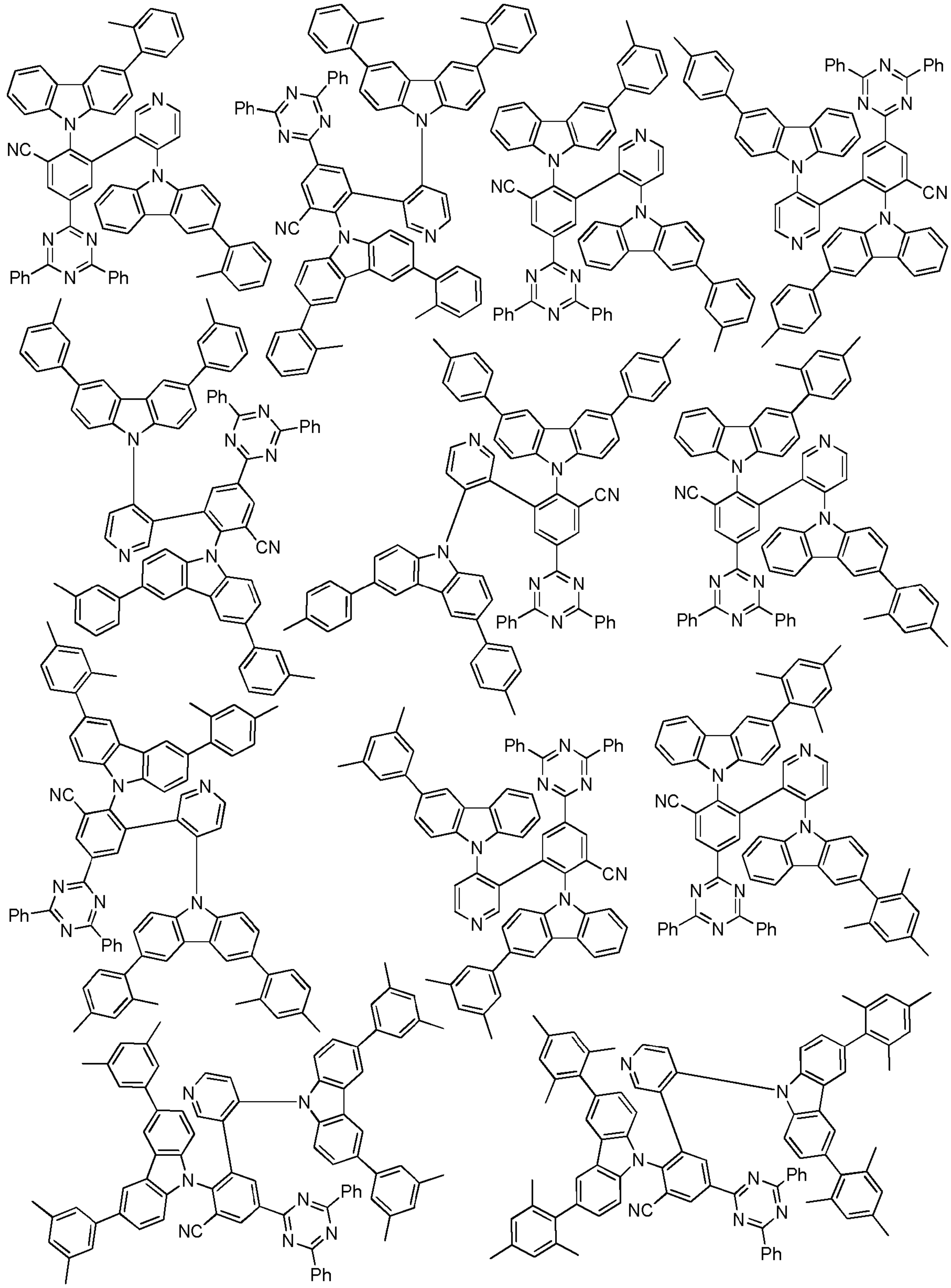

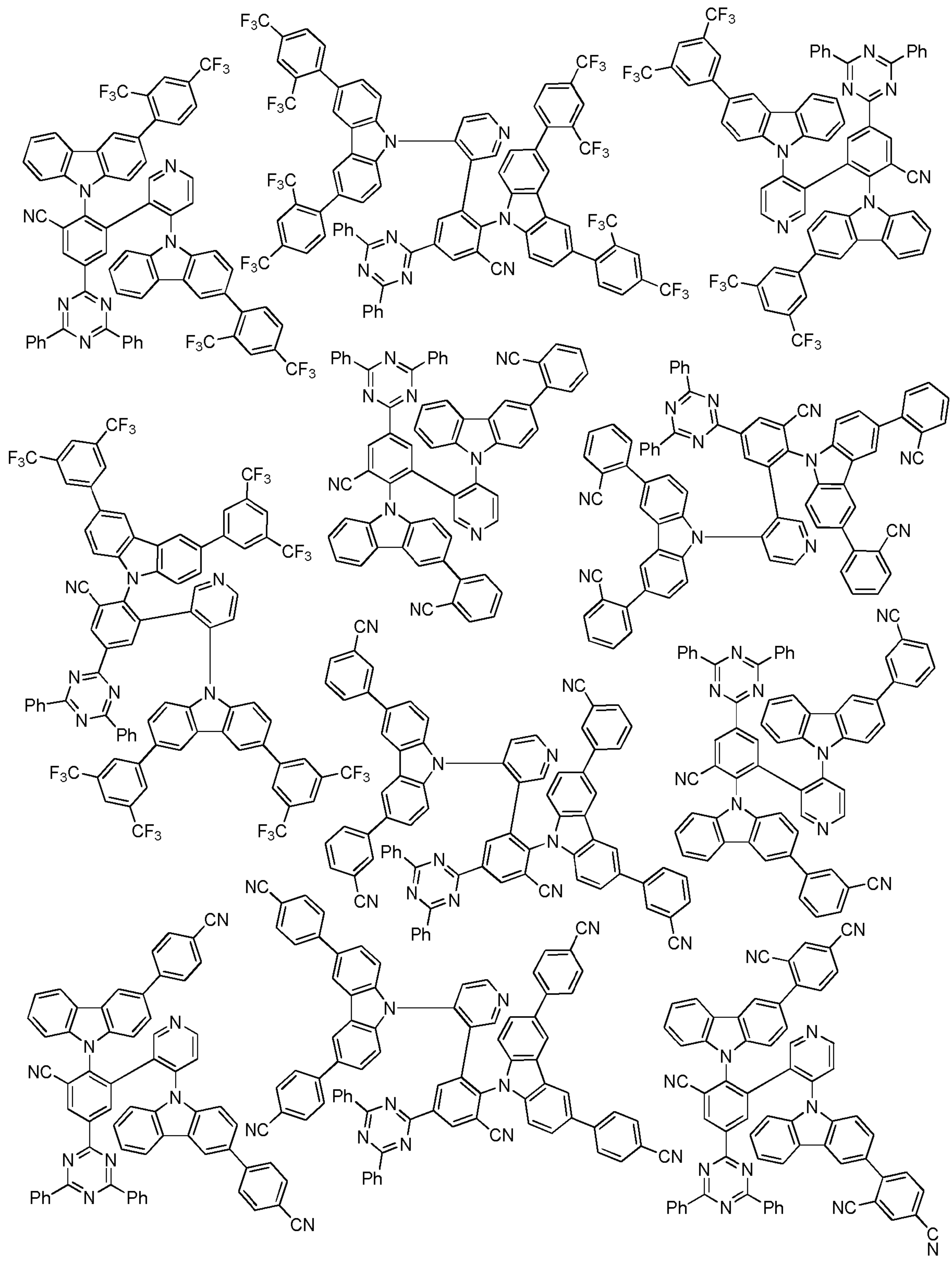

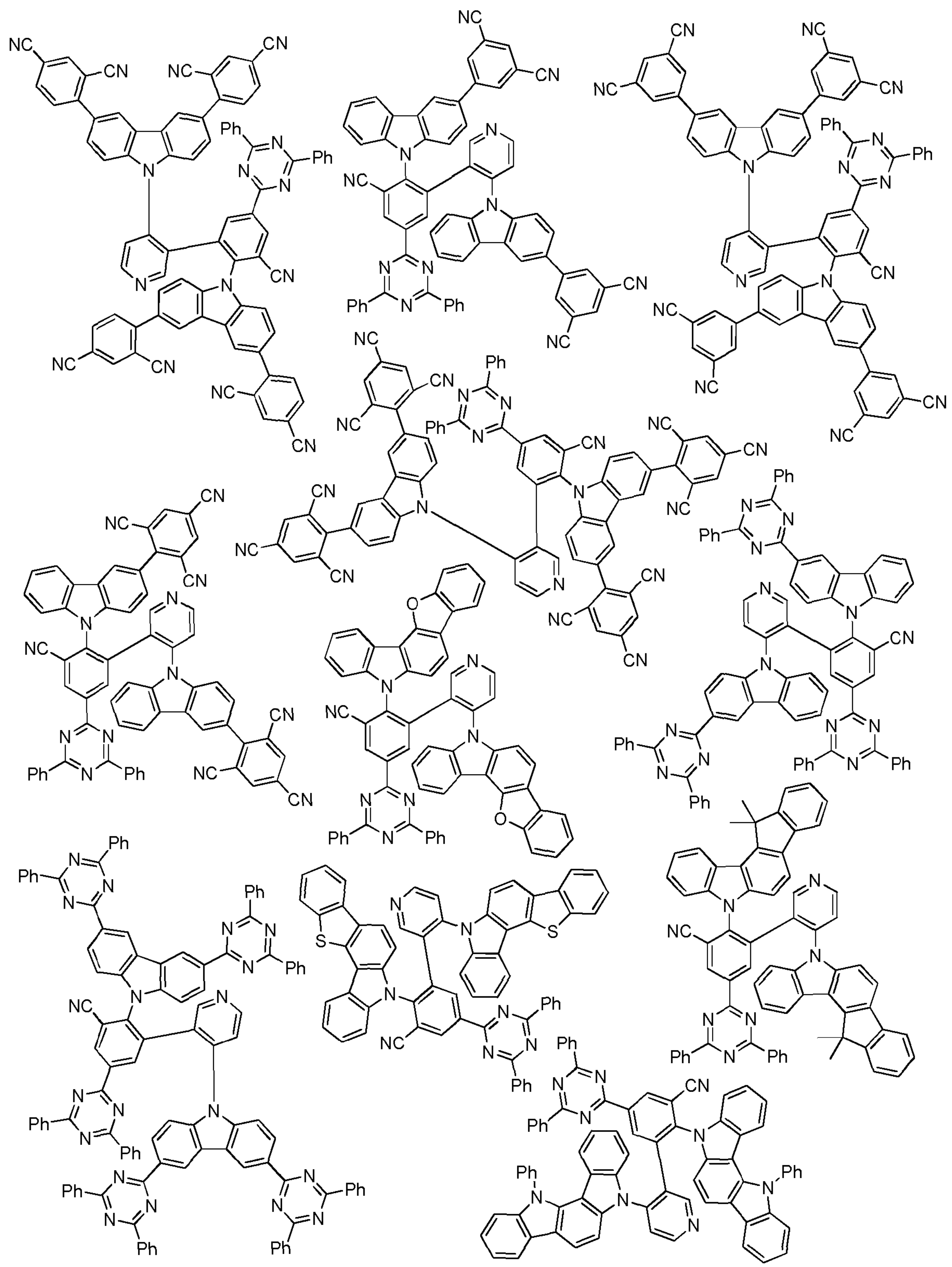

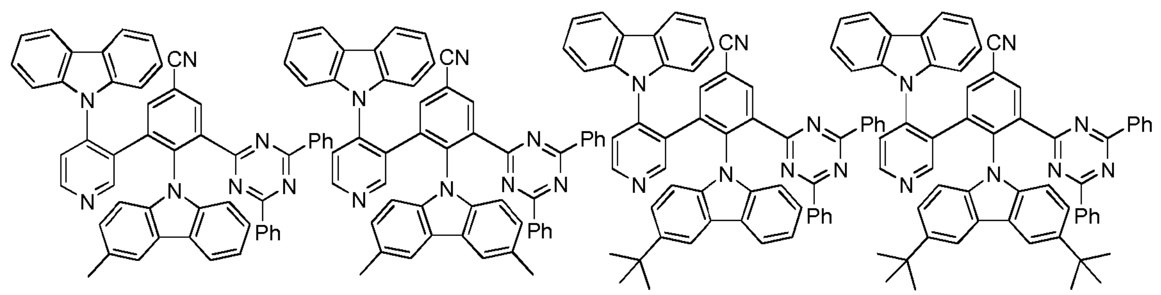

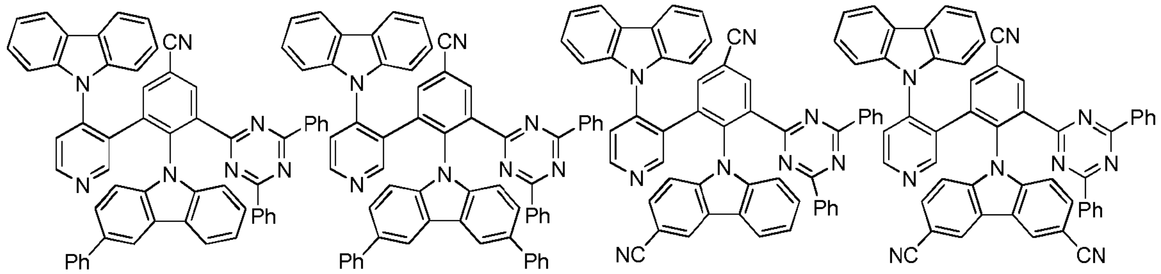

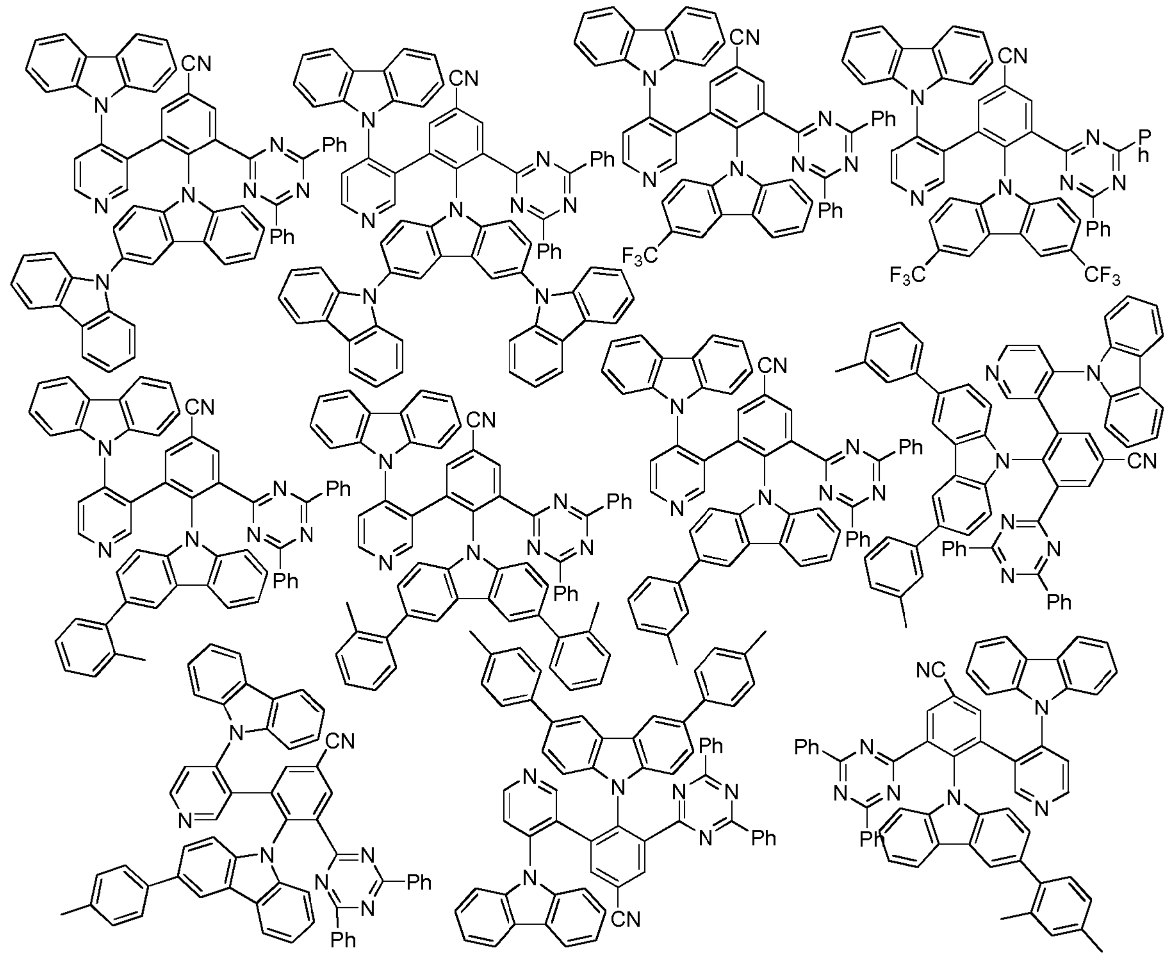

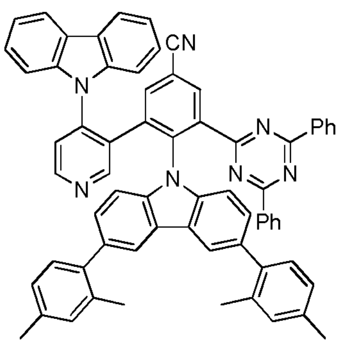

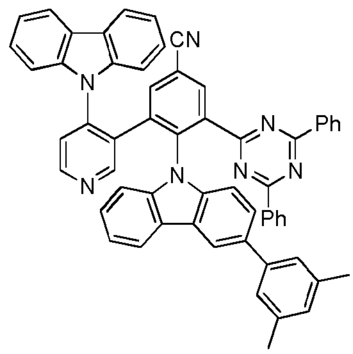

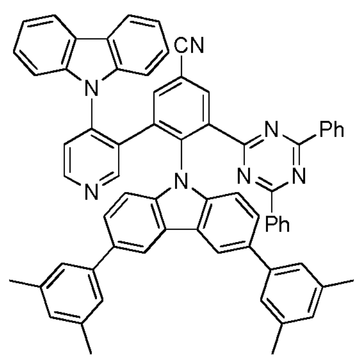

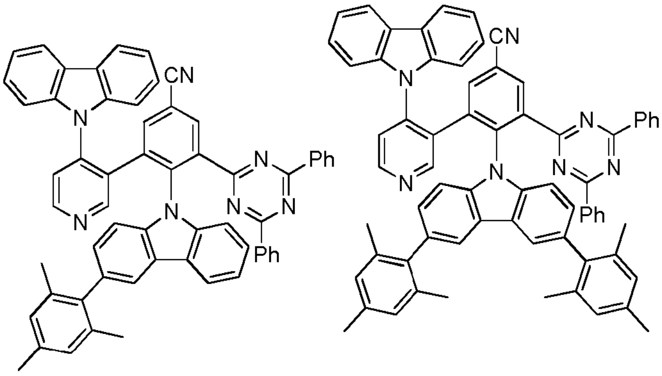

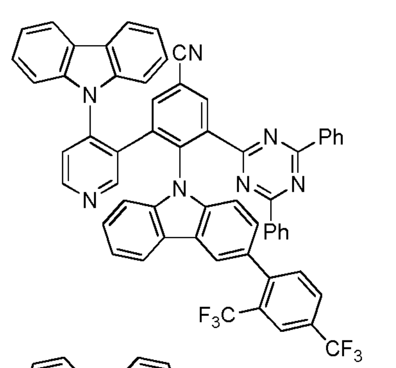

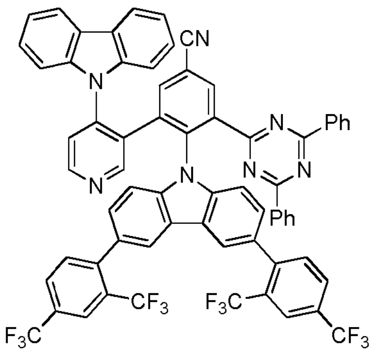

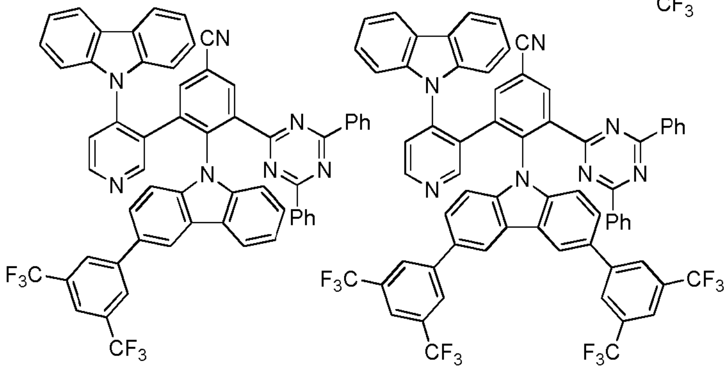

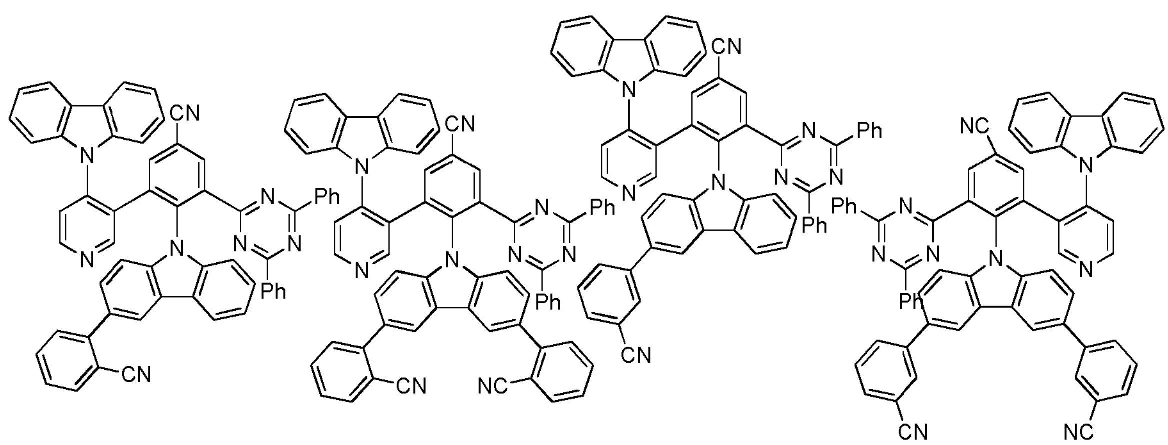

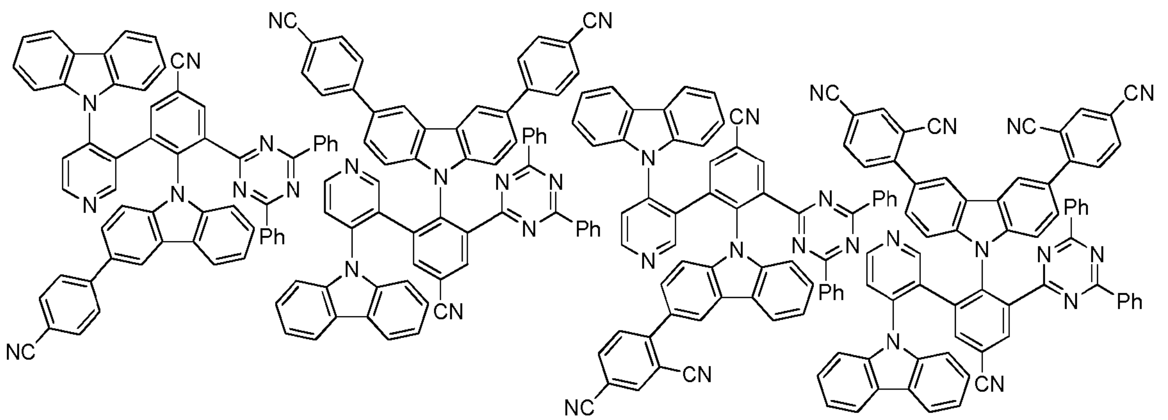

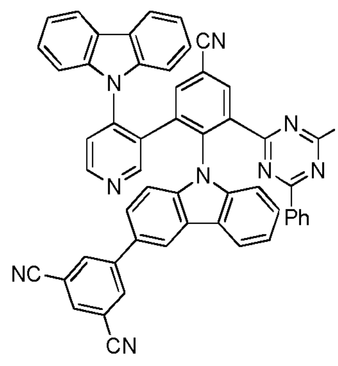

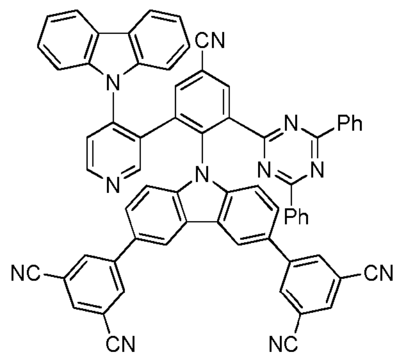

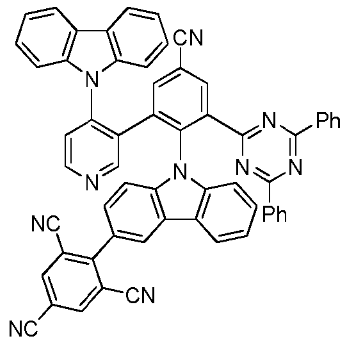

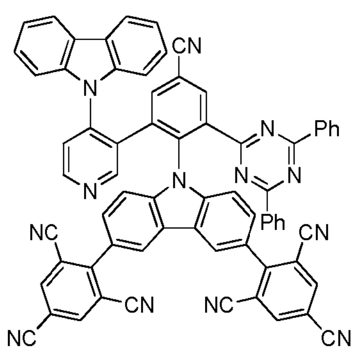

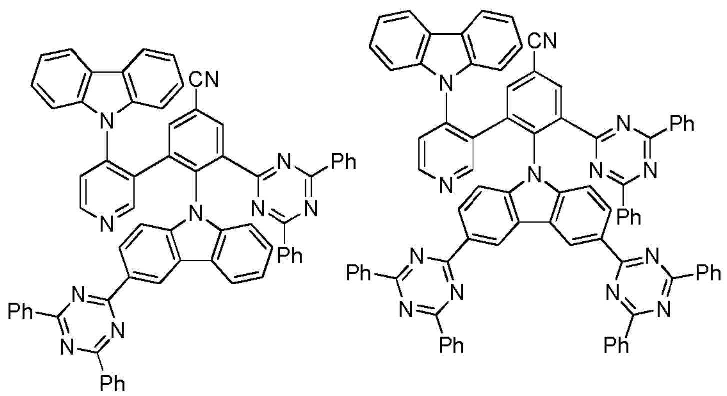

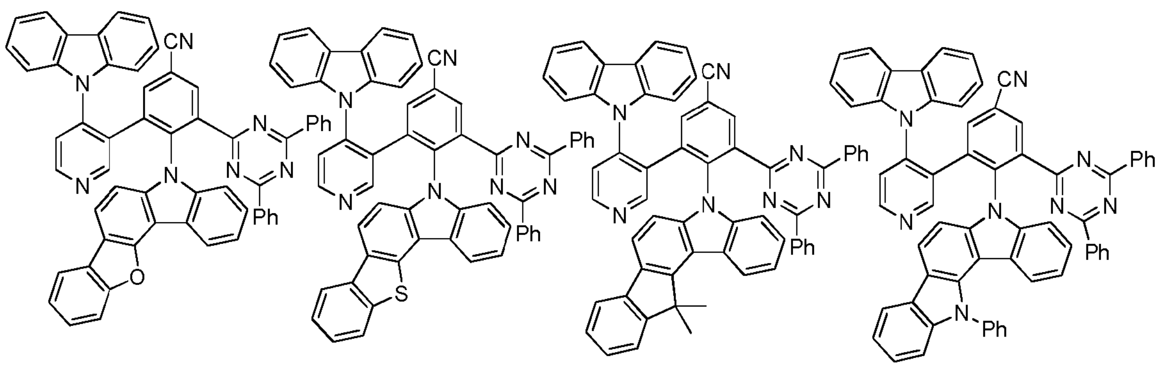

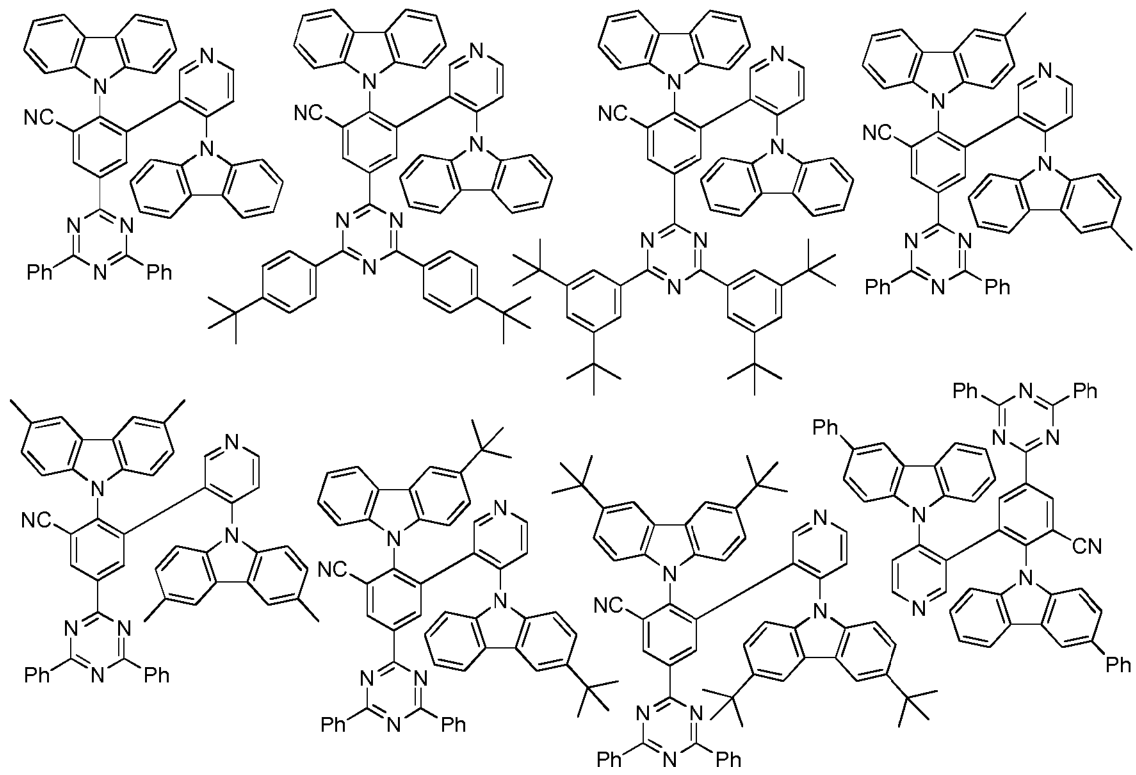

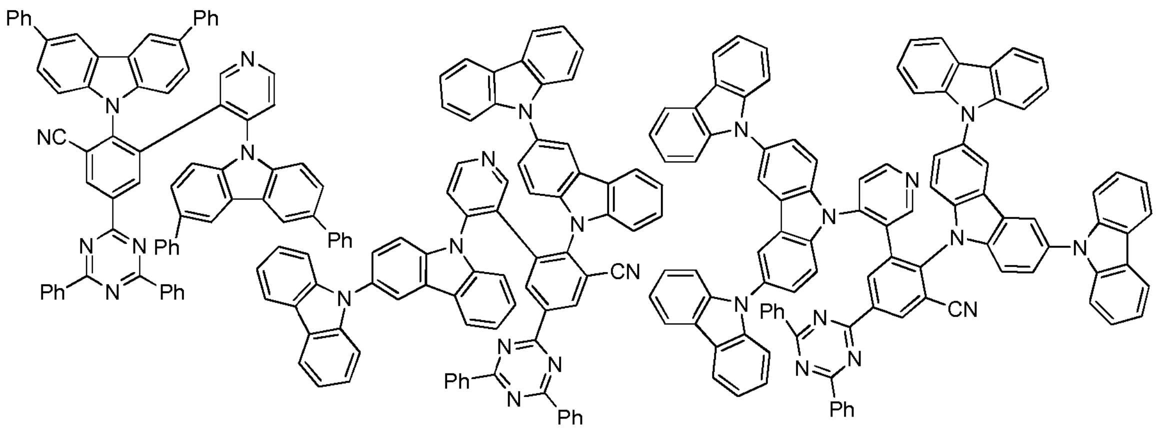

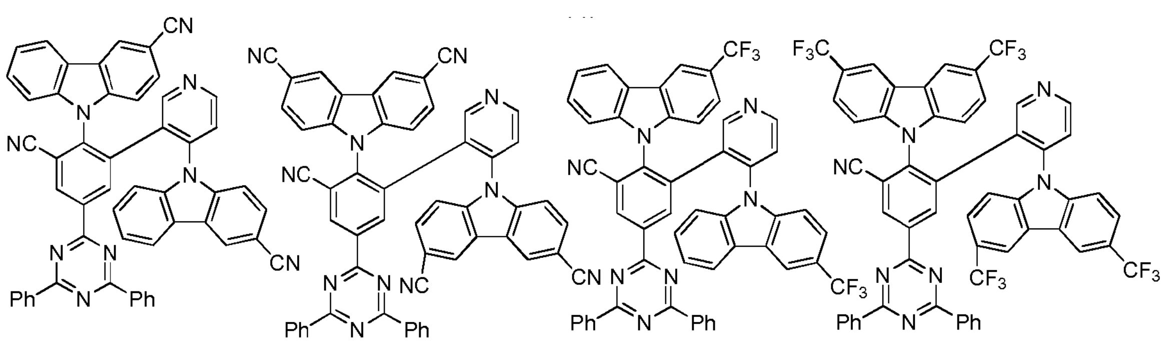

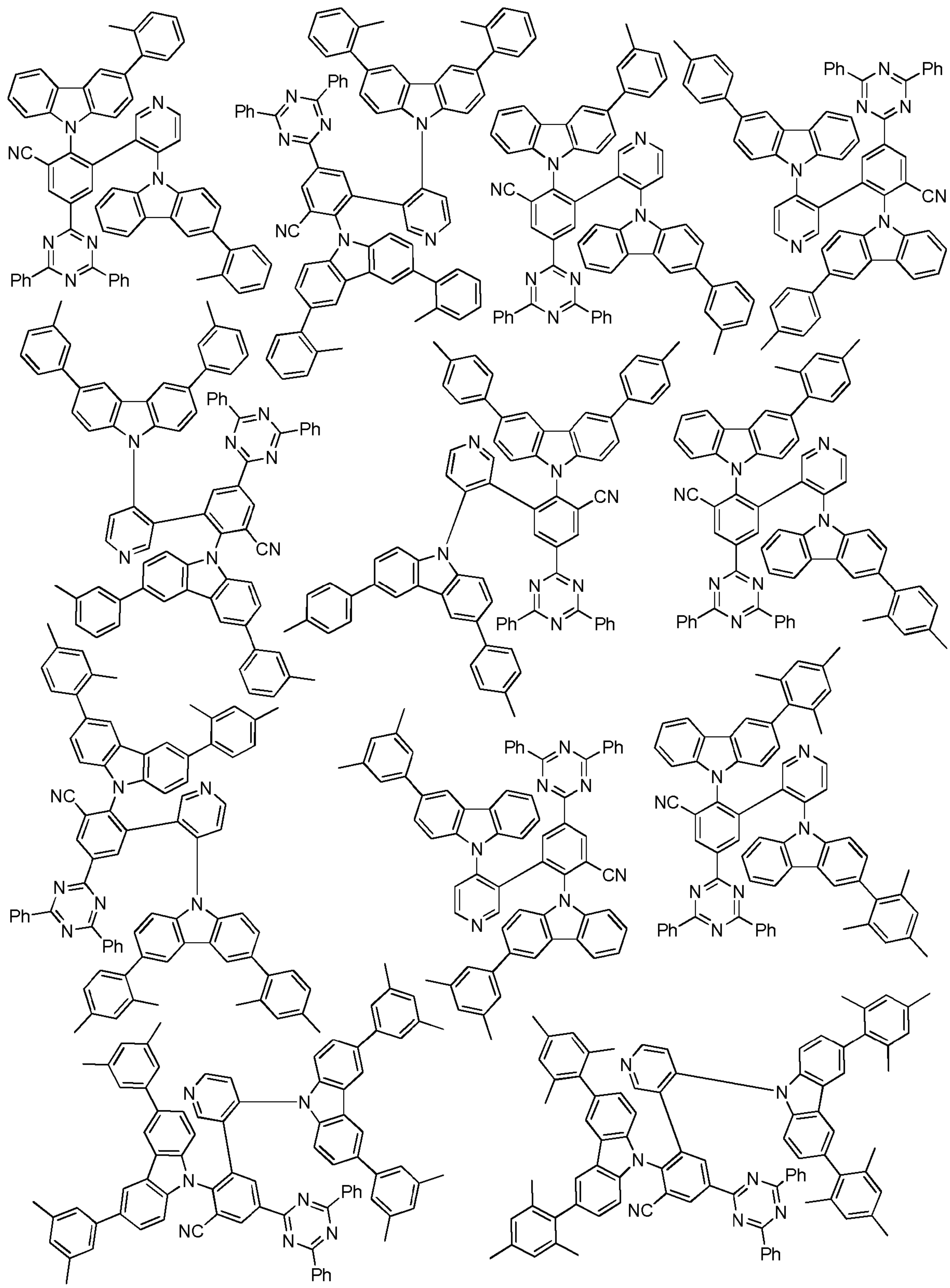

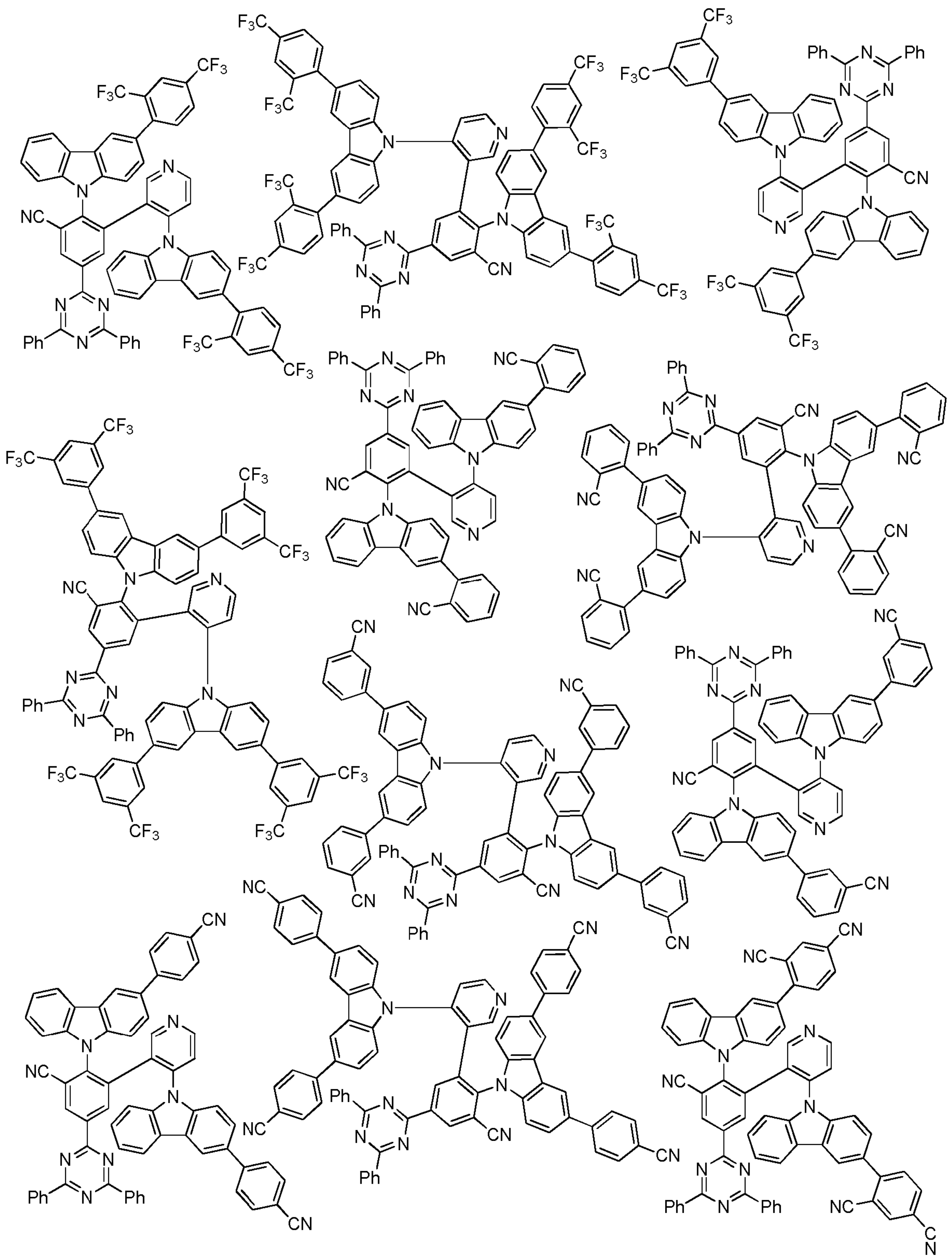

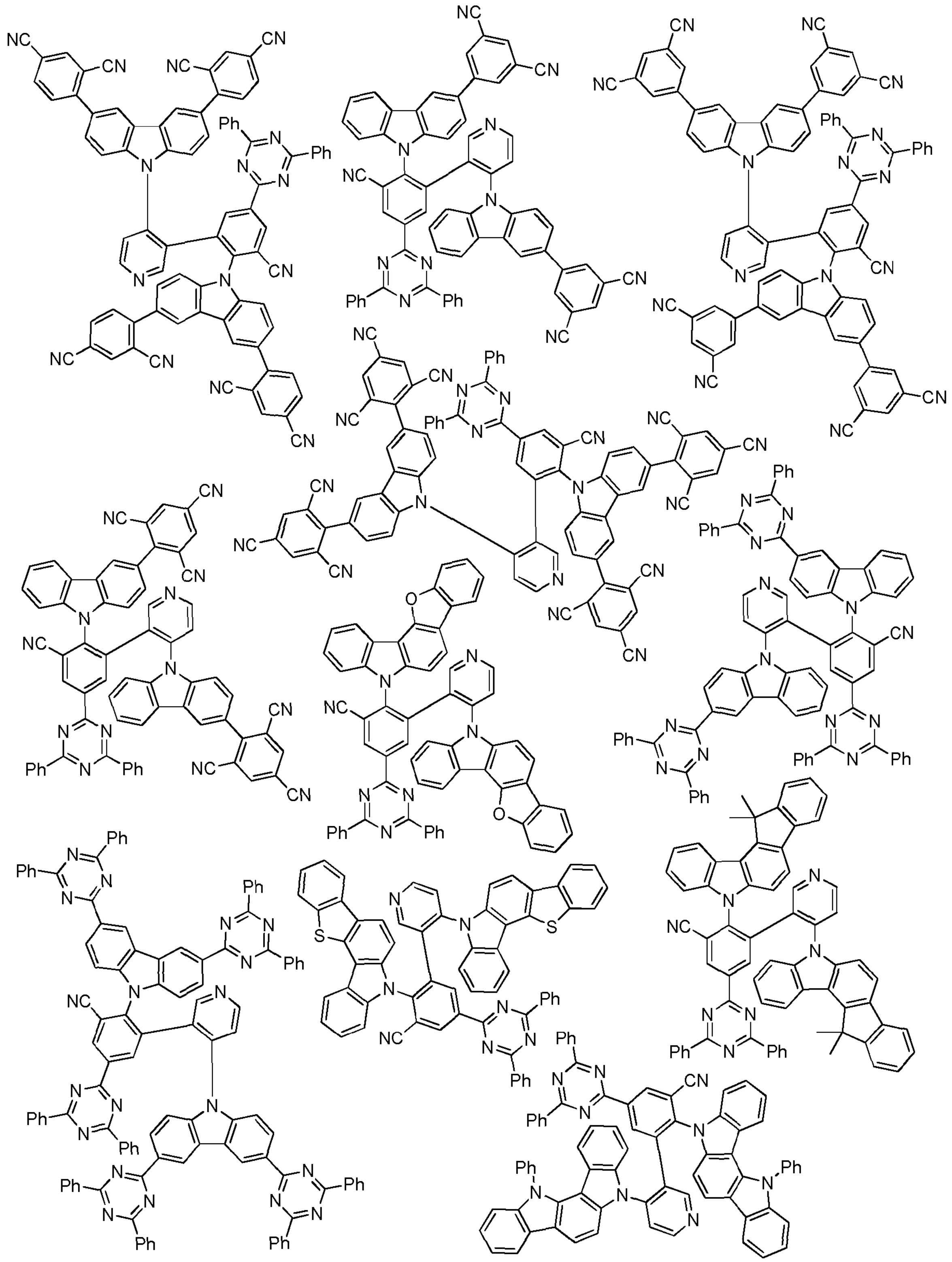

**Figure**

Figure 1 Emission spectrum of example **1** (10% by weight) in PMMA.

## Claims

1. An organic molecule, consisting of

- a first chemical moiety comprising a structure of formula I,

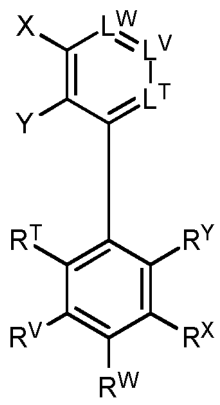

Formula I

and

- two second chemical moieties, each independently comprising a structure of formula II,

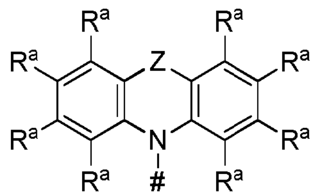

Formula II

wherein the first chemical moiety is linked to each of the two second chemical moieties via a single bond; wherein

$L^T$ is N or C-$R^1$;
$L^V$ is N or C-$R^1$;
$L^W$ is N or C-W;
X is the binding site of a single bond linking the first chemical moiety to one of the two second chemical moieties or is $R^2$;
Y is the binding site of a single bond linking the first chemical moiety to one of the two second chemical moieties or is $R^2$;
W is the binding site of a single bond linking the first chemical moiety to one of the two second chemical moieties or is $R^2$;
$R^T$ is selected from the group consisting of $R^I$, CN and 1,3,5-triazinyl, which is substituted with two substituents $R^{Tz}$;
$R^V$ is selected from the group consisting of $R^I$, CN and 1,3,5-triazinyl, which is substituted with two substituents $R^{Tz}$;
$R^W$ is selected from the group consisting of $R^I$, CN and 1,3,5-triazinyl, which is substituted with two substituents $R^{Tz}$;
$R^X$ is selected from the group consisting of $R^I$, CN and 1,3,5-triazinyl, which is substituted with two substituents $R^{Tz}$;
$R^Y$ is the binding site of a single bond linking the first chemical moiety to one of the two second chemical moieties;
# represents the binding site of a single bond linking the second chemical moieties to the first chemical moiety;
Z is at each occurrence independently selected from the group consisting of a direct bond, $CR^3R^4$, $C=CR^3R^4$, C=O, $C=NR^3$, $NR^3$, O, $SiR^3R^4$, S, S(O) and $S(O)_2$;

$R^1$, $R^2$, $R^I$ is independently from each other at each occurrence independently selected from the group consisting of:
hydrogen, deuterium,
$C_1$-$C_5$-alkyl,
wherein one or more hydrogen atoms are optionally substituted by deuterium;
C2-C8-alkenyl,
wherein one or more hydrogen atoms are optionally substituted by deuterium;
C2-C8-alkynyl,
wherein one or more hydrogen atoms are optionally substituted by deuterium; and
$C_6$-$C_{18}$-aryl,
which is optionally substituted with one or more substituents $R^6$;
$R^{Tz}$ is at each occurrence independently selected from the group consisting of:

hydrogen, deuterium,
$C_1$-$C_5$-alkyl,
wherein one or more hydrogen atoms are optionally substituted by deuterium;
$C_6$-$C_{18}$-aryl,
which is optionally substituted with one or more substituents $R^6$, and $C_3$-$C_{17}$-heteroaryl,
which is optionally substituted with one or more substituents $R^6$;
$R^a$, $R^3$ and $R^4$ is at each occurrence independently selected from the group consisting of: hydrogen, deuterium, $N(R^5)_2$, $OR^5$, $Si(R^5)_3$, $B(OR^5)_2$, $OSO_2R^5$, $CF_3$, CN, F, Br, I, $C_1$-$C_{40}$-alkyl,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$;
$R^5$ is at each occurrence independently selected from the group consisting of hydrogen, deuterium, $N(R^6)_2$, $OR^6$, $Si(R^6)_3$, $B(OR^6)_2$, $OSO_2R^6$, $CF_3$, CN, F, Br, I,
$C_1$-$C_{40}$-alkyl,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-alkoxy,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-thioalkoxy,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkenyl,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkynyl,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_6$-$C_{60}$-aryl,
which is optionally substituted with one or more substituents $R^6$; and
$C_3$-$C_{57}$-heteroaryl,
which is optionally substituted with one or more substituents $R^6$;
$R^6$ is at each occurrence independently selected from the group consisting of: hydrogen, deuterium, OPh, $CF_3$, CN, F,
$C_1$-$C_5$-alkyl,
wherein one or more hydrogen atoms are optionally, independently from each other substituted by deuterium, CN, $CF_3$, or F;
$C_1$-$C_5$-alkoxy,
wherein one or more hydrogen atoms are optionally, independently from each other substituted by deuterium, CN, $CF_3$, or F;
$C_1$-$C_5$-thioalkoxy,
wherein one or more hydrogen atoms are optionally, independently from each other substituted by deuterium, CN, $CF_3$, or F;
C2-C5-alkenyl,
wherein one or more hydrogen atoms are optionally, independently from each other substituted by deuterium, CN, $CF_3$, or F;
C2-C5-alkynyl,
wherein one or more hydrogen atoms are optionally, independently from each other substituted by deuterium, CN, $CF_3$, or F;
$C_6$-$C_{18}$-aryl,
which is optionally substituted with one or more $C_1$-$C_5$-alkyl substituents;
$C_3$-$C_{17}$-heteroaryl,
which is optionally substituted with one or more $C_1$-$C_5$-alkyl substituents;
$N(C_6$-$C_{18}$-aryl$)_2$;
$N(C_3$-$C_{17}$-heteroaryl$)_2$,
and $N(C_3$-$C_{17}$-heteroaryl$)(C_6$-$C_{18}$-aryl$)$;
wherein the substituents $R^a$, $R^3$, $R^4$ or $R^5$ independently from each other optionally form a mono- or polycyclic, aliphatic, aromatic and/or benzo-fused ring system with one or more substituents $R^a$, $R^3$,

$R^4$ or $R^5$;
wherein
exactly one ring member selected from the group consisting of $L^T$, $L^V$ and $L^W$ is N,
exactly one substituent selected from the group consisting of $R^T$, $R^V$, $R^W$ and $R^X$ is 1,3,5-triazinyl, which is substituted with two substituents $R^{Tz}$,
exactly one substituent selected from the group consisting of $R^T$, $R^V$, $R^W$ and $R^X$ is CN, and exactly one substituent selected from the group consisting of W, Y and X represents the binding site of a single bond linking the first chemical moiety and one of the two second chemical moieties.

2. The organic molecule according to claim 1, wherein the first chemical moiety comprises a structure of Formula Ia:

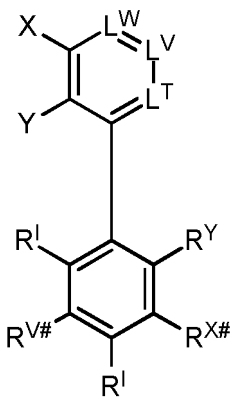

Formula Ia

wherein
exactly one substituent selected from the group consisting of $R^{V\#}$ and $R^{X\#}$ is 1,3,5-triazinyl, which is substituted with two substituents $R^{Tz}$,
exactly one substituent selected from the group consisting of $R^{V\#}$ and $R^{X\#}$ is CN.

3. The organic molecule according to claim 1 or 2, wherein $R^1$, $R^2$ and $R^I$ is independently from each other at each occurrence independently selected from the group consisting of H, methyl, mesityl, tolyl, and phenyl.

4. The organic molecule according to one or more of claims 1 to 3, wherein $R^{Tz}$ is phenyl at each occurrence, which is optionally substituted with one or more substituents $R^5$.

5. The organic molecule according to one or more of claims 1 to 4, wherein the two second chemical moieties independently comprise a structure of Formula IIa:

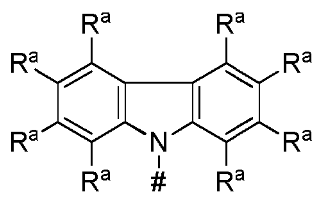

Formula IIa

wherein # and $R^a$ are defined as in claim 1.

6. The organic molecule according to one or more of claims 1 to 5, wherein at least one of the two second chemical moieties independently comprise a structure of Formula IIb:

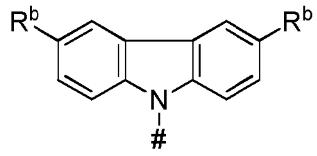

Formula IIb

wherein

$R^b$ is at each occurrence independently selected from the group consisting of: deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-alkyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$;
and wherein apart from that the definitions in claim 1 apply.

**7.** The organic molecule according to one or more of claims 1 to 5, wherein at least one of the two second chemical moieties independently comprise a structure of Formula IIc:

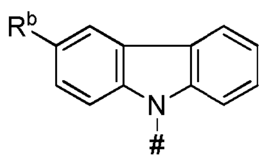

Formula IIc

wherein

$R^b$ is at each occurrence independently selected from the group consisting of deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-alkyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$;
and wherein apart from that the definitions in claim 1 apply.

**8.** The organic molecule according to claim 6 or 7, wherein $R^b$ is at each occurrence independently selected from the group consisting of

- Me, $^i$Pr, $^t$Bu, CN, $CF_3$,
- Ph, which is optionally substituted with one or more substituents independently from each other selected from the group consisting of Me, $^i$Pr, $^t$Bu, CN, $CF_3$ and Ph;
- pyridinyl, which is optionally substituted with one or more substituents independently from each other selected from the group consisting of Me, $^i$Pr, $^t$Bu, CN, $CF_3$ and Ph;
- pyrimidinyl, which is optionally substituted with one or more substituents independently from each other selected from the group consisting of Me, $^i$Pr, $^t$Bu, CN, $CF_3$ and Ph;
- carbazolyl, which is optionally substituted with one or more substituents independently from each other selected from the group consisting of Me, $^i$Pr, $^t$Bu, CN, $CF_3$ and Ph;
- triazinyl, which is optionally substituted with one or more substituents independently from each other selected from the group consisting of Me, $^i$Pr, $^t$Bu, CN, $CF_3$, and Ph;
and
- $N(Ph)_2$.

**9.** Use of an organic molecule according to one or more of claims 1 to 8 as a luminescent emitter and/or a host material

and/or an electron transport material and/or a hole injection material and/or a hole blocking material in an optoelectronic device.

**10.** The use according to claim 9, wherein the optoelectronic device is selected from the group consisting of:

- organic light-emitting diodes (OLEDs),
- light-emitting electrochemical cells,
- OLED-sensors,
- organic diodes,
- organic solar cells,
- organic transistors,
- organic field-effect transistors,
- organic lasers, and
- down-conversion elements.

**11.** A composition, comprising:

(a) an organic molecule according to one or more of claims 1 to 8, in particular in the form of an emitter and/or a host, and
(b) an emitter and/or host material, which differs from the organic molecule, and
(c) optionally, a dye and/or a solvent.

**12.** An optoelectronic device, comprising an organic molecule according to one or more of claims 1 to 8 or a composition according to claim 11, in particular in form of a device selected from the group consisting of organic light-emitting diode (OLED), light-emitting electrochemical cell, OLED-sensor, organic diode, organic solar cell, organic transistor, organic field-effect transistor, organic laser, and down-conversion element.

**13.** The optoelectronic device according to claim 12, comprising

- a substrate,
- an anode, and
- a cathode, wherein the anode or the cathode are disposed on the substrate, and
- at least a light-emitting layer, which is arranged between the anode and the cathode and which comprises the organic molecule or the composition.

**14.** A method for producing an optoelectronic device, wherein an organic molecule according to any one of claims 1 to 8 or a composition according to claim 11 is used.

**15.** The method according to claim 14, comprising the processing of the organic molecule by a vacuum evaporation method or from a solution.

**Patentansprüche**

**1.** Organisches Molekül, bestehend aus

- einer ersten chemischen Gruppierung, die eine Struktur der Formel I

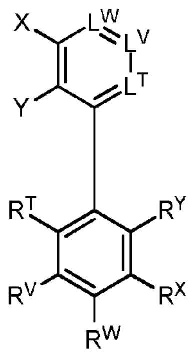

Formel I

umfasst, und

- zwei zweiten chemischen Gruppierungen, die unabhängig voneinander eine Struktur der Formel II

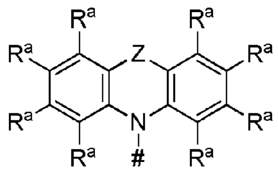

Formel II

umfassen, wobei die erste chemische Gruppierung über eine Einfachbindung mit jeder der zwei zweiten chemischen Gruppierungen verknüpft ist;
wobei

$L^T$ für N oder C-$R^1$ steht;
$L^V$ für N oder C-$R^1$ steht;
$L^W$ für N oder C-W steht;
X für die Bindungsstelle einer Einfachbindung steht, die die erste chemische Gruppierung mit einer der zwei zweiten chemischen Gruppierungen verknüpft, oder für $R^2$ steht;
Y für die Bindungsstelle einer Einfachbindung steht, die die erste chemische Gruppierung mit einer der zwei zweiten chemischen Gruppierungen verknüpft, oder für $R^2$ steht;
W für die Bindungsstelle einer Einfachbindung steht, die die erste chemische Gruppierung mit einer der zwei zweiten chemischen Gruppierungen verknüpft, oder für $R^2$ steht;
$R^T$ aus der Gruppe bestehend aus $R^I$, CN und 1,3,5-Triazinyl, das durch zwei Substituenten $R^{Tz}$ substituiert ist, ausgewählt ist;
$R^V$ aus der Gruppe bestehend aus $R^I$, CN und 1,3,5-Triazinyl, das durch zwei Substituenten $R^{Tz}$ substituiert ist, ausgewählt ist;
$R^W$ aus der Gruppe bestehend aus $R^I$, CN und 1,3,5-Triazinyl, das durch zwei Substituenten $R^{Tz}$ substituiert ist, ausgewählt ist;
$R^X$ aus der Gruppe bestehend aus $R^I$, CN und 1,3,5-Triazinyl, das durch zwei Substituenten $R^{Tz}$ substituiert ist, ausgewählt ist;
$R^Y$ für die Bindungsstelle einer Einfachbindung steht, die die erste chemische Gruppierung mit einer der zwei zweiten chemischen Gruppierungen verknüpft;
# für die Bindungsstelle einer Einfachbindung steht, die die zweiten chemischen Gruppierungen mit der ersten chemischen Gruppierung verknüpft;
Z bei jedem Auftreten unabhängig aus der Gruppe bestehend aus einer direkten Bindung, $CR^3R^4$, $C=CR^3R^4$, C=O, $C=NR^3$, $NR^3$, O, $SiR^3R^4$, S, S(O) und $S(O)_2$ ausgewählt ist;
$R^1$, $R^2$ und $R^I$ unabhängig voneinander bei jedem Auftreten ausgewählt sind aus der Gruppe bestehend aus:

Wasserstoff, Deuterium,
$C_1$-$C_5$-Alkyl,
wobei ein oder mehrere Wasserstoffatome optional durch Deuterium substituiert sind;
$C_2$-$C_8$-Alkenyl,

wobei ein oder mehrere Wasserstoffatome optional durch Deuterium substituiert sind;
$C_2$-$C_8$-Alkinyl,
wobei ein oder mehrere Wasserstoffatome optional durch Deuterium substituiert sind; und
$C_6$-$C_{18}$-Aryl,
das optional durch einen oder mehrere Substituenten $R^6$ substituiert ist;
$R^{Tz}$ bei jedem Auftreten unabhängig ausgewählt ist aus der Gruppe bestehend aus:

Wasserstoff, Deuterium,
$C_1$-$C_5$-Alkyl,
wobei ein oder mehrere Wasserstoffatome optional durch Deuterium substituiert sind;
$C_6$-$C_{18}$-Aryl,
das optional durch einen oder mehrere Substituenten $R^6$ substituiert ist; und
$C_3$-$C_{17}$-Heteroaryl,
das optional durch einen oder mehrere Substituenten $R^6$ substituiert ist;
$R^a$, $R^3$ und $R^4$ bei jedem Auftreten unabhängig ausgewählt sind aus der Gruppe bestehend aus:

Wasserstoff, Deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-Alkyl,
das optional durch einen oder mehrere Substituenten $R^5$ substituiert ist und wobei eine oder mehrere nicht benachbarte $CH_2$-Gruppen optional durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;
$C_1$-$C_{40}$-Alkoxy,
das optional durch einen oder mehrere Substituenten $R^5$ substituiert ist und wobei eine oder mehrere nicht benachbarte $CH_2$-Gruppen optional durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;
$C_1$-$C_{40}$-Thioalkoxy,
das optional durch einen oder mehrere Substituenten $R^5$ substituiert ist und wobei eine oder mehrere nicht benachbarte $CH_2$-Gruppen optional durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;
$C_2$-$C_{40}$-Alkenyl,
das optional durch einen oder mehrere Substituenten $R^5$ substituiert ist und wobei eine oder mehrere nicht benachbarte $CH_2$-Gruppen optional durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;
$C_2$-$C_{40}$-Alkinyl,
das optional durch einen oder mehrere Substituenten $R^5$ substituiert ist und wobei eine oder mehrere nicht benachbarte $CH_2$-Gruppen optional durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;
$C_6$-$C_{60}$-Aryl,
das optional durch einen oder mehrere Substituenten $R^5$ substituiert ist; und
$C_3$-$C_{57}$-Heteroaryl,
das optional durch einen oder mehrere Substituenten $R^5$ substituiert ist;
$R^5$ bei jedem Auftreten unabhängig ausgewählt ist aus der Gruppe bestehend aus Wasserstoff, Deuterium, $N(R^6)_2$, $OR^6$, $Si(R^6)_3$, $B(OR^6)_2$, $OSO_2R^6$, $CF_3$, CN, F, Br, I,
$C_1$-$C_{40}$-Alkyl,
das optional durch einen oder mehrere Substituenten $R^6$ substituiert ist und wobei eine oder mehrere nicht benachbarte $CH_2$-Gruppen optional durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sind;
$C_1$-$C_{40}$-Alkoxy,
das optional durch einen oder mehrere Substituenten $R^6$ substituiert ist und wobei eine oder mehrere nicht benachbarte $CH_2$-Gruppen optional durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sind;
$C_1$-$C_{40}$-Thioalkoxy,
das optional durch einen oder mehrere Substituenten $R^6$ substituiert ist und wobei eine oder mehrere nicht benachbarte $CH_2$-Gruppen optional durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sind;
$C_2$-$C_{40}$-Alkenyl,
das optional durch einen oder mehrere Substituenten $R^6$ substituiert ist und wobei eine oder mehrere nicht benachbarte $CH_2$-Gruppen optional durch $R^6C=CR^6$, C=C, $Si(R^6)_2$, $Ge(R^6)_2$,

$Sn(R^6)_2$, C=O, C=S, C=Se, $C=NR^6$, $P(=O)(R^6)$, SO, $SO_2$, $NR^6$, O, S oder $CONR^6$ ersetzt sind;
$C_2$-$C_{40}$-Alkinyl,
das optional durch einen oder mehrere Substituenten $R^6$ substituiert ist und wobei eine oder mehrere nicht benachbarte $CH_2$-Gruppen optional durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sind;
$C_6$-$C_{60}$-Aryl,
das optional durch einen oder mehrere Substituenten $R^6$ substituiert ist; und
$C_3$-$C_{57}$-Heteroaryl,
das optional durch einen oder mehrere Substituenten $R^6$ substituiert ist;
$R^6$ bei jedem Auftreten unabhängig voneinander ausgewählt ist aus der Gruppe bestehend aus:

Wasserstoff, Deuterium, OPh, $CF_3$, CN, F,
$C_1$-$C_5$-Alkyl,
wobei ein oder mehrere Wasserstoffatome optional unabhängig voneinander durch Deuterium, CN, $CF_3$ oder F ersetzt sind;
$C_1$-$C_5$-Alkoxy,
wobei ein oder mehrere Wasserstoffatome optional unabhängig voneinander durch Deuterium, CN, $CF_3$ oder F ersetzt sind;
$C_1$-$C_5$-Thioalkoxy,
wobei ein oder mehrere Wasserstoffatome optional unabhängig voneinander durch Deuterium, CN, $CF_3$ oder F ersetzt sind;
$C_2$-$C_5$-Alkenyl,
wobei ein oder mehrere Wasserstoffatome optional unabhängig voneinander durch Deuterium, CN, $CF_3$ oder F ersetzt sind;
$C_2$-$C_5$-Alkinyl,
wobei ein oder mehrere Wasserstoffatome optional unabhängig voneinander durch Deuterium, CN, $CF_3$ oder F ersetzt sind;
$C_6$-$C_{18}$-Aryl,
das optional durch einen oder mehrere $C_1$-$C_5$-Alkylsubstituenten substituiert ist;
$C_3$-$C_{17}$-Heteroaryl,
das optional durch einen oder mehrere $C_1$-$C_5$-Alkylsubstituenten substituiert ist;
$N(C_6$-$C_{18}$-Aryl$)_2$;
$N(C_3$-$C_{17}$-Heteroaryl$)_2$,
und $N(C_3$-$C_{17}$-Heteroaryl$)(C_6$-$C_{18}$-aryl)
wobei die Substituenten $R^a$, $R^3$, $R^4$ oder $R^5$ unabhängig voneinander optional mit einem oder mehreren Substituenten $R^a$, $R^3$, $R^4$ oder $R^5$ ein mono- oder polycyclisches, aliphatisches, aromatisches und/oder benzoanneliertes Ringsystem bilden;
wobei
genau ein Ringatom aus der Gruppe bestehend aus $L^T$, $L^V$ und $L^W$ für N steht, genau ein Substituent aus der Gruppe bestehend aus $R^T$, $R^V$, $R^W$ und $R^X$ für 1,3,5-Triazinyl, das durch zwei Substituenten $R^{Tz}$ substituiert ist, steht;
genau ein Substituent aus der Gruppe bestehend aus $R^T$, $R^V$, $R^W$ und $R^X$ für CN steht und genau ein Substituent aus der Gruppe bestehend aus W, Y und X für die Bindungsstelle einer Einfachbindung steht, die die erste chemische Gruppierung und eine der zwei zweiten chemischen Gruppierungen verknüpft.

**2.** Organisches Molekül nach Anspruch 1, wobei die erste chemische Gruppierung eine Struktur der Formel Ia:

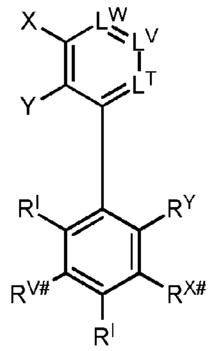

Formel Ia

umfasst, wobei
genau ein Substituent aus der Gruppe bestehend aus $R^{V\#}$ und $R^{X\#}$ für 1,3,5-Triazinyl, das durch zwei Substituenten $R^{Tz}$ substituiert ist, steht,
genau ein Substituent aus der Gruppe bestehend aus $R^{V\#}$ und $R^{X\#}$ für CN steht.

3. Organisches Molekül nach Anspruch 1 oder 2, wobei $R^1$, $R^2$ und $R^I$ bei jedem Auftreten unabhängig voneinander aus der Gruppe bestehend aus H, Methyl, Mesityl, Tolyl und Phenyl ausgewählt sind.

4. Organisches Molekül nach einem oder mehreren der Ansprüche 1 bis 3, wobei $R^{Tz}$ bei jedem Auftreten für Phenyl, das optional durch einen oder mehrere Substituenten $R^5$ substituiert ist, steht.

5. Organisches Molekül nach einem oder mehreren der Ansprüche 1 bis 4, wobei die zwei zweiten chemischen Gruppierungen unabhängig eine Struktur der Formel IIa:

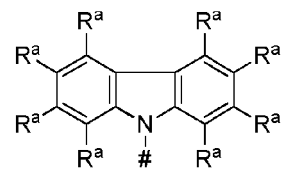

Formel IIa

umfassen, wobei # und $R^a$ wie in Anspruch 1 definiert sind.

6. Organisches Molekül nach einem oder mehreren der Ansprüche 1 bis 5, wobei die zwei zweiten chemischen Gruppierungen bei jedem Auftreten unabhängig voneinander eine Struktur der Formel IIb:

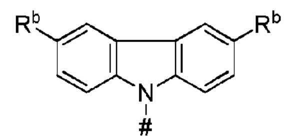

Formel IIb

umfassen, wobei
$R^b$ bei jedem Auftreten unabhängig aus der Gruppe bestehend aus:

Deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-Alkyl,
das optional durch einen oder mehrere Substituenten $R^5$ substituiert ist und wobei eine oder mehrere nicht benachbarte $CH_2$-Gruppen optional durch $R^5C{=}CR^5$, C≡C, $Si(R^5)_2$, $Ge(R^5)_2$, $Sn(R^5)_2$, C=O, C=S, C=Se, $C{=}NR^5$, $P({=}O)(R^5)$, SO, $SO_2$, $NR^5$, O, S oder $CONR^5$ ersetzt sind;

$C_1$-$C_{40}$-Alkoxy,
das optional durch einen oder mehrere Substituenten $R^5$ substituiert ist und wobei eine oder mehrere nicht benachbarte $CH_2$-Gruppen optional durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;
$C_1$-$C_{40}$-Thioalkoxy,
das optional durch einen oder mehrere Substituenten $R^5$ substituiert ist und wobei eine oder mehrere nicht benachbarte $CH_2$-Gruppen optional durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;
$C_2$-$C_{40}$-Alkenyl,
das optional durch einen oder mehrere Substituenten $R^5$ substituiert ist und wobei eine oder mehrere nicht benachbarte $CH_2$-Gruppen optional durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;
$C_2$-$C_{40}$-Alkinyl,
das optional durch einen oder mehrere Substituenten $R^5$ substituiert ist und wobei eine oder mehrere nicht benachbarte $CH_2$-Gruppen optional durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;
$C_6$-$C_{60}$-Aryl,
das optional durch einen oder mehrere Substituenten $R^5$ substituiert ist; und
$C_3$-$C_{57}$-Heteroaryl,
das optional durch einen oder mehrere Substituenten $R^5$ substituiert ist;
ausgewählt ist;
und wobei davon abgesehen die Definitionen in Anspruch 1 gelten.

**7.** Organisches Molekül nach einem oder mehreren der Ansprüche 1 bis 5, wobei die zwei zweiten chemischen Gruppierungen bei jedem Auftreten unabhängig voneinander eine Struktur der Formel IIc:

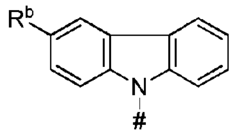

Formel IIc

umfassen, wobei
$R^b$ bei jedem Auftreten unabhängig aus der Gruppe bestehend aus:

Deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-Alkyl,
das optional durch einen oder mehrere Substituenten $R^5$ substituiert ist und wobei eine oder mehrere nicht benachbarte $CH_2$-Gruppen optional durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;
$C_1$-$C_{40}$-Alkoxy,
das optional durch einen oder mehrere Substituenten $R^5$ substituiert ist und wobei eine oder mehrere nicht benachbarte $CH_2$-Gruppen optional durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;
$C_1$-$C_{40}$-Thioalkoxy,
das optional durch einen oder mehrere Substituenten $R^5$ substituiert ist und wobei eine oder mehrere nicht benachbarte $CH_2$-Gruppen optional durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;
$C_2$-$C_{40}$-Alkenyl,
das optional durch einen oder mehrere Substituenten $R^5$ substituiert ist und wobei eine oder mehrere nicht benachbarte $CH_2$-Gruppen optional durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;
$C_2$-$C_{40}$-Alkinyl,
das optional durch einen oder mehrere Substituenten $R^5$ substituiert ist und wobei eine oder mehrere nicht benachbarte $CH_2$-Gruppen optional durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;
$C_6$-$C_{60}$-Aryl,

das optional durch einen oder mehrere Substituenten $R^5$ substituiert ist; und
$C_3$-$C_{57}$-Heteroaryl,
das optional durch einen oder mehrere Substituenten $R^5$ substituiert ist;
ausgewählt ist;
und wobei davon abgesehen die Definitionen in Anspruch 1 gelten.

**8.** Organisches Molekül nach Anspruch 6 oder 7, wobei $R^b$ bei jedem Auftreten unabhängig aus der Gruppe bestehend aus:

- Me, $^i$Pr, $^t$Bu, CN, $CF_3$,
- Ph, das optional durch einen oder mehrere Substituenten, die unabhängig voneinander aus der Gruppe bestehend aus Me, $^i$Pr, $^t$Bu, CN, $CF_3$ und Ph ausgewählt sind, substituiert ist;
- Pyridinyl, das optional durch einen oder mehrere Substituenten, die unabhängig voneinander aus der Gruppe bestehend aus Me, $^i$Pr, $^t$Bu, CN, $CF_3$ und Ph ausgewählt sind, substituiert ist;
- Pyrimidinyl, das optional durch einen oder mehrere Substituenten, die unabhängig voneinander aus der Gruppe bestehend aus Me, $^i$Pr, $^t$Bu, CN, $CF_3$ und Ph ausgewählt sind, substituiert ist;
- Carbazolyl, das optional durch einen oder mehrere Substituenten, die unabhängig voneinander aus der Gruppe bestehend aus Me, $^i$Pr, $^t$Bu, CN, $CF_3$ und Ph ausgewählt sind, substituiert ist;
- Triazinyl, das optional durch einen oder mehrere Substituenten, die unabhängig voneinander aus der Gruppe bestehend aus Me, $^i$Pr, $^t$Bu, CN, $CF_3$ und Ph ausgewählt sind, substituiert ist;
und
- $N(Ph)_2$

ausgewählt ist.

**9.** Verwendung eines organischen Moleküls nach einem der Ansprüche 1 bis 8 als lumineszierender Emitter und/oder als Wirtsmaterial und/oder als Elektronentransportmaterial und/oder als Lochinjektionsmaterial und/oder als Lochblockiermaterial in einer optoelektronischen Vorrichtung.

**10.** Verwendung nach Anspruch 9, wobei die optoelektronische Vorrichtung aus der Gruppe bestehend aus:

• organischen lichtemittierenden Dioden (OLEDs),
• lichtemittierenden elektrochemischen Zellen,
• OLED-Sensoren,
• organischen Dioden,
• organischen Solarzellen,
• organischen Transistoren,
• organischen Feldeffekttransistoren,
• organischen Lasern und
• Down-Konversions-Elementen

ausgewählt ist.

**11.** Zusammensetzung, umfassend:

(a) mindestens ein organisches Molekül nach einem oder mehreren der Ansprüche 1 bis 8, insbesondere in Form eines Emitters und/oder eines Wirts, und
(b) ein Emitter- und/oder Wirtsmaterial, das von dem organischen Molekül verschieden ist, und
(c) optional einen Farbstoff und/oder ein Lösungsmittel.

**12.** Optoelektronische Vorrichtung, umfassend ein organisches Molekül nach einem oder mehreren der Ansprüche 1 bis 8 oder eine Zusammensetzung nach Anspruch 11, insbesondere in Form einer Vorrichtung aus der Gruppe bestehend aus einer organischen lichtemittierenden Diode (OLED), einer lichtemittierenden elektrochemischen Zelle, einem OLED-Sensor, einer organischen Diode, einer organischen Solarzelle, einem organischen Transistor, einem organischen Feldeffekttransistor, einem organischen Laser und einem Down-Konversions-Element.

**13.** Optoelektronische Vorrichtung nach Anspruch 12, umfassend:

- ein Substrat,
- eine Anode und
- eine Kathode, wobei die Anode oder die Kathode auf das Substrat aufgebracht ist, und
- mindestens eine lichtemittierende Schicht, die zwischen Anode und Kathode angeordnet ist und die das organische Molekül oder die Zusammensetzung umfasst.

**14.** Verfahren zur Herstellung einer optoelektronischen Vorrichtung, bei dem ein organisches Molekül nach einem der Ansprüche 1 bis 8 oder eine Zusammensetzung nach Anspruch 11 verwendet wird.

**15.** Verfahren nach Anspruch 14, bei dem das organische Molekül durch ein Vakuumverdampfungsverfahren oder aus einer Lösung verarbeitet wird.

**Revendications**

**1.** Molécule organique, constituée de

- un premier fragment chimique comprenant une structure de formule I,

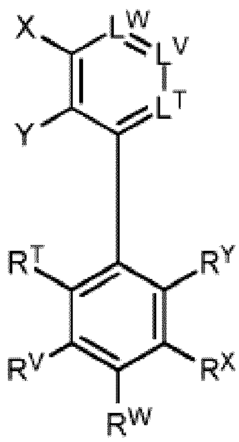

Formule I

et
- deux deuxièmes fragments chimiques, chacun indépendamment comprenant une structure de formule II,

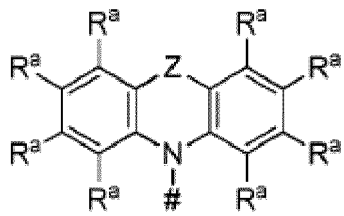

Formule II

le premier fragment chimique étant lié à chacun des deux deuxièmes fragments chimiques via une simple liaison ;

$L^T$ étant N ou C-$R^1$ ;
$L^V$ étant N ou C-$R^1$ ;
$L^W$ étant N ou C-W ;
X étant le site de liaison d'une simple liaison liant le premier fragment chimique à l'un des deux deuxièmes fragments chimiques ou étant $R^2$ ;
Y étant le site de liaison d'une simple liaison liant le premier fragment chimique à l'un des deux deuxièmes fragments chimiques ou étant $R^2$ ;

W étant le site de liaison d'une simple liaison liant le premier fragment chimique à l'un des deux deuxièmes fragments chimiques ou étant $R^2$ ;
$R^T$ étant choisi dans le groupe constitué par $R^I$, CN et 1,3,5-trazinyle, qui est substitué par deux substituants $R^{Tz}$ ;
$R^V$ étant choisi dans le groupe constitué par $R^I$, CN et 1,3,5-trazinyle, qui est substitué par deux substituants $R^{Tz}$ ;
$R^W$ étant choisi dans le groupe constitué par $R^I$, CN et 1,3,5-trazinyle, qui est substitué par deux substituants $R^{Tz}$ ;
$R^X$ étant choisi dans le groupe constitué par $R^I$, CN et 1,3,5-trazinyle, qui est substitué par deux substituants $R^{Tz}$ ;
$R^Y$ étant le site de liaison d'une simple liaison liant le premier fragment chimique à l'un des deux deuxièmes fragments chimiques ;
# représentant le site de liaison d'une simple liaison liant les deuxièmes fragments chimiques au premier fragment chimique ;
Z étant en chaque occurrence indépendamment choisi dans le groupe constitué par une liaison directe, $CR^3R^4$, $C=CR^3R^4$, C=O, $C=NR^3$, $NR^3$, O, $SiR^3R^4$, S, S(O) et $S(O)_2$ ;
$R^1$, $R^2$, $R^I$ étant en chaque occurrence indépendamment l'un de l'autre choisis dans le groupe constitué par :

hydrogène, deutérium,
$C_{1-5}$-alkyle,
un ou plusieurs atomes d'hydrogène étant éventuellement substitués par deutérium ;
$C_{2-8}$-alcényle,
un ou plusieurs atomes d'hydrogène étant éventuellement substitués par deutérium ;
$C_{2-8}$-alcynyle,
un ou plusieurs atomes d'hydrogène étant éventuellement substitués par deutérium ; et
$C_{6-18}$-aryle,
qui est éventuellement substitué par un ou plusieurs substituants $R^6$ ;
$R^{Tz}$ étant en chaque occurrence indépendamment choisi dans le groupe constitué par :

hydrogène, deutérium,
$C_{1-5}$-alkyle,
un ou plusieurs atomes d'hydrogène étant éventuellement substitués par deutérium ;
$C_{6-18}$-aryle,
qui est éventuellement substitué par un ou plusieurs substituants $R^6$, et
$C_{3-17}$-hétéroaryle,
qui est éventuellement substitué par un ou plusieurs substituants $R^6$ ;
$R^a$, $R^3$ et $R^4$ étant en chaque occurrence indépendamment choisis dans le groupe constitué par :
hydrogène, deutérium, $N(R^5)_2$, $OR^5$, $Si(R^5)_3$, $B(OR^5)_2$, $OSO_2R^5$, $CF_3$, CN, F, Br, I,
$C_{1-40}$-alkyle,

qui est éventuellement substitué par un ou plusieurs substituants $R^5$ et
un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$ ;

$C_{1-40}$-alcoxy,

qui est éventuellement substitué par un ou plusieurs substituants $R^5$ et
un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^{5;}$

$C_{1-40}$-thioalcoxy,

qui est éventuellement substitué par un ou plusieurs substituants $R^5$ et
un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^{5;}$

$C_{2-40}$-alcényle,

qui est éventuellement substitué par un ou plusieurs substituants $R^5$ et un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;

$C_{2-40}$-alcynyle,

qui est éventuellement substitué par un ou plusieurs substituants $R^5$ et un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$ ;

$C_{6-60}$-aryle,
qui est éventuellement substitué par un ou plusieurs substituants $R^5$; et
$C_{3-57}$-hétéroaryle,
qui est éventuellement substitué par un ou plusieurs substituants $R^5$;
$R^5$ étant en chaque occurrence indépendamment choisi dans le groupe constitué par hydrogène, deutérium, $N(R^6)_2$, $OR^6$, $Si(R^6)_3$, $B(OR^6)_2$, $OSO_2R^6$, $CF_3$, CN, F, Br, I,
$C_{1-40}$-alkyle,

qui est éventuellement substitué par un ou plusieurs substituants $R^6$ et un ou plusieurs groupes $CH_2$ non adjacents étant éventuellement substitués par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S, ou $CONR^6$;

$C_{1-40}$-alcoxy,

qui est éventuellement substitué par un ou plusieurs substituants $R^6$ et un ou plusieurs groupes $CH_2$ non adjacents étant éventuellement substitués par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S, ou $CONR^6$;

$C_{1-40}$-thioalcoxy,

qui est éventuellement substitué par un ou plusieurs substituants $R^6$ et un ou plusieurs groupes $CH_2$ non adjacents étant éventuellement substitués par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S, ou $CONR^6$;

$C_{2-40}$-alcényle,

qui est éventuellement substitué par un ou plusieurs substituants $R^6$ et un ou plusieurs groupes $CH_2$ non adjacents étant éventuellement substitués par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S, ou $CONR^6$;

$C_{2-40}$-alcynyle,

qui est éventuellement substitué par un ou plusieurs substituants $R^6$ et un ou plusieurs groupes $CH_2$ non adjacents étant éventuellement substitués par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S, ou $CONR^6$;

$C_{6-60}$-aryle,

qui est éventuellement substitué par un ou plusieurs substituants $R^6$ ; et

$C_{3-57}$-hétéroaryle,
qui est éventuellement substitué par un ou plusieurs substituants $R^6$ ;
$R^6$ étant en chaque occurrence indépendamment choisi dans le groupe constitué par : hydrogène, deutérium, OPh, $CF_3$, CN, F,
$C_{1-5}$-alkyle,
un ou plusieurs atomes d'hydrogène étant éventuellement, indépendamment les uns des autres, substitués par deutérium, CN, $CF_3$, ou F ;
$C_{1-5}$-alcoxy,
un ou plusieurs atomes d'hydrogène étant éventuellement, indépendamment les uns des autres, substitués par deutérium, CN, $CF_3$, ou F ;
$C_{1-5}$-thioalcoxy,
un ou plusieurs atomes d'hydrogène étant éventuellement, indépendamment les uns des autres, substitués par deutérium, CN, $CF_3$, ou F ;
$C_{2-5}$-alcényle,
un ou plusieurs atomes d'hydrogène étant éventuellement, indépendamment les uns des autres, substitués par deutérium, CN, $CF_3$, ou F ;
$C_{2-5}$-alcynyle,
un ou plusieurs atomes d'hydrogène étant éventuellement, indépendamment les uns des autres, substitués par deutérium, CN, $CF_3$, ou F ;
$C_{6-18}$-aryle,
qui est éventuellement substitué par un ou plusieurs substituants $C_{1-5}$-alkyle ;
$C_{3-17}$-hétéroaryle,
qui est éventuellement substitué par un ou plusieurs substituants $C_{1-5}$-alkyle ;
$N(C_{6-18}\text{-aryle})_2$;
$N(C_{3-17}\text{-hétéroaryle})_2$,
et $N(C_{3-17}\text{-hétéroaryle})(C_{6-18}\text{-aryle})$;
les substituants $R^a$, $R^3$, $R^4$ ou $R^5$ formant éventuellement indépendamment les uns des autres un système cyclique monocyclique ou polycyclique, aliphatique, aromatique et/ou benzo-condensé avec un ou plusieurs substituants $R^a$, $R^3$, $R^4$ ou $R^5$ ;
exactement un élément de cycle choisi dans le groupe constitué par $L^T$, $L^V$ et $L^W$ étant N,
exactement un substituant choisi dans le groupe constitué par $R^T$, $R^V$, $R^W$ et $R^X$ étant 1,3,5-triazinyle, qui est substitué par deux substituants $R^{Tz}$,
exactement un substituant choisi dans le groupe constitué par $R^T$, $R^V$, $R^W$ et $R^X$ étant CN, et
exactement un substituant choisi dans le groupe constitué par W, Y et X représentant le site de liaison d'une simple liaison liant le premier fragment chimique et l'un des deux deuxièmes fragments chimiques.

2. Molécule organique selon la revendication 1, le premier fragment chimique comprenant une structure de Formule Ia :

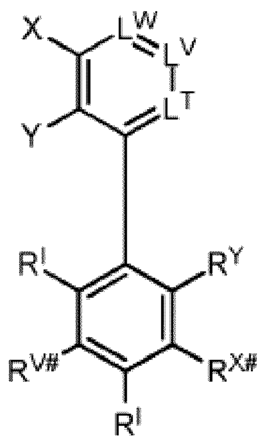

Formule Ia

exactement un substituant choisi dans le groupe constitué par $R^{V\#}$ et $R^{X\#}$ étant 1,3,5-triazinyle, qui est substitué par deux substituants $R^{Tz}$,

exactement un substituant choisi dans le groupe constitué par $R^{V\#}$ et $R^{X\#}$ étant CN.

**3.** Molécule organique selon la revendication 1 ou 2, $R^1$, $R^2$ et $R^I$ étant indépendamment les uns des autres en chaque occurrence indépendamment choisis dans le groupe constitué par H, méthyle, mésityle, tolyle, et phényle.

**4.** Molécule organique selon l'une ou plusieurs des revendications 1 à 3, $R^{Tz}$ étant phényle en chaque occurrence, qui est éventuellement substitué par un ou plusieurs substituants $R^5$.

**5.** Molécule organique selon l'une ou plusieurs des revendications 1 à 4, les deux deuxièmes fragments chimiques comprenant indépendamment une structure de Formule IIa :

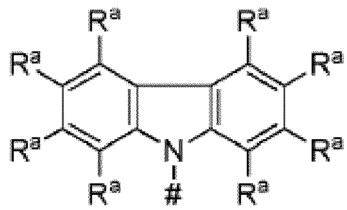

Formule IIa

# et $R^a$ étant définis comme dans la revendication 1.

**6.** Molécule organique selon l'une ou plusieurs des revendications 1 à 5, au moins l'un des deux deuxièmes fragments chimiques comprenant indépendamment une structure de Formule IIb :

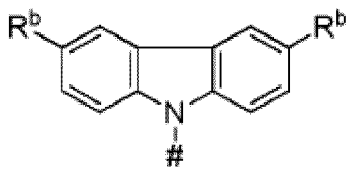

Formule IIb

$R^b$ étant en chaque occurrence indépendamment choisi dans le groupe constitué par : deutérium, $N(R^5)_2$, $OR^5$, $Si(R^5)_3$, $B(OR^5)_2$, $OSO_2R^5$, $CF_3$, CN, F, Br, I,
$C_{1-40}$-alkyle,

qui est éventuellement substitué par un ou plusieurs substituants $R^5$ et
un ou plusieurs groupes $CH_2$ non adjacents étant éventuellement substitués par $R^5C{=}CR^5$, C≡C, $Si(R^5)_2$, $Ge(R^5)_2$, $Sn(R^5)_2$, C=O, C=S, C=Se, $C{=}NR^5$, $P({=}O)(R^5)$, SO, $SO_2$, $NR^5$, O, S, ou $CONR^5$;

$C_{1-40}$-alcoxy,

qui est éventuellement substitué par un ou plusieurs substituants $R^5$ et
un ou plusieurs groupes $CH_2$ non adjacents étant éventuellement substitués par $R^5C{=}CR^5$, C≡C, $Si(R^5)_2$, $Ge(R^5)_2$, $Sn(R^5)_2$, C=O, C=S, C=Se, $C{=}NR^5$, $P({=}O)(R^5)$, SO, $SO_2$, $NR^5$, O, S, ou $CONR^5$;

$C_{1-40}$-thioalcoxy,

qui est éventuellement substitué par un ou plusieurs substituants $R^5$ et
un ou plusieurs groupes $CH_2$ non adjacents étant éventuellement substitués par $R^5C{=}CR^5$, C≡C, $Si(R^5)_2$, $Ge(R^5)_2$, $Sn(R^5)_2$, C=O, C=S, C=Se, $C{=}NR^5$, $P({=}O)(R^5)$, SO, $SO_2$, $NR^5$, O, S, ou $CONR^5$;

$C_{2-40}$-alcényle,

qui est éventuellement substitué par un ou plusieurs substituants $R^5$ et

un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;

$C_{2-40}$-alcynyle,

qui est éventuellement substitué par un ou plusieurs substituants $R^5$ et un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;

$C_{6-60}$-aryle, qui est éventuellement substitué par un ou plusieurs substituants $R^5$; et $C_{3-57}$-hétéroaryle, qui est éventuellement substitué par un ou plusieurs substituants $R^5$; et mis à part cela, les définitions dans la revendication 1 s'appliquant.

7. Molécule organique selon l'une ou plusieurs des revendications 1 à 5, au moins l'un des deux deuxièmes fragments chimiques comprenant indépendamment une structure de Formule IIc :

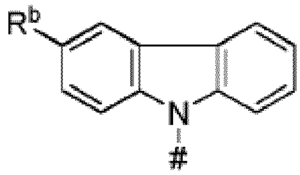

Formule IIc

$R^b$ étant en chaque occurrence indépendamment choisi dans le groupe constitué par deutérium, $N(R^5)_2$, $OR^5$, $Si(R^5)_3$, $B(OR^5)_2$, $OSO_2R^5$, $CF_3$, CN, F, Br, I,
$C_{1-40}$-alkyle,
qui est éventuellement substitué par un ou plusieurs substituants $R^5$ et un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;
$C_{1-40}$-alcoxy,

qui est éventuellement substitué par un ou plusieurs substituants $R^5$ et un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;

$C_{1-40}$-thioalcoxy,

qui est éventuellement substitué par un ou plusieurs substituants $R^5$ et un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;

$C_{2-40}$-alcényle,

qui est éventuellement substitué par un ou plusieurs substituants $R^5$ et un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;

$C_{2-40}$-alcynyle,

qui est éventuellement substitué par un ou plusieurs substituants $R^5$ et un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;

$C_{6-60}$-aryle, qui est éventuellement substitué par un ou plusieurs substituants $R^5$; et $C_3$

$_{-57}$-hétéroaryle,
qui est éventuellement substitué par un ou plusieurs substituants $R^5$;
et mis à part cela, les définitions dans la revendication 1 s'appliquant.

**8.** Molécule organique selon la revendication 6 ou 7, $R^b$ étant en chaque occurrence indépendamment choisi dans le groupe constitué par

- Me, $^i$Pr, $^t$Bu, CN, $CF_3$,
- Ph, qui est éventuellement substitué par un ou plusieurs substituants choisis indépendamment les uns des autres dans le groupe constitué par Me, $^i$Pr, $^t$Bu, CN, $CF_3$ et Ph ;
- pyridinyle, qui est éventuellement substitué par un ou plusieurs substituants choisis indépendamment les uns des autres dans le groupe constitué par Me, $^i$Pr, $^t$Bu, CN, $CF_3$ et Ph ;
- pyrimidinyle, qui est éventuellement substitué par un ou plusieurs substituants choisis indépendamment les uns des autres dans le groupe constitué par Me, $^i$Pr, $^t$Bu, CN, $CF_3$ et Ph ;
- carbazolyle, qui est éventuellement substitué par un ou plusieurs substituants choisis indépendamment les uns des autres dans le groupe constitué par Me, $^i$Pr, $^t$Bu, CN, $CF_3$ et Ph ;
- triazinyle, qui est éventuellement substitué par un ou plusieurs substituants choisis indépendamment les uns des autres dans le groupe constitué par Me, $^i$Pr, $^t$Bu, CN, $CF_3$ et Ph ;
et
- $N(Ph)_2$.

**9.** Utilisation d'une molécule organique selon l'une ou plusieurs des revendications 1 à 8 en tant qu'émetteur luminescent et/ou en tant que matériau hôte et/ou en tant que matériau de transport d'électrons et/ou en tant que matériau d'injection de trous et/ou en tant que matériau de blocage de trous dans un dispositif optoélectronique.

**10.** Utilisation selon la revendication 9, le dispositif optoélectronique étant choisi dans le groupe constitué par :

• des diodes luminescentes organiques (OLED),
• des cellules électrochimiques luminescentes,
• des capteurs OLED,
• des diodes organiques,
• des cellules solaires organiques,
• des transistors organiques,
• des transistors organiques à effet de champ,
• des lasers organiques, et
• des éléments de conversion par abaissement.

**11.** Composition, comprenant :

(a) une molécule organique selon l'une ou plusieurs des revendications 1 à 8, en particulier sous la forme d'un émetteur et/ou d'un hôte, et
(b) un matériau émetteur et/ou hôte, qui diffère de la molécule organique, et
(c) éventuellement, un colorant et/ou un solvant.

**12.** Dispositif optoélectronique, comprenant une molécule organique selon l'une ou plusieurs des revendications 1 à 8 ou une composition selon la revendication 11, en particulier sous la forme d'un dispositif choisi dans le groupe constitué par une diode luminescente organique (OLED), une cellule électrochimique luminescente, un capteur OLED, une diode organique, une cellule solaire organique, un transistor organique, un transistor organique à effet de champ, un laser organique, et un élément de conversion par abaissement.

**13.** Dispositif optoélectronique selon la revendication 12, comprenant

- un substrat,
- une anode, et
- une cathode, l'anode ou la cathode étant disposée sur le substrat, et
- au moins une couche luminescente, qui est agencée entre l'anode et la cathode et qui comprend la molécule organique ou la composition.

**14.** Procédé pour la production d'un dispositif optoélectronique, une molécule organique selon l'une quelconque des revendications 1 à 8 ou une composition selon la revendication 11 étant utilisée.

**15.** Procédé selon la revendication 14, comprenant le traitement de la molécule organique par un procédé d'évaporation sous vide ou à partir d'une solution.

Figure 1

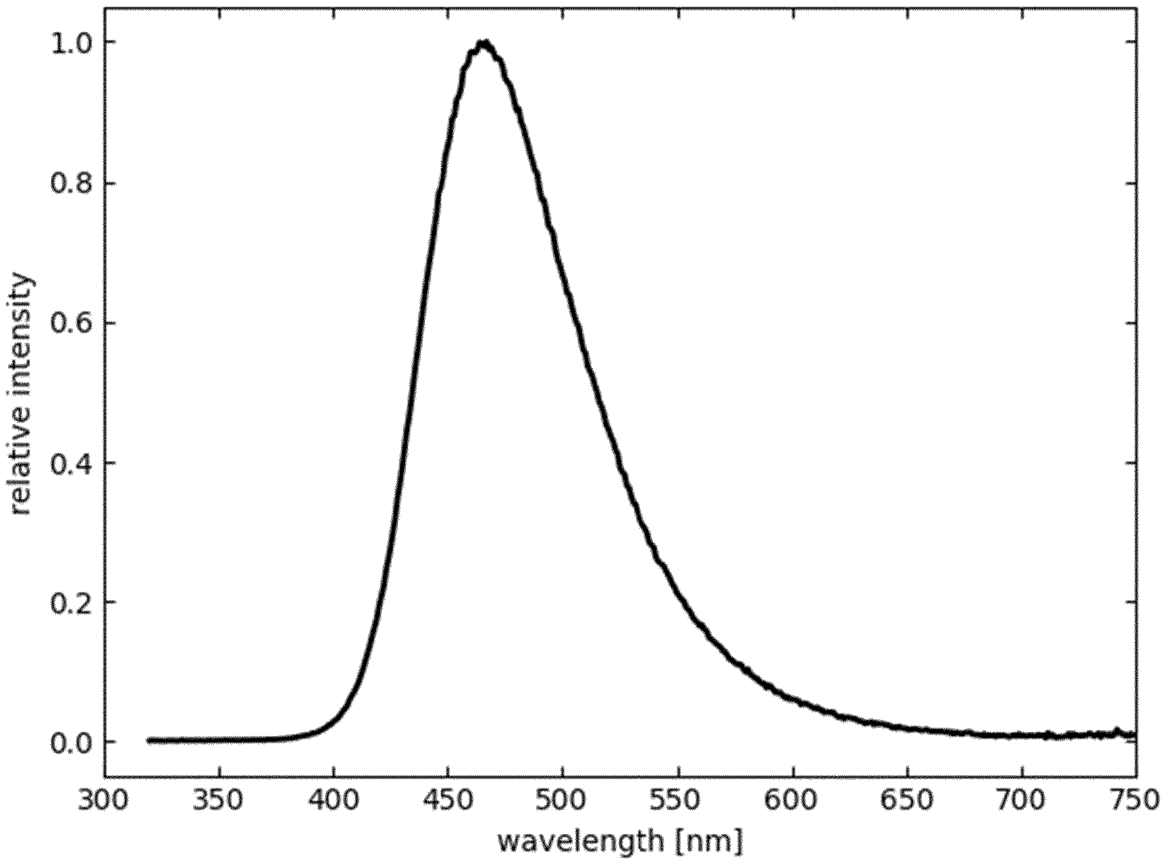
1.0
0.8
0.6
0.4
0.2
0.0
relative intensity
300
350
400
450
500
550
600
650
700
750
wavelength [nm]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3421461 A
- WO 2019002355 A
- US 20180215711 A
- US 20170186962 A


### Non-patent literature cited in the description


- *CHEMICAL ABSTRACTS,* 3842-55-5
- *CHEMICAL ABSTRACTS,* 584-08-7
- *CHEMICAL ABSTRACTS,* 14221-01-3
- *CHEMICAL ABSTRACTS,* 73183-34-3
- *CHEMICAL ABSTRACTS,* 127-08-2
- *CHEMICAL ABSTRACTS,* 72287-26-4
- *CHEMICAL ABSTRACTS,* 7778-53-2
- *CHEMICAL ABSTRACTS,* 357927-50-5
- *CHEMICAL ABSTRACTS,* 86-74-8
- *CHEMICAL ABSTRACTS,* 855738-89-5